# EUROPEAN PATENT SPECIFICATION

(11) **EP 3 508 476 B1**
(45) Date of publication and mention of the grant of the patent: **22.11.2023**
(21) Application number: 17845547.3
(22) Date of filing: 01.09.2017
(51) Int. Cl.: C07C 323/25, C07K 5/06, C07K 5/062, C07K 5/08, C07K 5/083, A61K 31/16, A61P 39/02

(54) **AMINO MERCAPTAN COMPOUND AND PREPARATION METHOD THEREFOR AND USE THEREOF IN PROTECTION AGAINST RADIATION**
AMINO-MERCAPTAN-VERBINDUNG UND HERSTELLUNGSVERFAHREN DAFÜR UND VERWENDUNG DAVON BEIM SCHUTZ GEGEN STRAHLUNG
COMPOSÉ AMINOMERCAPTAN, SON PROCÉDÉ DE PRÉPARATION ET SON UTILISATION DANS LA PROTECTION CONTRE LE RAYONNEMENT

(30) Priority: 05.09.2016 CN 201610802313
(43) Date of publication of application: 10.07.2019
(73) Proprietor: Shanghai Kechow Pharma, Inc., Shanghai 201203 (CN)
(72) Inventor: TIAN, Hongqi, Tianjin 300192 (CN); CHENG, Ying, Tianjin 300192 (CN); ZHANG, Qianru, Tianjin 300192 (CN); ZHU, Zhimei, Tianjin 300192 (CN); WANG, Yueying, Tianjin 300192 (CN)
(74) Representative: Swea IP Law AB
(86) International application number: PCT/CN2017/100158
(87) International publication number: WO 2018/041245

(56) References cited:
- CN-A- 1 679 488
- CN-A- 103 772 245
- CN-A- 106 432 014
- JP-A- H04 321 674
- US-A1- 2004 229 815
- US-A1- 2006 013 784
- US-A1- 2006 287 398
- US-A1- 2013 316 942
- US-A1- 2014 193 340
- E.R. ATKINSON, ET AL.: "Potential antiradiation drugs. I. Amide, hydroxamic acid, and hydrazine derivatives of mercapto acids. Amino thioacids", JOURNAL OF MEDICINAL CHEMISTRY, vol. 8, no. 1, 1 January 1965 (1965-01-01) , pages 29-33, XP002263028, American Chemical Society, Washington, DC, US ISSN: 0022-2623
- N. HARUKI, ET AL.: "Syntheses of Aminothiol Derivatives", JOURNAL OF THE PHARMACEUTICAL SOCIETY OF JAPAN, vol. 84, no. 10, October 1964 (1964-10), pages 944-955, XP055693046, PHARMACEUTICAL SOCIETY OF JAPAN, JP ISSN: 0031-6903, DOI: 10.1248/yakushi1947.84.10_944
- J.R. PIPER, ET AL.: "S-2,.omega.-Diaminoalkyl dihydrogen phosphorothioates as antiradiation agents", JOURNAL OF MEDICINAL CHEMISTRY, vol. 22, no. 6, 1 June 1979 (1979-06-01), pages 631-639, XP002314914, American Chemical Society, Washington, DC, US ISSN: 0022-2623, DOI: 10.1021/jm00192a006
- DATABASE REGISTRY 14 June 2015 (2015-06-14), retrieved from STN Database accession no. RN- 1779639-66-5
- PANFENG FU, ET AL.: "Induction of cellular antioxidant defense by amifostine improves ventilator-induced lung injury", CRITICAL CARE MEDICINE, vol. 39, no. 12, December 2011 (2011-12), pages 2711-2721, XP055970127, Lippincott, Philadelphia, PA, US ISSN: 0090-3493, DOI: 10.1097/CCM.0b013e3182284a5f
- E. OBRADOR, ET AL.: "Radioprotection and Radiomitigation: From the Bench to Clinical Practice", BIOMEDICINES, vol. 8, no. 11, 30 October 2020 (2020-10-30), page 461, XP055970131, MDPI, Basel, CH ISSN: 2227-9059, DOI: 10.3390/biomedicines8110461

## Description

### Technical Field

The present invention relates to the field of medicine, in particular to the protection against ionizing radiation damage, in particular to a new class of compounds having the effect of radiation protecti on. The present invention also relates to a method for prepari ng the compound. The present invention also relates to the use of the compound in the prevention and treatment of damages and diseases caused by ionizing radiation.

### Background Art

With the vigorous development of the global nuclear cause, the nuclear technology has been widely used in various fields, such as nuclear power plants, aerospace, national defense and biomedicine, and the chance of human exposure to ionizing radiation and thus causing damage has increased. In the meantime, with the nuclear war and hidden nuclear terror incident worries brought about by the worlds tense nuclear security situation, the protection and treatment of body damage caused by ionizing radiation (referred to as radiation damage) is receiving more and more attention.

On the other hand, the incidence of malignant tumors and the number of patients has been increasing in recent years. Radiation therapy plays an indispensable role as one of the main treatments. However, high-dose radiation exposure inevitably leads to acute radiation damage to normal tissues and organs surrounding the tumor and even to the whole body. The side effects of radiation damage seriously restrict the wide application of radiotherapy in tumor therapy, and also significantly affect the efficacy and quality of life of cancer patients after radiotherapy.

At present, there are related mainly drugs for radiation damage treatment: sulfur compounds, hormones, cytokines and Chinese herbal medicines, which have their own inherent defects. For example, sulfur compounds are generally associated with greater side effects, such as amifostine, a representative of such compounds, is currently recognized as the best protective compound and is the first selective broad-spectrum cytoprotective agent approved by the international regulatory agencies, but the extremely short half-life (7 min) and high price (the domestic medical market price is 400-500 yuan per dose) limit its application. The prevention and treatment of radiation damage by hormone drugs are mainly for the nucleated cells, hematopoietic stem cells and progenitor cells in bone marrow, and the effects of such drugs on the sexual and reproductive systems limit their widespread use. Cytokine drugs, such as interleukins and colony stimulating factor drugs can alleviate and treat radiation-induced bone marrow hematopoietic system damage, but their radiation protection effect is closely related to the time of admi ni strati on (in the medical practice for prevention and treatment, such drugs require a high level of medical testing and attention), with obvious inflammatory effect, and it is expensive and difficult to store at room temperature. The anti-radi ati on components of C hi nese herbal medicines mainly comprise phenol s, polysaccharides and natural flavones, which have the characteristics of unclear active ingredients and low toxicity, and after years of research, there are no similar drugs on the market or to be marketed.

Atkinson et al, Journal of Medicinal Chemistry, 1965, 8(1), 29-33, discloses a racemic cysteinamide and its usefulness as an anti-radiation drug.

US20130316942 discloses potent compounds having combined antioxidant, anti-inflammatory, anti-radiation and metal chelating properties. Specifically, the present invention relates to short peptides having these properties, and to methods and uses of such short peptides in clinical and cosmetic applications.

### Summary of the Invention

One purpose of the present invention is to provide a new class of compounds with radiation protection. It has the effects of prolonging the survival period and reducing the death rate of animals after sublethal dose irradiation. It can be used alone as a radiation damage protection and treatment drug, or in combination with radiotherapy, which can alleviate and prevent the adverse reactions caused by radiotherapy.

Another purpose of the present invention is to provide a method for preparing the compound.

The further purpose of the present invention is to provide the use of the compound in the preparation of drugs for the prevention and/or treatment of damages and diseases related to ionizing radiation.

According to an aspect of the present invention, a compound having the following chemical structural formula is provided: 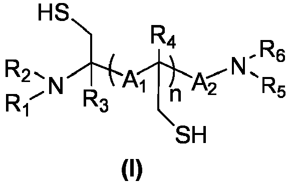 wherein
A₁ is selected from: -C(O)NR⁸-, and -R⁷-NR⁸-;
A₂ is selected from: carbonyl, and unsubstituted C₁₋₆ alkylene;
R¹ is selected from: hydrogen, and unsubstituted C₁-C₅ alkyl;
R² is unsubstituted C₁-C₅ alkyl;
R⁵ is selected from: hydrogen, unsubstituted C₁-C₅ alkyl;
R⁶ is unsubstituted C₁-C₅ alkyl;
n is an integer from 1 to 10;
R³ and R⁴ are independently selected from: hydrogen, and unsubstituted C₁₋₆ alkyl;
R⁷ is unsubstituted C₁-C₆ alkylene;
R⁸ is selected from: hydrogen, and unsubstituted C₁-C₆ alkyl;
or a stereoisomer thereof or a pharmaceutically acceptable salt, or a solvate thereof.

Preferably, A₁ is selected from: -C(O)NR⁸- or -CH₂-NR⁸-;
preferably, A₂ is selected from: carbonyl, unsubstituted C₁₋₃ alkyl; more preferably, A₂ is selected from: carbonyl or methylene; further preferably, A₂ is selected from: carbonyl or methylene;
preferably, R¹ and R² may be the same or different and are selected from hydrogen and C₁₋₃ alkyl and R² and R⁶ are the same or different and are selected from C₁₋₃ alkyl; more preferably, R¹ and R² may be the same or different and are selected from hydrogen, methyl and ethyl and R² and R⁶ are the same or different and are selected from methyl and ethyl; more preferably R¹ is hydrogen and R² is C₁-C₃ alkyl (e.g. methyl, ethyl or propyl), as well as R⁵ is hydrogen and R⁶ is C₁-C₃ alkyl (e.g. methyl, ethyl or propyl); even more preferably R¹ is hydrogen and R² is methyl, as well as R⁵ is hydrogen and R⁶ is methyl; or alternatively, R¹and R² are methyl or ethyl, as well as R⁵ and R⁶ are methyl or ethyl; n is an integer from 1 to 10 (including 1, 2, 3, 4, 5, 6, 7, 8, 9 and 10);
preferably, R³ and R⁴ are independently selected from: hydrogen or unsubstituted C₁₋₃ alkyl; more preferably, R³ and R⁴ are independently selected from: hydrogen or methyl;
preferably, R⁷ is unsubstituted C₁-C₃ alkylene; more preferably, R⁷ is methylene;
preferably, R⁸ is selected from: hydrogen, unsubstituted C₁-C₃ alkyl; more preferably, R⁸ is selected from: hydrogen, methyl or ethyl; further preferably, R⁸ is hydrogen;
in the compound, the chiral carbon directly attached to R³ and R⁴ is in the R configuration or the S configuration. Preferably, the chiral carbon directly attached to R³ and R⁴ is in the R configuration.

More preferably, the chiral carbon directly attached to R³ and R⁴ is in the R configuration; one of R¹ and R² is hydrogen, and the other is methyl, as well as one of R⁵ and R⁶ is hydrogen, and the other is methyl.

In a preferred embodiment of the present invention, the compound has the following general formula:

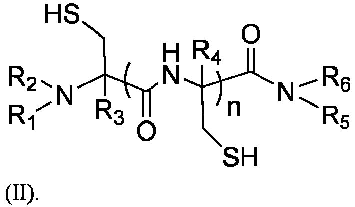

In another preferred embodiment of the present invention, the compound has the following general formulas: 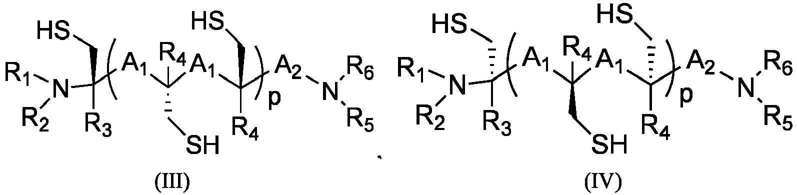 wherein p is an integer of from 1 to 5 (including 1, 2, 3, 4 and 5);
in a preferred embodiment of the present invention, the R¹, R², R⁵, R⁶, and R⁸ are all hydrogen;

In another preferred embodiment of the present invention, R¹ and R⁵ are hydrogen, and R² and R⁶ are selected from: methyl or ethyl. The following compounds are disclosed:

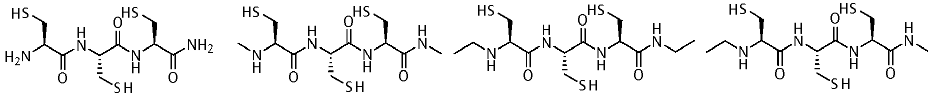

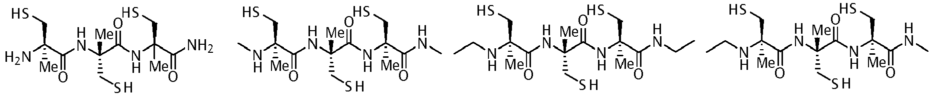

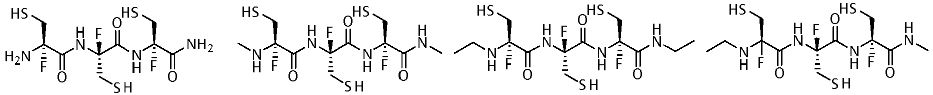

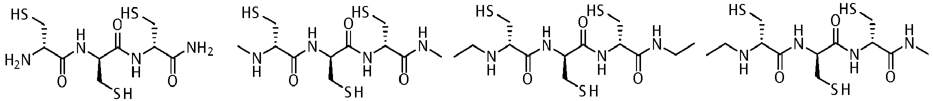

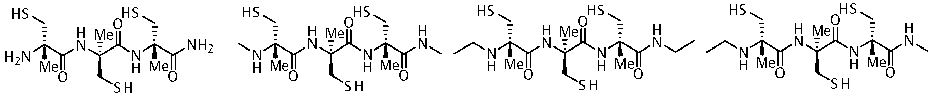

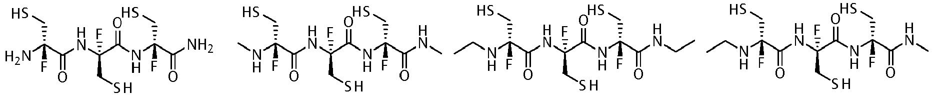

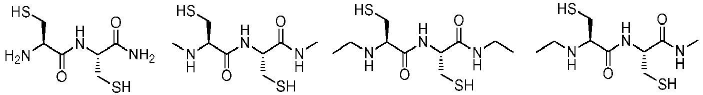

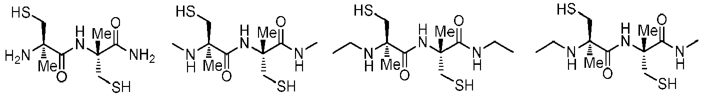

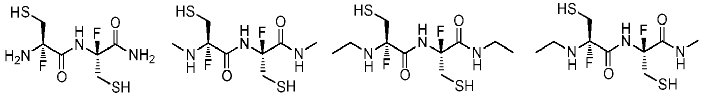

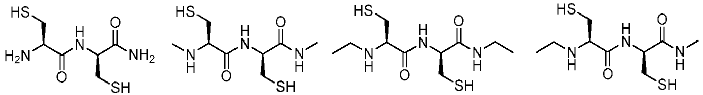

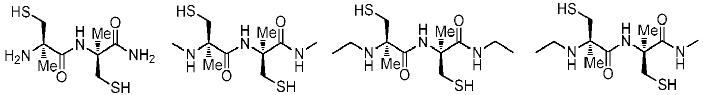

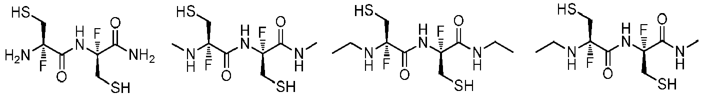

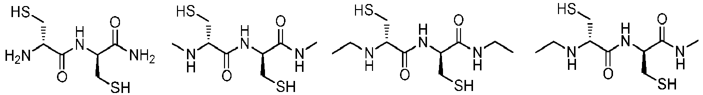

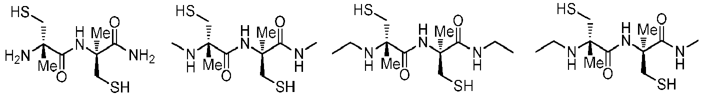

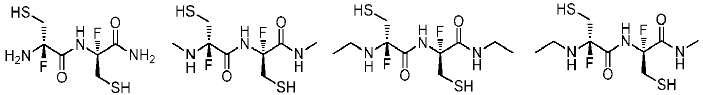

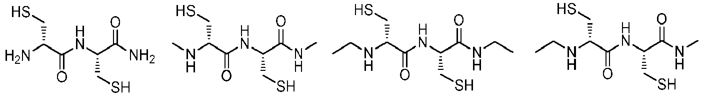

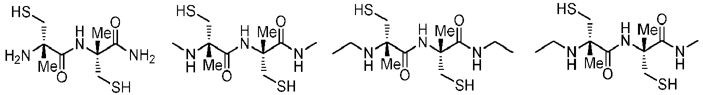

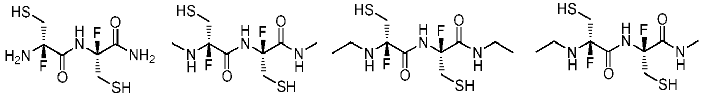

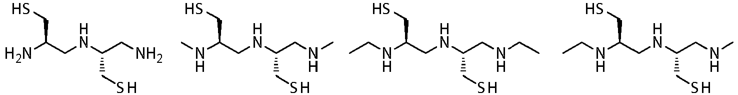

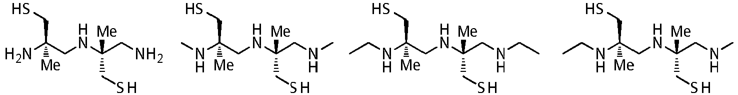

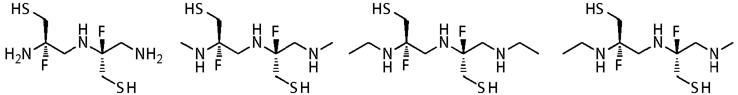

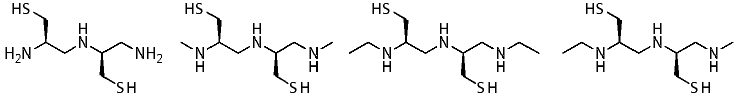

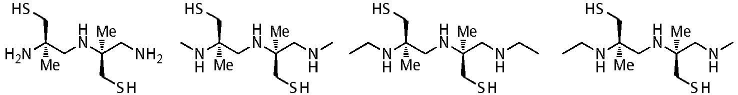

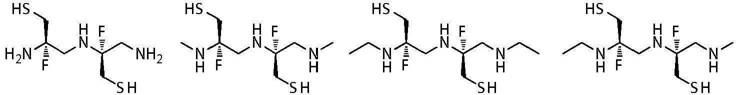

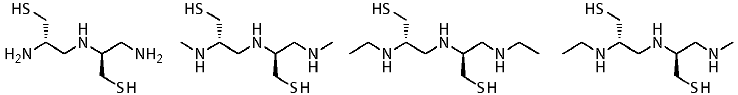

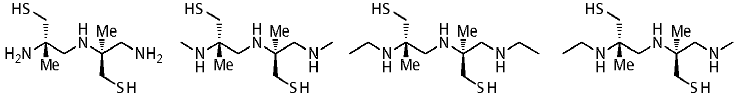

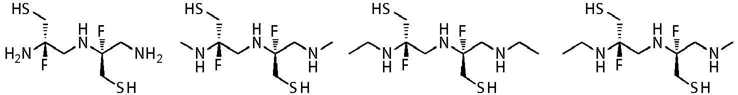

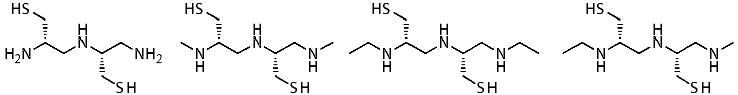

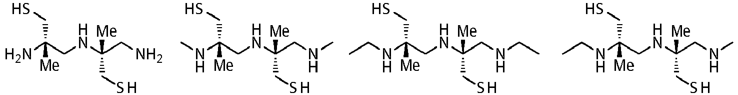

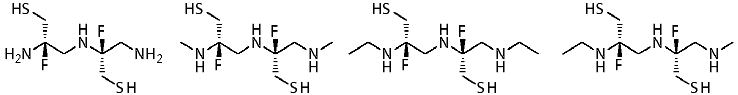

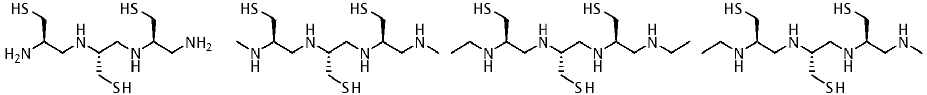

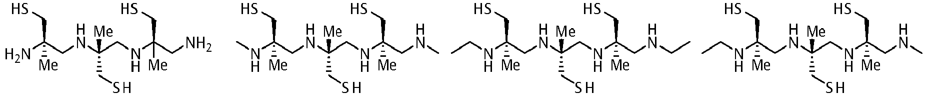

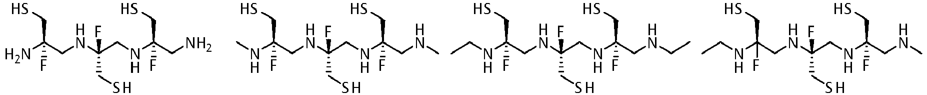

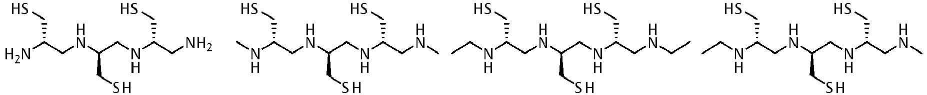

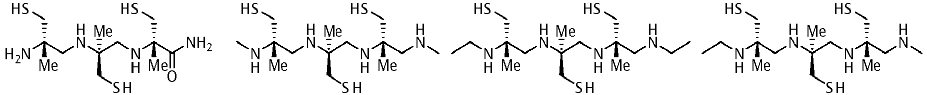

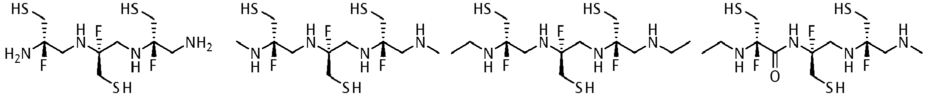

In a preferred embodiment of the present invention, the above compounds may preferably be the following compounds, but are not limited to the following compounds:

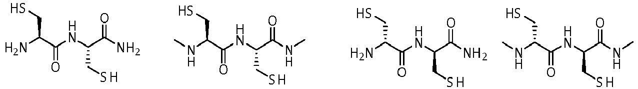

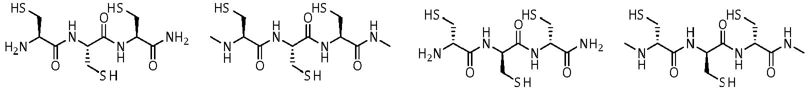

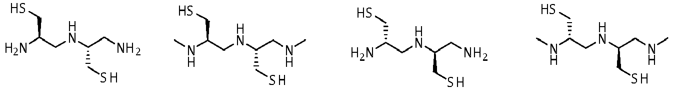

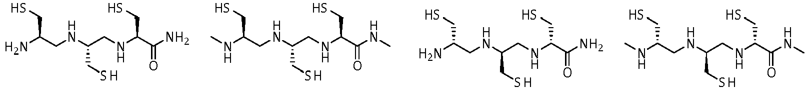

A ccordi ng to another aspect of the present i nventi on, the method for prepari ng the compound of the present i nventi on is provided:
preferably, as in route 1: 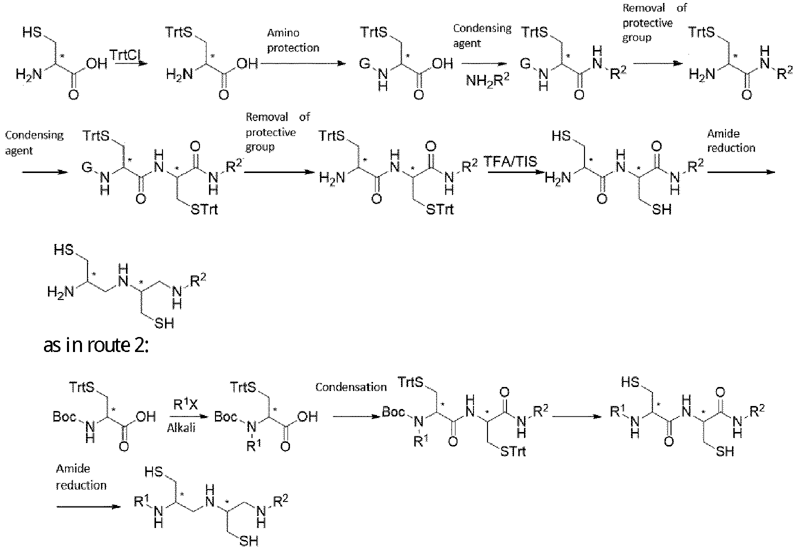
wherein R¹ and R² may be the same or different and are selected from: hydrogen, substituted or unsubstituted C₁-C₅ alkyl or heteroalkyl;
preferably, the amino-protecting group G is selected from: tert-butyloxycarbonyl, fluorenylmethoxycarbonyl (Fmoc), benzyloxycarbonyl, benzyl, and the like;
X is a halogen;
preferably, the condensing agent is selected from Carbonyldiimidazole (CDI), 1-hydroxybenzotriazole (HOBT), 2-(7-oxidized benzotriazole)-N,N,N',N'-tetramethyluronium hexafluorophosphate (HATU), N, N'-dicyclohexylcarbodiimide(DCC), BOP, PyBOP, HBTU, TBTU, EDCI, etc.;
the corresponding amino-protecting group may be removed by an acid method, such as formic acid, hydrochloric acid and trifluoroacetic acid or an alkali method, such as piperidine, aqueous ammonia and tri ethyl ami ne, or the correspondi ng ami no- protect! ng group may also be removed by hydrogenati on; and
amide can be reduced to amine by borane, NaBH₄, NaB H₄-Lewis acid, lithium aluminum hydride and other methods.

According to a further aspect of the present invention, a pharmaceutical composition comprising the compound of the present invention and a derivative thereof are provided;
preferably, the above pharmaceutical composition further comprises one or more pharmaceutically acceptable vehicles, carriers, adjuvants, auxiliaries or diluents;
preferably, the dosage forms of the pharmaceutical composition comprise but are not limited to: injections, emulsions, microemulsions, submicro-emulsions, nanoparticles, tablets, capsules, pills, inhalants, lozenges, gels, powder, suppositories, suspensions, creams, jellies, sprays, etc.;
preferably, the pharmaceutical composition can be administered by means of, but not limited to: subcutaneous injection, intramuscular injection, intravenous injection, oral administration, rectal administration, vaginal administration, nasal administration, transdermal administration, subconjunctival administration, intraocular administration, eyelid administration, retrobulbar administration, retinal administration, choroidal administration, intrathecal injection, and the like.

According to a further aspect of the present invention, the use of the compound of the present invention (e.g., a compound of formula I) and the pharmaceutical composition thereof in the preparation of drugs and/or cosmetics for the treatment and/or prevention of radiation damage or chemotherapy damage is provided.

The radiation comprises ionizing radiation, non-ionizing radiation or a combination of various types of radi ati on;
the ionizing radiation includes but is not limited to: alpha rays, beta rays, gamma rays, X rays, and neutron radi ati on;
the radiation damage comprises direct damage and indirect damage caused by radiation; preferably, the radiation damage comprises radiation-induced reduction of peripheral blood leukocytes, platelets and erythrocytes in mammal s;
the chemotherapeutic drugs refer to the anti-tumor drugs that act on DNA, RNA, and tubulin, and that are vital to the survival of cells;
preferably, the use comprises the use of the above compound and the pharmaceutical composition thereof in the preparation of drugs and/or cosmetics for the treatment and/or prevention of sunburn damage, more preferably, the use comprises the use of the above compound and the pharmaceutical composition thereof in the preparation of cosmetics for the treatment and/or prevention of sunburn damage.

The compound of the present invention or a pharmaceutical composition thereof can be used alone as a radiation damage protection and treatment drug, or can be used in combination with a known radioprotectant, or can be combined with radiation therapy or chemotherapy to treat tumors, thereby reducing the adverse reactions of radiotherapy to surrounding tissues and organs and even the whole body, and alleviating and preventing the adverse reactions caused by radiotherapy.

The present invention also provides the use of the above compound or the pharmaceutical composition thereof in the preparati on of anti-tumor drugs.

The present invention provides a novel stable compound which has the effects of reducing biological damage caused by ionizing radiation, extending the survival period and survival rate of the radiated animals, and significantly alleviating the side effects of radiotherapy, and has a low toxicity. The present invention opens up a new way for protection and treatment of ionizing radiation damage, wherein the radiation damage comprises direct damage and indirect damage caused by radiation; including radiation-induced reduction of peripheral blood leukocytes, platelets and erythrocytes in mammals. The chemotherapeutic drugs refer to the anti-tumor drugs that act on DNA, RNA, and tubuli n, and that are vital to the survival of cells. The compounds provided by the present invention and derivatives thereof can also be used in combination with known radioprotectants.

### Brief Description of the Drawings

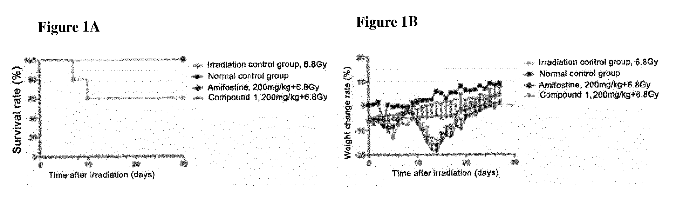

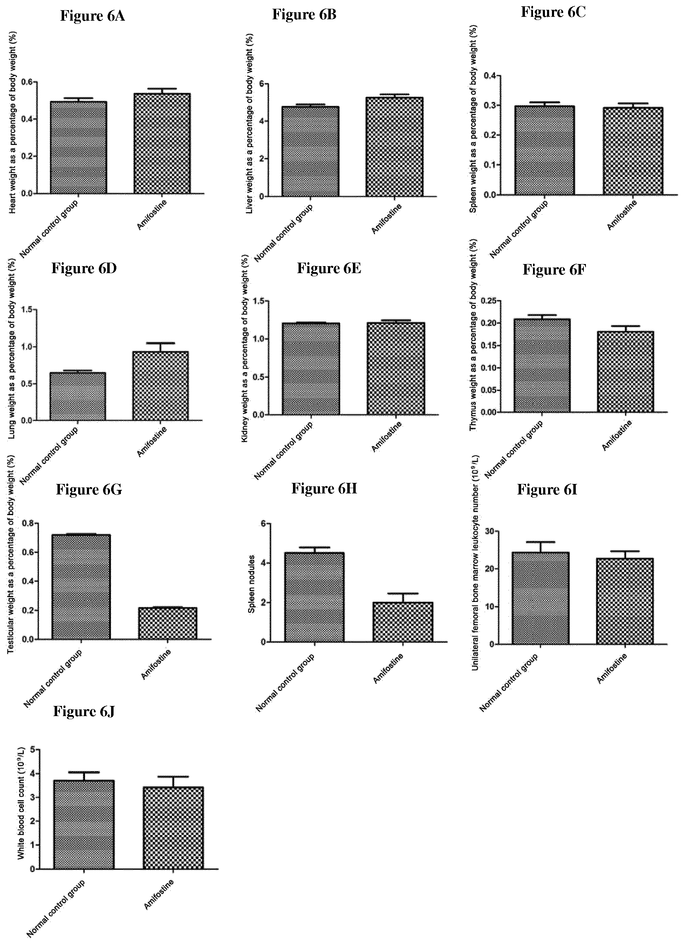

F i gures 1A and 1B show the effect of compound 1 on the mice irradiated with 6.8Gy ∴rays, wherein Figure 1A shows the effect of compound 1 on 30-day survival rate of mice irradiated with 6.8Gy ∴rays, Figure 1B shows the effect of compound 1 on the body weight of mice irradiated with 6.8Gy ∴rays.

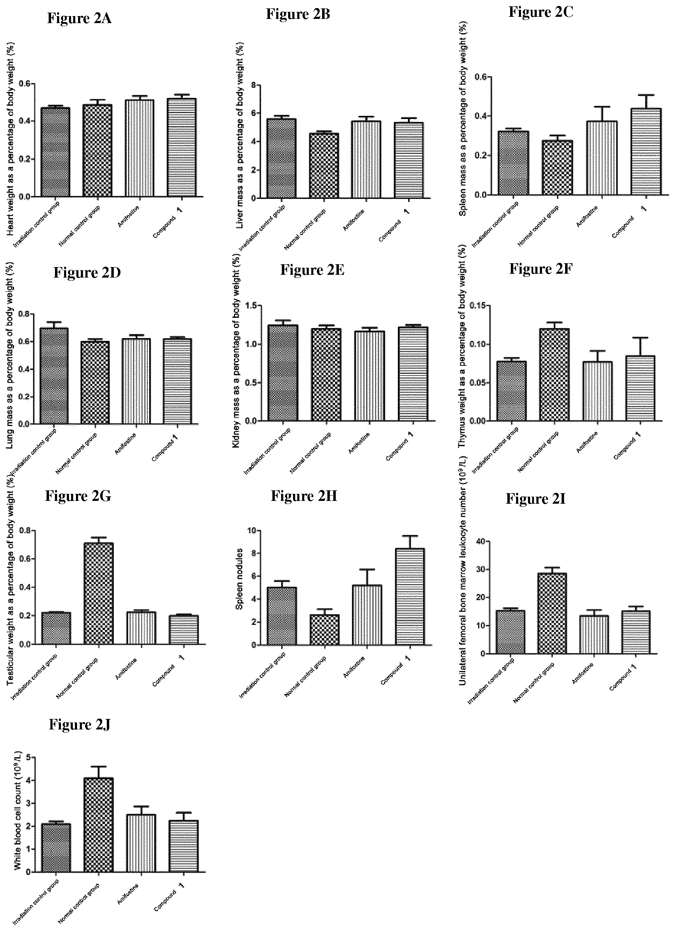

Figures 2A-2J show the effect of compound 1 on organs and white blood cells in mice 30 days after irradiation with 6.8Gy ∴rays, wherein Figure 2A shows the effect of compound 1 on heart in mice 30 days after irradiation with 6.8Gy ∴rays, Figure 2B shows the effect of compound 1 on liver in mice 30 days after irradiation with 6.8Gy ∴rays, Figure 2C shows the effect of compound 1 on spleen in mice 30 days after irradiation with 6.8Gy ∴rays, Figure 2D shows the effect of compound 1 on lung in mice 30 days after irradiation with 6.8Gy ∴rays, Figure 2E shows the effect of compound 1 on kidney in mice 30 days after irradiation with 6.8Gy ∴rays, Figure 2F shows the effect of compound 1 on thymus in mice 30 days after irradiation with 6.8Gy ∴rays, Figure 2G shows the effect of compound 1 on testis in mice 30 days after irradiation with 6.8Gy ∴rays, Figure 2H shows the effect of compound 1 on splenic nodules in mice 30 days after irradiation with 6.8Gy ∴rays, Figure 21 shows the effect of compound 1 on unilateral femoral bone marrow leukocytes in mice 30 days after irradiation with 6.8Gy ∴rays, Figure 2J shows the effect of compound 1 on white blood cells in blood of mice 30 days after irradiation with 6.8Gy ∴rays.

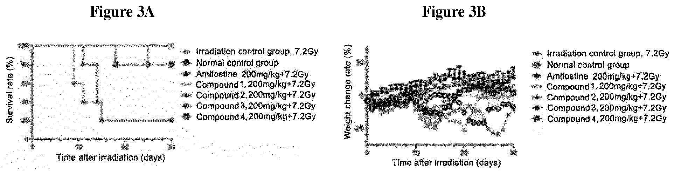

Figures 3A and B show the effect of compounds 1-4 on the survival rate and body weight of mice 30 days after irradiation with 7.2Gy ∴rays, wherein Figure 3A shows the effect of compounds 1-4 on survival rate of mice 30 days after irradiation with 7.2Gy ∴rays, Figure 3B shows the effect of compounds 1-4 on the body weight of mice irradiated with 7.2Gy ∴rays.

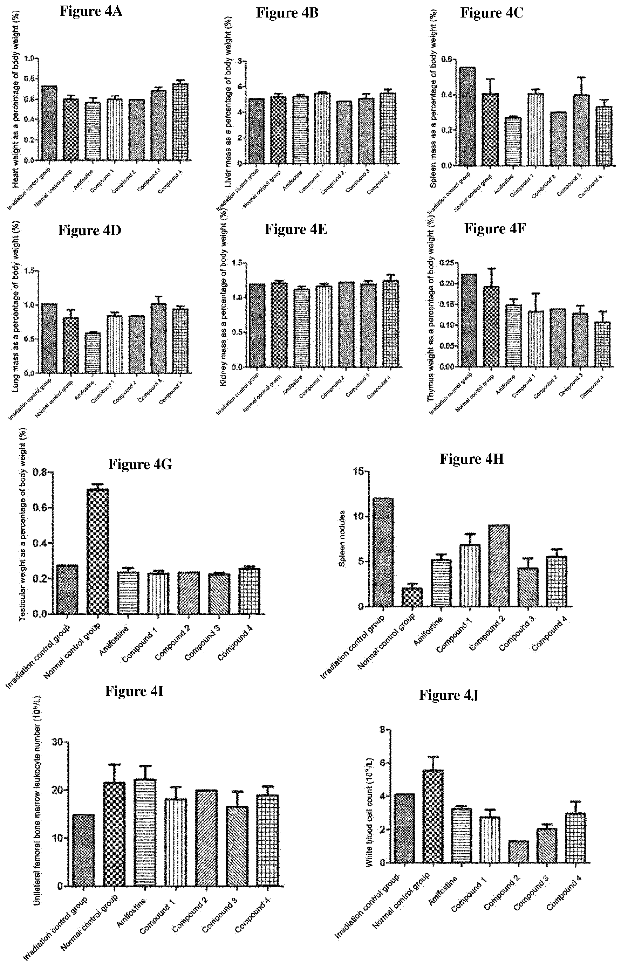

Figures4A-4J show the effect of compounds 1-4 on organs and white blood cells in mice 30 days after irradiation with 7.2Gy ∴rays, wherein Figure 4A shows the effect of compounds 1-4 on heart in mice 30 days after irradiation with 7.2Gy ∴rays, Figure 4B shows the effect of compounds 1-4 on liver in mice 30 days after irradiation with 7.2Gy ∴rays, Figure 4C shows the effect of compounds 1-4 on spleen in mice 30 days after irradiation with 7.2Gy ∴rays, Figure 4D shows the effect of compounds 1-4 on lung in mi ce 30 days after irradiation with 7.2Gy ∴rays, Figure4E shows the effect of compounds 1-4 on kidney in mice 30 days after irradiation with 7.2Gy ∴rays, Figure4F shows the effect of compounds 1-4 on thymus in mice 30 days after irradiation with 7.2Gy ∴rays, Figure 4G shows the effect of compounds 1-4 on test is in mice 30 days after irradiation with 7.2Gy ∴rays, Figure 4H shows the effect of compounds 1-4 on splenic nodules in mice 30 days after irradiation with 7.2Gy ∴rays, Figure 4I shows the effect of compounds 1-4 on unilateral femoral bone marrow leukocytes in mice 30 days after irradiation with 7.2Gy ∴rays, Figure4J shows the effect of compounds 1-4 on white blood cells in blood of mice 30 days after irradiation with 7.2Gy ∴rays.

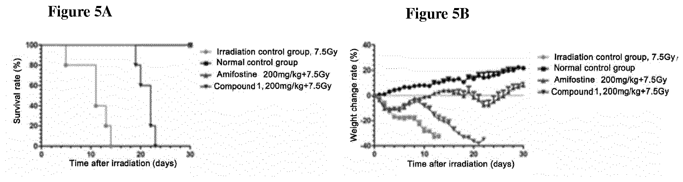

Figures 5A and 5B show the effect of compound 1 on survival rate and body weight of mice 30 days after irradiation with 7.5Gy ∴rays, wherein Figure 5A shows the effect of compound 1 on survival rate of mice 30 days after irradiation with 7.5Gy ∴rays, Figure 5B shows the effect of compound 1 on the body weight of mice irradiated with 7.5Gy ∴rays.
F i gures 6A-6J show the effect of ami fosti ne on organs and white blood cells in mi ce 30 days after irradiation with 7.5Gy ∴rays, wherein Figure 6A shows the effect of amifostine on heart in mice 30 days after irradiation with 7.5Gy ∴rays, Figure 6B shows the effect of amifostine on liver in mice 30 days after irradiation with 7.5Gy ∴rays, Figure 6C shows the effect of amifostineon spleen in mice 30 days after irradiation with 7.5Gy .-.rays, Figure 6D shows the effect of amifostine on lung in mice 30 days after irradiation with 7.5Gy ∴ rays, Figure 6E shows the effect of amifostine on kidney in mice 30 days after irradiation with 7.5Gy ∴rays, Figure 6F shows the effect of amifostine on thymus in mice 30 days after irradiation with 7.5Gy ∴rays, Figure 6G shows the effect of amifostine on testis in mice 30 days after irradiation with 7.5Gy ∴rays, Figure 6H shows the effect of amifostine on splenic nodules in mice 30 days after irradiation with 7.5Gy ∴rays, Figure 61 shows the effect of amifostine on unilateral femoral bone marrow leukocytes in mice 30 days after irradiation with 7.5Gy ∴rays, Figure 6J shows the effect of amifostine on white blood cells in blood of mice 30 days after irradiation with 7.5Gy ∴rays.
Figures 7A-7F show the effects of irradiation on peripheral blood, wherein Figure 7A shows the peripheral white blood cell (WBC) count, Figure 7B shows the peripheral red blood cell (RBC) count, Figure 7C shows the peripheral hemoglobin (HGB) concentration, Figure 7D shows the peripheral platelet (PLT) count, Figure 7E shows the peripheral blood lymphocyte ratio (LY %), Figure 7F shows the peripheral blood neutrophil ratio (NE%).
Figures 8A-8E show the effect of irradiation on bone marrow cells, wherein Figure 8A shows the number of leukocytes in bone marrow, Figure 8B shows the ratio of hematopoietic stem cell (LSK) in bone marrow cells, Figure 8C shows the ratio of hematopoietic progenitor cells (H PC) in bone marrow cells, Figure 8D shows the ratio of C D34-LSK in bone marrow cells, Figure 8E shows the ratio of CD34+LSK in bone marrow cells.
Figures 9A and 9B show the effect of irradiation on the levels of reactive oxygen species (ROS) in LSK cells and the levels of ROS in H PC cells, wherein Figure 9A shows the levels of reactive oxygen species (ROS) in LSK cells, Figure 9B shows the levels of ROS in HPC cells.
Figure 10 shows the 30-day survival rate of Example 4 and ami fosti ne in mice exposed to local abdominal radiation of 18Gy.
Figure 11 shows the protective effect of Example 4 on the i ntesti nal tract
Figure 12 shows the effect of Example4 and amifostine on radiation pneumonia.
Figure 13 shows the effect of stereoisomerism of the compound on the survival rate.

### Detailed Description of Embodiments

According to the present invention, the term 'radiation damage_ in the present invention refers to the injury caused by various rays in the electromagnetic spectrum, such as microwave, infrared ray, visible light, ultraviolet ray, X ray, beta ray and gamma ray. Neutron or proton beam irradiation can also cause such damage.

The term 'pharmaceutically acceptable salt_ refers to any salt (generally referred to as non-toxic) that is physiologically compatible when used in an appropriate manner for treatment, use, or especially in humans and/or mammals. Unless otherwise stated, salts of acidic groups which may be present in the compounds of the present invention (for example, but not limited to, potassium salts, sodium salts, magnesium salts, calcium salts, etc.) or salts of basic groups (for example, but not limited to, sulfates, hydrochlorides, phosphates, nitrates, carbonates, etc.).

The term 'solvate_ refers to a complex compound of molecules of solute or ions in a solution, formed by attracting neighboring solvent molecules through intermolecular forces, such as coulomb force, van der Waals force, charge transfer force, and hydrogen bond. In one embodi ment, the solvent is water, that is, the compound of the invention forms a hydrate.

Depending on the substituent, the compound in formula (I) may be in the form of optically active isomers or mixtures of isomers of different compositions, and the mixtures may be separated by conventional means if appropri ate. The present i nventi on provi des pure i somers and mi xtures of i somers, methods for preparati on and uses, and compositions comprising them. For the sake of simplicity, it is referred to below as a compound of formula (I), which refers to both pure optical isomers and, where appropriate, mixtures of different proportions of isomers.

A person skilled in the art will have a better understandi ng of the above and other purposes, advantages and characteristics of the present invention according to the following and the detailed description of the specific embodiments of the present invention in conjunction with the accompanying drawings. A person skilled in the art will have a better understanding of the above and other purposes, advantages and characteristics of the present invention according to the following and the detailed description of the specific embodiments of the present invention in conjunction with the accompanying drawings.

### Synthesis

Suitable solvents commonly used in organic reactions can be used in the following various steps of the preparation method of the present invention, such as, but not limited to, aliphatic and aromatic, optionally hydrocarbon or halogenated hydrocarbons (e.g., pentane, hexane, heptane, cyclohexane, petroleum ether, gasoline, volatile oil, benzene, toluene, xylene, dichloromethane, dichloroethane, chloroform carbon tetrachloride, chlorobenzene and o-dichlorobenzene); aliphatic and aromatic, optional alcohols (e.g., methanol, ethanol, propanol, isopropanol, tertiary butanol, ethyleneglycol, etc.), ethers(e.g., diethyl ether and dibutyl ether, ethylene glycol dimethyl ether and diglyme, tetrahydrofuran and dioxane, etc.), esters (e.g., methyl acetate or ethyl acetate, etc.), nitriles (e.g., acetonitrile or propionitrile, etc.), ketone (e.g., acetone, methyl ethyl ketone, etc.), amides (e.g., dimethylformamide, dimethylacetamide, N-methylpyrrolidone, etc.); as well as dimethyl sulfoxide, tetramethylene sulfone and hexamethyl phosphoric triamide and N,N-di methyl propylene urea (DMPU), etc.

### Synthesis Examples:

The present invention may be further explained by the following examples which do not imply any I i mi tati on to the present i nventi on.

### Example 1: Synthesis of (R)-2-amino-N-((R)-1-amino-3-mercapto-1-oxopropan-2-yl)-3-mercaptopropanamide tri fl uoroacetate:

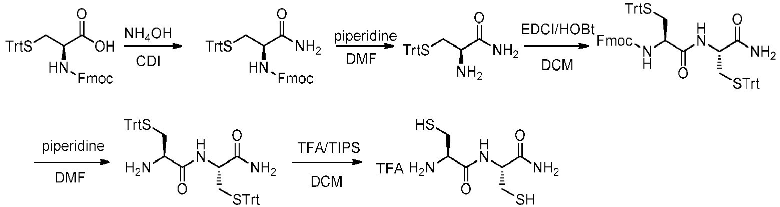

### Step 1: Synthesis of (9H-fluoren-9-yl) methyl (R)-(1-amino-1-oxo-3-(tritylthio)propan-2-yl) carbamate:

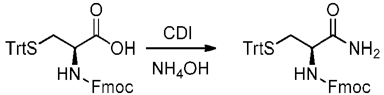

The compound (R)-2-(((9H-fluoren-9-yl)methoxy)carbonyl)amino)-3-(tritylthio) propionic acid (10 g, 17.07 mmol) was dissolved in tetrahydrofuran (50ml). N,N'-carbonyldiimidazole (5.59g, 34.48mmol) was added at 0- 5 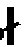 C. After stirring for 2 hours under nitrogen protection, aqueous ammonia (5ml, 68.28mmol) was added, and reacted at 0-5 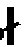 C for 30 minutes. After the reaction was completed as detected by TLC, 2 M hydrochloric acid (60ml) was added for quenching. The reaction mixture was extracted with ethyl acetate, the organic phase was washed with a saturated saline, then dried with sodium sulfate, and concentrated to obtain a crude product After adding anhydrous methanol (20ml) and stirring at room temperature overnight, white solids were precipitated, and filtered to obtain the product in the filter cake. The methanol phasewas concentrated and separated by column chromatography (methanol: dichloromethane: 1%-5%) to obtain the target product as a white solid (9.3g, yield: 93.19%). ¹H NMR (400MHz, DMSO-d6) 7.89(d, 2H), 7.74(d, 2H), 7.58(d, 1H), 7.3(m, 18H), 7.11(s, 1H), 4.24(m, 3H), 4.01(m, 1H), 2.39(m, 2H).

### Step 2: Synthesis of (R)-2-amino-3-(tritylthio) propionamide:

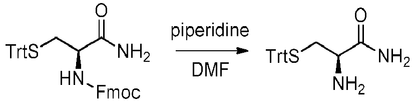

The compound (9H-fluoren-9-yl) methyl (R)-(1-amino-1-oxo-3-(tritylthio)propan-2-yl) carbamate (4g, 6.84mmol) was dissolved in N,N-dimethylformamide (20ml). Piperidine (0.14ml, 1.368mmol) was added and reacted at room temperature for 4 hours. After the reaction was completed as detected by TL C, the reacti on mixture was washed with a saturated saline and extracted with dichloromethane. The organic phase was dried with sodium sulfate, concentrated, and separated by column chromatography (methanol: dichloromethane = 1%-5%) to obtain the target product as a yellow oil (2.3g, yield: 92%). ¹H NMR (400M Hz, DMSO-d6) 7.29(m, 17H), 3.08(d, 1H), 2.33(d, 1H), 2.18(s, 1H), 1.85(s, 2H).

### Step3: Synthesis of (9H-fluoren-9-yl) methyl ((R)-1-(((R)-1-amino-1-oxo-3-(tritylthio)propan-2-yl)amino)-1-oxo-3-(tritylthio)propan-2-yl) carbamate:

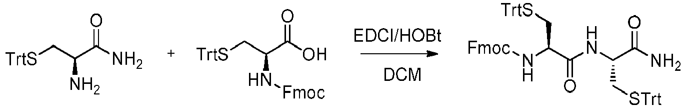

The compound (R)-2-((((9H-fluoren-9-yl)methoxy)carbonyl)amino)-3-(tritylthio) propanoic acid (1.29g, 2.21 mmol) was dissolved in dichloromethane (15ml). 1-hydroxybenzotriazole (448rrg, 3.315mmol) and E DCI (635mg, 3.315mmol) were added, and stirred at room temperature for 5min. (9H-fluoren-9-yl) methyl (R)-(1-amino-1-oxo-3-(tritylthio)propan-2-yl) carbamate (960mg, 2.65mmol) was added and reacted at room temperature for 30 minutes. After the reaction was completed as detected by T L C, the mixture was washed with a saturated saline and extracted with dichloromethane. The organic phase was dried with sodium sulfate, concentrated, and separated by column chromatography (methanol : dichloromethane: 1%-5%) to obtain the product as a white solid (2.05g, yield: 99.76%). ¹H NMR (400MHz, DMSO-d6) 7.89(m, 3H), 7.7(m, 3H), 7.4(m, 2H), 7.24(m, 34H), 4.21(m, 4H), 4.10(m, 1H), 2.33(m, 4H).

### Step4: Synthesis of (R)-2-amino-N-((R)-1-amino-1-oxo-3-(tritylthio) propan-2-yl)-3-(tritylthio) propionamide:

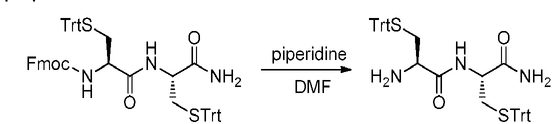

The compound (9H-fluoren-9-yl) methyl ((R)-1-(((R)-1-amino-1-oxo-3-(tritylthio)propan-2-yl) amino)-1-oxo-3-(tritylthio)propan-2-yl) carbamate (2.05g, 2.2mmol) was dissolved in N,N-dimethylformamide (20ml). Piperidine (0.04ml, 0.44mmol) was added and stirred at room temperature for 4 hours. After the reaction was completed as detected by T L C detecti on, the reacti on rri xture was washed with a saturated sal i ne and extracted with dichloromethane. The organic phase was dried with sodium sulfate, concentrated, and separated by column chromatography (methanol : dichloromethane: 1%-5%) to obtain the product as a white solid (600mg, yield: 38.54%). ¹H NMR (400MHz, DMSO-d6) 8.1(s,1H), 7.37-7.14(m, 32H), 4.23(m, 1H), 3.17(m,1H), 2.39(dd, 1H), 2.33(d, 2H), 2.19(m, 1H).

### Step 5: Synthesis of (R)-2-amino-N-((R)-1-amino-3-mercapto-1-oxopropan-2-yl)-3-mercaptopropanamide trifluoroacetate:

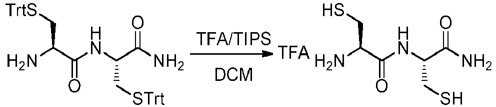

The compound (R)-2-amino-N-((R)-1-amino-1-oxo-3-(tritylthio)propan-2-yl)-3-(tritylthio) propionamide (150mg, 0.21 mmol) wasdissolvedindichloromethane(5ml). Triisopropylsilane(0.11ml, 0.525mmol) and trifluoroacetic acid (1 ml) were added at 0 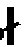 C under a nitrogen atmosphere and stirred in an ice bath for 2 hours. After the reaction was completed as detected by T L C, the mixture was concentrated, diethyl ether was added, and stirred in the ice bath. White solids were precipitated, filtered and dried to obtain the product (60mg, yield: 84.7%). ¹H NMR (400MHz, DMSO-d6) 8.72(d, 1H), 8.23(s, 3H), 7.56(s, 1H), 7.32(s, 1H), 4.43(m, 1H), 4.09(m, 1H), 2.99(d, 2H), 2.89(m, 1H), 2.74(m, 1H); HESI:224.05 [M +H]+.

### Example 2: Synthesis of (R)-3-mercapto-N-((R)-3-mercapto-1-(methylamino)-1-oxopropan-2-yl)-2-(methylamino) propionamide trifluoroacetate:

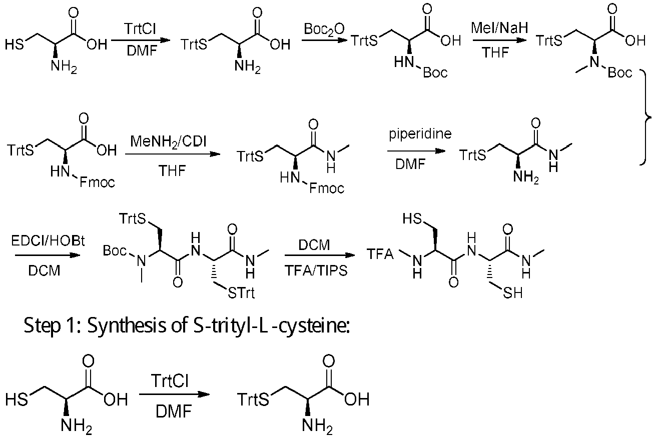

The compound L-cysteine hydrochloride (10g, 63.45rrmol) was dissolved in N,N-dimethylformamide (120ml). Triphenylchloromethane (19.46g, 69.795mmol) was added, heated to 60-65 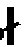 C, and reacted for 8h. After the reacti on was completed as detected by T LC, the reaction was cooled to room temperature, and 10% sodium acetate solution (300ml) was added. White solids were then precipitated and filtered. Filter residue was washed with pure water (300ml), then washed with acetone (200ml), and dried to obtain the product as a white solid (17.56g, yield: 76.15%).¹H NMR (400MHz, DMSO-d6) 7.28(m, 18H), 2.92(dd, 1H), 2.59(dd, 1H), 2.41(dd, 1H).

### Step 2: Synthesis of N-(tert-butoxycarbonyl)-S-trityl-L-cysteine:

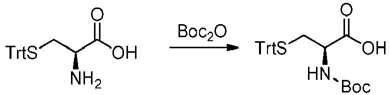

The compound S-trityl-L-cysteine (5g, 13.76mmol) was dissolved in a mixture of dioxane (40ml), water (20ml) and 1M sodium hydroxide solution (14ml), and stirred in an ice bath. Boc-anhydride (3.5ml, 15.14mmol) was added, then reacted until the mixture was naturally warmed to room temperature, and stirred for 8 hours. After the reaction was completed as detected by T L C, the reaction mixture was concentrated to 20-25ml. Ethyl acetate was added, and the sodi um bisulfate sol uti on was added dropwi se under the ice bath while sti rri ng. After pH was adjusted to 2-3, ethyl acetate was used for extraction. The organic layer was washed with a saturated saline, then dried with sodium sulfate, concentrated, and separated by column chromatography (methanol : dichloromethane: 1%-5%) to obtain the product as a white solid (5.5g, yield :86.21%). ¹H NMR (400MHz, DMSO-d6) 7.26(m, 16H), 3.78(d, 1H), 2.51(m, 1H), 2.36(dd, 1H), 1.4(d, 9H).

### Step3: Synthesis of N-(tert-butoxycarbonyl)-N-methyl-S-trityl-L-cysteine:

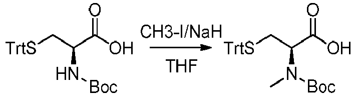

The compound N-(tert-butoxycarbonyl)-S-trityl-L-cysteine (2.1g, 4.53mmol) was dissolved in anhydrous tetrahydrofuran (6ml). Sodium hydride (436rrg, 10.9mmol) was dissolved in anhydrous tetrahydrofuran (14ml). The solution of amino acid in tetrahydrofuran was added dropwi se to the solution of sodi um hydri de in tetrahydrofuran in an ice bath. Then, methyl iodide (0.93ml, 14.95mmol) was slowly added dropwise and stirred overnight. After the reaction was completed as detected by TL C, phosphate buffer at pH=7 was added for quenchi ng. pH was adjusted to 6-7 with a saturated ammonium chloride sol ution, and was extracted with ethyl acetate. The organic layer was washed with a saturated saline, then dried with sodium sulfate, concentrated, and separated by column chromatography (methanol : dichloromethane: 1%-5%) to obtain the product as a white solid (1.3g, yield: 60.19%). ¹H NMR (400MHz, DMSO-d6) 7.3(m, 15H), 3.75(s, 1H), 2.8(s, 1H), 2.66(d, 4H), 1.4(d, 9H).

### Step4: Synthesis of (9H-fluoren-9-yl) methyl (R)-(1-(methylamino)-1-oxo-3-(tritylthio)propan-2-yl) carbamate:

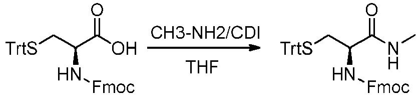

The compound (R)-2-(((9H-fluoren-9-yl)methoxy)carbonyl)amino)-3-(tritylthio) propionic acid (10g, 17.07mmol) was dissolved in tetrahydrofuran (50ml). N,N'-carbonyldiimidazole (5.59g, 34.48mmol) was added at 0-5 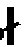 C. After stirring for 2 hours under nitrogen atmosphere, methylamine (3.03ml, 68.28mmol) was added, and reacted at 0-5 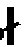 C for 2 hours. After the reactants were consumed, 2M hydrochloric acid (60ml) was added for quenching, and the reaction mixture was extracted with dichloromethane. The organic layer was washed with a saturated saline, then dried with sodium sulfate and concentrated to obtain a crude product. Methanol (20ml) was added and stirred overnight at room temperature. White solids were precipitated, and filtered to obtain the product in filter residue. The methanol phase was concentrated, and separated by column chromatography (methanol: dichloromethane: 1%-5%) to obtain a white solid (9.44g, yield: 92.37%). ¹H NMR (400MHz, DMSO) 7.89(d, 2H), 7.81(d, 1H), 7.74(d, 2H), 7.66(d, 1H), 7.41(t, 2H), 7.29(m, 17H), 4.31(d, 1H), 4.22(t, 2H), 4.00(d, 1H), 2.53(d, 3H), 2.39(d, 2H).

### Step 5: Synthesis of (R)-2-amino-N-methyl-3-(tritylthio) propionamide

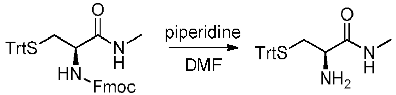

The compound (9H-fluoren-9-yl) methyl (R)-(1-(methylamino)-1-oxo-3-(tritylthio)propan-2-yl) carbamate (2g, 3.34mmol) was dissolved in N,N-dimethylformamide (20ml). Piperidine (0.07ml, 0.668mmol) was added and reacted at room temperature for 4 hours. After the reaction was completed as detected by T L C, the reaction mixture was washed with a saturated saline and extracted with dichloromethane. The organic phase was dried with sodium sulfate, concentrated, and separated by column chromatography (methanol : dichloromethane: 1%-5%) to obtain the product as a yellowish white solid (879rrg, yield: 69.76%). ¹H NMR (400MHz, CDCl₃) 7.77(d, 1H), 7.29(m, 15H), 3.08(m, 1H), 2.55(d. 3H), 2.37(dd, 1H), 2.19(dd, 1H), 1.80(s, 2H).

### Step 6: Synthesis of tert-butyl methyl ((R)-1-(((R)-1-(methylamino)-1-oxo-3-(tritylthio)propan-2-yl)amino)-1-oxo-3-(tritylthio)propan-2-yl) carbamate:

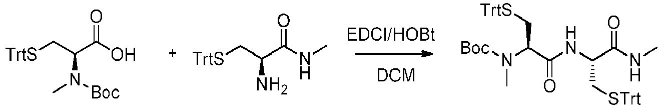

The compound N-(tert-butoxycarbonyl)-N-methyl-S-trityl-L-cysteine (150g, 0.314mmol) was dissolved in dichloromethane(5ml). 1-hydroxybenzotriazole(63.7rrg, 0.471mmol) and EDCI (90.3mg, 0.471mmol) were added, and stirred at room temperature for 5min. (R)-2-amino-N-methyl-3-(tritylthio) propanamide (141.9mg, 0.377mmol) was added and stirred at room temperature for 30 minutes. After the reaction was completed as detected by T L C, the mixture was washed with a saturated saline and extracted with dichloromethane. The organic phase was dried with sodium sulfate, then concentrated and purified with TL C (dichloromethane: methanol: 15: 1) to obtain the product as a white solid (260rrg, yield: 99.2%). ¹H NMR (400MHz, CDCl₃) 7.39(m, 12H), 7.22(m, 20H), 4.1(d, 1H), 3.95(s, 1H), 2.61(dd, 10H), 1.39(s, 9H).

### Step 7: Synthesis of (R)-3-mercapto-N-((R)-3-mercapto-1-(methylamino)-1-oxopropan-2-yl)-2-(methylamino) propionami de tri fl uoroacetate:

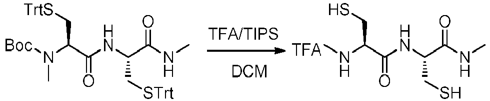

The compound tert-butyl methyl ((R)-1-(((R)-1-(methylamino)-1-oxo-3-(tritylthio)propan-2-yl)amino)-1-oxo-3-(tritylthio)propan-2-yl) carbamate (1.1g, 1.32mmol) was dissolved in dichloromethane: trifluoroacetic acid : triisopropylsilane (50 : 47 : 3 by volume) (25ml), stirred at room temperature for 5min. After the reaction was completed as detected by T L C, the mixture was concentrated, diethyl ether was added, and stirred in an ice bath. White solids were precipitated, filtered and dried to obtain the product (400mg, yield: 86.9%). ¹H NMR (400MHz,MeOD) 4.5(m, 1H), 4.07(t, 1H), 3.18-2.96(m, 3H), 2.82(m, 1H), 2.78(s, 3H), 2.74(s, 3H); HESI:252.08[M +H]⁺.

### Example3: Synthesis of (R)-2-amino-N-((R)-1-(((R)-1-amino-3-mercapto-1-oxopropan-2-yl)amino)-3-mercapto-1-oxopropan-2-yl)-3-mercaptopropanamide trifluoroacetate:

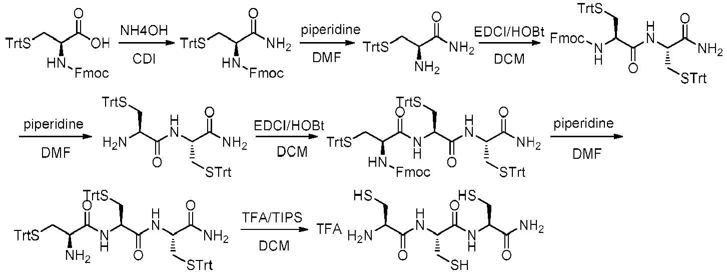

### Step 1: Synthesis of (9H-fluoren-9-yl) methyl (R)-(1-amino-1-oxo-3-(tritylthio)propan-2-yl) carbamate:

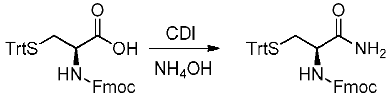

The compound (R)-2-(((9H-fluoren-9-yl)methoxy)carbonyl)amino)-3-(tritylthio) propionic acid (10g, 17.07mmol) was dissolved in tetrahydrofuran (50ml). N,N'-carbonyldiimidazole (5.59g, 34.48mmol) was added at 0-5 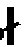 C. After stirring for 2 hours under nitrogen atmosphere, aqueous ammonia (5ml, 68.28mmol) was added, and reacted at 0-5 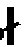 C for 30 minutes. After the reactants were consumed, 2M hydrochloric acid (60ml) was added for quenching. The reaction mixture was extracted with ethyl acetate. The organic layer was washed with a saturated saline, then dried with sodium sulfate, evaporated under reduced pressure to remove the solvent, thereby obtaining a crude product. After adding anhydrous methanol (20ml) and stirring at room temperature overnight, white solids were precipitated, and filtered to obtain a product in the filter residue. The methanol phase was concentrated and separated by column chromatography (methanol: dichloromethane: 1%-5%) to obtain a white solid (a total of 9.3g products, yield: 93.19%). ¹H NMR (400MHz, DMSO-d6) 7.89(d, 2H), 7.74(d, 2H), 7.58(d, 1H), 7.3(m, 18H), 7.11(s, 1H), 4.24(m, 3H), 4.01(m, 1H), 2.39(m, 2H).

### Step 2: Synthesis of (R)-2-amino-3-(tritylthio) propionamide:

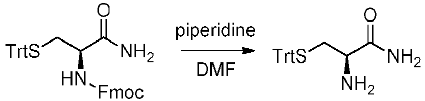

The compound (9H-fluoren-9-yl) methyl (R)-(1-amino-1-oxo-3-(tritylthio)propan-2-yl) carbamate (4g, 6.84mmol) was dissolved in N,N-dimethylformamide (20ml). Piperidine (0.14ml, 1.368mmol) was added and reacted at room temperature for 4 hours. After the reaction was completed as detected by TL C, the reacti on mixture was washed with a saturated saline and extracted with dichloromethane. The organic phase was dried with sodium sulfate, then concentrated, and separated by column chromatography (methanol : dichloromethane: 1%-5%) to obtain the product as a yellow oil (2.3g, yield: 92%).¹H NMR (400MHz, DMSO-d6) 7.29(m, 17H), 3.08(d, 1H), 2.33(d, 1H), 2.18(s, 1H), 1.85(s, 2H).

### Step3: Synthesis of (9H-fluoren-9-yl) methyl ((R)-1-(((R)-1-amino-1-oxo-3-(tritylthio)propan-2-yl)amino)-1 - oxo-3-(tritylthio)propan-2-yl) carbamate:

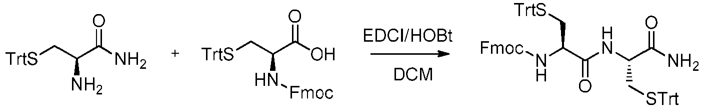

The compound (R)-2-((((9H-fluoren-9-yl)methoxy)carbonyl)amino)-3-(tritylthio) propanoic acid (1.29g, 2.21mmol) was dissolved in dichloromethane (15ml). 1-hydroxybenzotriazole (448mg, 3.315mmol) and E DCI (635mg, 3.315mmol) were added, and stirred at room temperature for 5min. (9H-fluoren-9-yl) methyl (R)-(1-amino-1-oxo-3-(tritylthio)propan-2-yl) carbamate (960mg, 2.65mmol) was added and reacted at room temperature for 30 minutes. After the reaction was completed as detected by T L C, the mixture was washed with a saturated saline and extracted with dichloromethane. The organic phase was dried with sodium sulfate, then concentrated, and separated by column chromatography (methanol : dichloromethane: 1%-5%) to obtain the product as a white solid (2.05g, yield: 99.76%).¹H NMR (400MHz, DMSO-d6) 7.89(m, 3H), 7.7(m, 3H), 7.4(m, 2H), 7.24(m, 34H), 4.21(m, 4H), 4.10(m, 1H), 2.33(m, 4H).

### Step4: Synthesis of (R)-2-amino-N-((R)-1-amino-1-oxo-3-(tritylthio) propan-2-yl)-3-(tritylthio) propionamide:

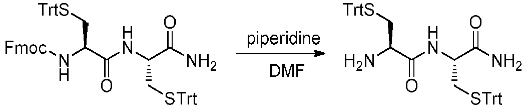

The compound (9H-fluoren-9-yl) methyl ((R)-1-(((R)-1-amino-1-oxo-3-(tritylthio)propan-2-yl) amino)-1-oxo-3-(tritylthio)propan-2-yl) carbamate (2.05g, 2.2mmol) was dissolved in N,N-dimethylformamide (20ml). Piperidine (0.04ml, 0.44mmol) was added and stirred at room temperature for 4 hours. After the reaction was completed as detected by T L C detecti on, the reacti on rri xture was washed with a saturated saline and extracted with dichloromethane. The organic phase was dried with sodium sulfate, then concentrated, and separated by column chromatography (methanol: dichloromethane: 1%-5%) to obtain a white solid (600mg, yield: 38.54%).¹H NMR (400MHz, DMSO-d6) 8.1(s,1H), 7.37-7.14(m, 32H), 4.23(m, 1H), 3.17(m,1H), 2.39(dd, 1H), 2.33(d, 2H), 2.19(m, 1H).

### Step 5: Synthesis of (9H-fluoren-9-yl) methyl ((4R,7R,10R)-4-carbamoyl-6,9-dioxo 1,1,1,13,13,13-hexaphenyl-7-((tritylthio)methyl) -2,12-dithia-5,8-diazatridec-10-yl) carbamate:

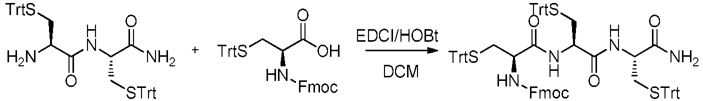

The compound (R)-2-((((9H-fluoren-9-yl)methoxy)carbonyl)amino)-3-(tritylthio) propanoic acid (688.21rrg, 1.175mmol) was dissolved in dichloromethane (5ml). 1-hydroxybenzotriazole (237.95rrg, 1.76mmol) and EDCI (337.39mg, 1.76mmol) were added, and stirred at room temperature for 5min. (R)-2-amino-N-((R)-1-amino-1-oxo-3-(tritylthio)propan-2-yl)-3-(tritylthio) propanamide (1g, 1.41mmol) was added and reacted at room temperature for 30 rri nutes. After the reacti on was completed as detected by T L C, the mixture was washed with a saturated saline and extracted with dichloromethane. The organic phase was dried with sodium sulfate, then concentrated, and separated by column chromatography (methanol : dichloromethane: 1%-5%) to obtain the product as a white solid (1.4g, yield: 93.33%). ¹H NMR (400MHz, DMSO) 8.03(s, 3H), 7.87(d, 2H), 7.55(d, 2H), 7.38-7.26(m, 49H), 4.81-4.70(m, 5H), 4.46(m,1H), 3.28-2.81(m, 6H).

### Step 6: Synthesis of (R)-2-amino-N-((R)-1-(((R)-1-amino-1-oxo-3-(tritylthio)propan-2-yl)amino)-1-oxo-3-(tritylthio)propan-2-yl)-3-(tritylthio) propionamide:

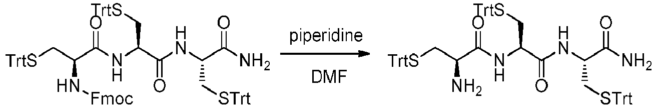

The compound (9H-fluoren-9-yl) methyl ((4R,7R,10R)-4-carbamoyl-6,9-dioxo 1,1,1,13,13,13-hexaphenyl-7-((tritylthio)methyl) -2,12-dithia-5,8-diazatridec-10-yl) carbamate (1.4g, 1.1mmol) was dissolved in N,N-dimethylformamide (10ml). Piperidine (0.02ml, 0.22mmol) was added and stirred at room temperature for4 hours. After the reacti on was completed as detected by T L C, the reacti on mi xture was washed wi th a saturated saline and extracted with dichloromethane. The organic phase was dried with sodium sulfate, then concentrated, and separated by column chromatography (methanol: dichloromethane: 1%-5%) to obtain a white solid (748nrg, yield: 64.48%). ¹H NMR (400MHz, DMSO) 8.03(s, 2H), 7.33-7.16(m, 45H), 4.81(m, 2H), 3.84(m, 1H), 3.26-2.78(m, 6H).

### Step 7: Synthesis of (R)-2-amino-N-((R)-1-(((R)-1-amino-3-mercapto-1-oxopropan-2-yl)amino)-3-mercapto-1-oxopropan-2-yl)-3-mercaptopropanamide trifluoroacetate:

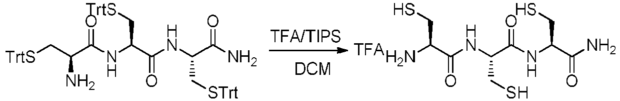

The compound (R)-2-amino-N-((R)-1-(((R)-1-amino-1-oxo-3-(tritylthio)propan-2-yl)amino)-1-oxo-3-(tritylthio)propan-2-yl)-3-(tritylthio) propionamide (1.2g, 1.1mmol) was dissolved in dichloromethane (10ml). Triisopropylsilane (0.56m1, 2.75mmol) and trifluoroacetic acid (2ml) were added at 0 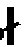 C under a nitrogen atmosphere and stirred in an ice bath for 2 hours. After the reaction was completed as detected by T LC, the mixture was concentrated, diethyl ether was added, and stirred in the ice bath. White solids were precipitated, filtered and dried to obtain the product (62nrg, yield: 17.27%). ¹H NMR (400MHz, DMSO-d6) 8.49-8.14(m, 2H), 7.50-7.16(m, 3H), 4.63-4.05(m, 3H), 2.85-2.63(m, 5H), 2.32-2.23(m, 1H).HESI:327.06[M +H]⁺.

### Example4: Synthesis of (R)-3-mercapto-N-((R)-3-mercapto-1-(((R)-3-mercapto-1-(methylamino)-1-oxopropan-2-yl)amino)-1-oxopropan-2-yl)-2-(methylamino) propanamidetrifluoroacetate:

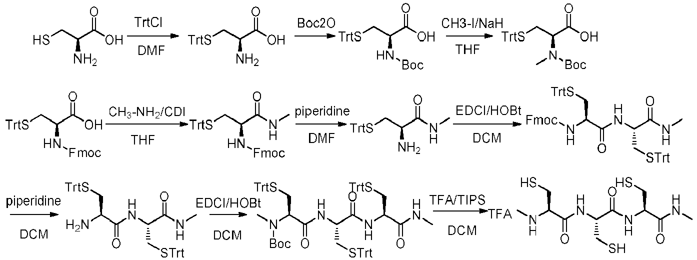

### Step 1: Synthesis of S-trityl-L-cysteine:

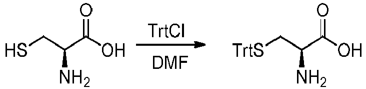

The compound L-cysteine hydrochloride (10g, 63.45rrmol) was dissolved in N,N-dimethylformamide (120ml). Triphenylchloromethane (19.46g, 69.795mmol) was added, heated to 60-65 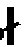 C, and reacted for 8h. After the reacti on was completed as detected by T LC, the reaction was cooled to room temperature, and 10% sodium acetate solution (300ml) was added. White solids were then precipitated and filtered. Filter residue was washed with pure water (300ml), then washed with acetone (200ml), and dried to obtain the product as a white solid (17.56g, yield: 76.15%).¹H NMR (400MHz, DMSO) 7.28(m, 18H), 2.92(dd, 1H), 2.59(dd, 1H), 2.41(dd, 1H). Step 2: Synthesis of N-(tert-butoxycarbonyl)-S-trityl-L-cysteine:

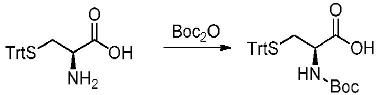

The compound S-trityl-L-cysteine (5g, 13.76mmol) was dissolved in a mixture of dioxane (40ml), water (20ml) and 1M sodium hydroxide solution (14ml), and stirred in an ice bath. Boc-anhydride (3.5ml, 15.14mmol) was added, then reacted until the mixture was naturally warmed to room temperature, and stirred for 8 hours. After the reaction was completed as detected by T LC, the reaction mixture was concentrated to 20-25ml. Ethyl acetate was added, and the sodi um bisulfate sol uti on was added dropwi se under the ice bath while sti rri ng. After pH was adjusted to 2-3, ethyl acetate was used for extraction. The organic layer was washed with a saturated saline, then dried with sodium sulfate, concentrated, and separated by column chromatography (methanol : di chloromethane: 1%-5%) to obtain the product as a white solid (5.5g, yield :86.21 %).' H NMR (400M Hz, DMSO-d6) 7.26(m, 16H), 3.78(m, 1H), 2.51(m, 1H), 2.36(dd, 1H), 1.4(s, 9H).

### Step3: Synthesis of N-(tert-butoxycarbonyl)-N-methyl-S-trityl-L-cysteine:

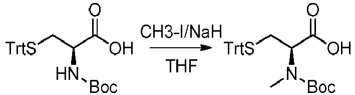

The compound N-(tert-butoxycarbonyl)-S-trityl-L-cysteine (2.1g, 4.53mmol) was dissolved in anhydrous tetrahydrofuran (6ml). Sodium hydride (436rrg, 10.9mmol) was dissolved in anhydrous tetrahydrofuran (14ml). The solution of amino acid in tetrahydrofuran was added dropwi se to the solution of sodi um hydri de in tetrahydrofuran in an ice bath. Then, methyl iodide (0.93ml, 14.95mmol) was slowly added dropwise and stirred overnight. After the reaction was completed as detected by TL C, phosphate buffer at pH of 7 was added for quenchi ng. pH was adjusted to 6-7 with a saturated ammonium chloride sol ution, and was extracted with ethyl acetate. The organic layer was washed with a saturated saline, then dried with sodium sulfate, concentrated, and separated by column chromatography (methanol : dichloromethane: 1%-5%) to obtain the product as a white solid (1.3g, yield: 60.19%).¹H NMR (400MHz, DMSO-d6) 7.3(m, 15H), 3.75(s, 1H), 2.8(s, 1H), 2.66(d, 4H), 1.4(d, 9H).

### Step4: Synthesis of (9H-fluoren-9-yl) methyl (R)-(1-(methylamino)-1-oxo-3-(tritylthio)propan-2-yl) carbamate:

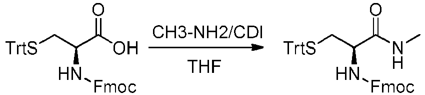

The compound (R)-2-(((9H-fluoren-9-yl)methoxy)carbonyl)amino)-3-(tritylthio) propionic acid (10g, 17.07mmol) was dissolved in tetrahydrofuran (50ml). N,N'-carbonyldiimidazole (5.59g, 34.48mmol) was added at 0-5 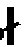 C. After stirring for 2 hours under nitrogen atmosphere, methylamine (3.03ml, 68.28mmol) was added, and reacted at 0-5 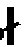 C for 2 hours. After the reactants were consumed, 2M hydrochloric acid (60ml) was added for quenching, and the reaction mixture was extracted with dichloromethane. The organic layer was washed with a saturated saline, then dried with sodium sulfate and then concentrated to obtain a crude product. Methanol (20ml) was added and stirred overnight at room temperature. White solids were precipitated, and fi ltered to obtain the product in filter residue. The methanol phase was concentrated, and separated by column chromatography (methanol: dichloromethane: 1%-5%) to obtain a white solid (9.44g, yield: 92.37%).¹H NMR (400MHz, DMSO-d6) 7.89(d, 2H), 7.81(d, 1H), 7.74(d, 2H), 7.66(d, 1H), 7.41(t, 2H), 7.29(m, 17H), 4.31(d, 1H), 4.22(t, 2H), 4.00(d, 1H), 2.53(d, 3H), 2.39(d, 2H).

### Step 5: Synthesis of (R)-2-amino-N-methyl-3-(tritylthio) propionamide:

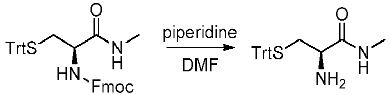

The compound (9H-fluoren-9-yl) methyl (R)-(1-(methylamino)-1-oxo-3-(tritylthio)propan-2-yl) carbamate (2g, 3.34mmol) was dissolved in N,N-dimethylformamide (20ml). Piperidine (0.07ml, 0.668mmol) was added and reacted at room temperature for 4 hours. After the reaction was completed as detected by T L C, the reaction mixture was washed with a saturated saline and extracted with dichloromethane. The organic phase was dried with sodium sulfate, concentrated, and separated by column chromatography (methanol : dichloromethane: 1%-5%) to obtain the product as a yellowish white solid (879rrg, yield: 69.76%). ¹H NMR (400MHz, DMSO-d6) 7.77(d, 1H), 7.29(m, 15H), 3.08(m, 1H), 2.55(d, 3H), 2.37(m, 1H), 2.19(m, 1H), 1.80(s, 2H).

### Step 6: Synthesis of (9H-fluoren-9-yl) methyl ((R)-1-(((R)-1-(methylamino) -1-oxo-3-(tritylthio)propan-2-yl)amino)-1-oxo-3-(tritylthio)propan-2-yl) carbamate:

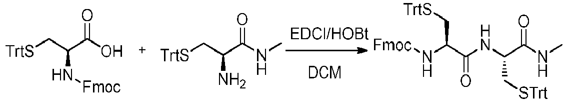

The compound (R)-2-((((9H-fluoren-9-yl)methoxy)carbonyl)amino)-3-(tritylthio) propanoic acid (100rrg, 0.17mmol) was dissolved in dichloromethane (5ml). 1-hydroxybenzotriazole (34.5mg, 0.255 mmol) and E DCI (48.9mg, 0.255mmol) were added, and stirred at room temperature for 5min. (R)-2-amino-N-methyl-3-(tritylthio) propanamide (76.8rrg, 0.204mmol) was added and reacted at room temperature for 30 minutes. After the reaction was completed as detected by T L C, the mixture was washed with a saturated saline and extracted with dichloromethane. The organic phase was dried with sodium sulfate, then concentrated, and separated by column chromatography (methanol : dichloromethane: 1%-5%) to obtain the product as a white solid (160rrg, yield: 99.68%).¹H NMR (400MHz, DMSO-d6) 7.89(d, 2H), 7.71(m, 4H), 7.40(m, 2H), 7.38-7.25(m, 30H), 4.25(m, 4H), 4.01(m, 1H), 2.50-2.33(m, 7H).

### Step 7: Synthesis of (R)-2-amino-N-((R)-1-(methylamino)-1-oxo-3-(tritylthio)propan-2-yl)-3-(tritylthio) propionamide:

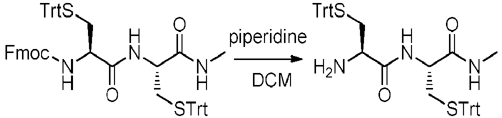

The compound (9H-fluoren-9-yl) methyl ((R)-1-(((R)-1-(methylamino)-1-oxo-3-(tritylthio)propan-2-yl) amino)-1-oxo-3-(tritylthio)propan-2-yl) carbamate(3.6g, 3.8mmol) was dissolved in N,N-dimethylformamide (15ml). Piperidine (0.07ml, 0.76mmol) was added and reacted at room temperature for 4 hours. After the reaction was completed as detected by T L C detection, the reaction mixture was washed with a saturated saline and extracted with dichloromethane. The organic phase was dried with sodium sulfate, then concentrated, and separated by column chromatography (methanol: dichloromethane: 1%-5%) to obtain the product as a white solid (1.3g, yield: 47.44%).¹H NMR (400MHz, DMSO-d6) 8.12(s, 1H), 7.83(d, 1H), 7.27(m, 30H), 4.25(s, 1H), 3.29(m, 2H), 3.20(s, 1H), 2.65-2.23(m, 5H).

### Step 8: Synthesis of tert-butyl methyl((4R,7R,10R)-3,6,9-trioxo-13,13,13-triphenyl-4,7-bis((tritylthio)methyl)-12-thia-2,5,8-triazatridec-10-yl) carbamate:

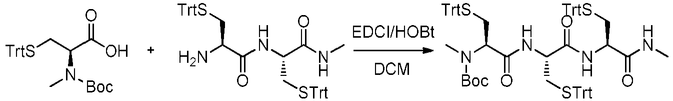

The compound N-(tert-butoxycarbonyl)-N-methyl-S-trityl-L-cysteine (509rrg, 1.07mmol) was dissolved in dichloromethane(10ml). 1-hydroxybenzotriazole(218rrg, 1.61mmol) and EDCI (309mg, 1.61mmol) were added, and stirred at room temperature for 5min. (R)-2-amino-N-((R)-1-(methylamino)-1-oxo-3-(tritylthio)propan-2-yl)-3-(tritylthio) propanamide (924rrg, 1.28mmol) was added and reacted at room temperature for 30 minutes. After the reaction was completed as detected by T L C, the mixture was washed with a saturated saline and extracted with dichloromethane. The organic phase was dried with sodium sulfate, concentrated, and separated by column chromatography (methanol : dichloromethane: 1%-5%) to obtain the product as a white solid (985mg, yield: 77.93%).¹H NMR (400MHz, DMSO-d6) 8.11(d, 1H), 7.73(d, 2H), 7.32-7.21(m, 4SH), 4.25(m, 3H), 2.62(m, 1H), 2.49(m, 6H) 2.47-2.23(m, 5H), 1.35-1.21(d, 9H).

### Step 9: Synthesis of (R)-3-mercapto-N-((R)-3-mercapto-1-(((R)-3-mercapto-1-(methylamino)-1-oxopropan-2-yl)amino)-1-oxopropan-2-yl)-2-(methylamino) propanamide trifluoroacetate:

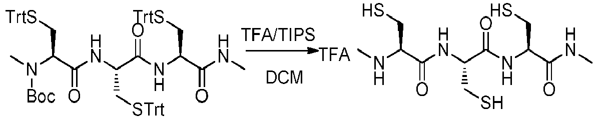

The compound tert-butyl methyl((4R,7R,10R)-3,6,9-trioxo-13,13,13-triphenyl-4,7-bis((tritylthio)methyl)-12-thia-2,5,8-triazatridec-10-yl) carbamate (1.5g, 1.27mmol) was dissolved in dichloromethane: trifluoroacetic acid : triisopropylsilane (50 : 47 : 3 by volume) (40ml), stirred at room temperature for 5min. After the reaction was completed as detected by T L C, the mixture was concentrated, diethyl ether was added, and stirred in an ice bath. White solids were precipitated, filtered and dried to obtain the product (446mg, yield: 77.77%). ¹HNMR(400MHz, MeOD) 4.54(m, 1H), 4.42(m, 1H), 4.05(t, J=5.2Hz, 1H), 3.13(m, 1H),2.99(m, 2H), 2.85(m, 2H), 2.79(rn 1H), 2.73(s, 3H), 2.70(s, 3H).MS: C₁₁H₂₂N₄O₃S₃, the calculated value is 354.51, and the measured value is 355.1, [M +H]⁺.

### Example 5: Synthesis of (S)-2-amino-N-((S)-1-amino-3-mercapto-1-oxopropan-2-yl)-3-mercaptopropanamide trifluoroacetate:

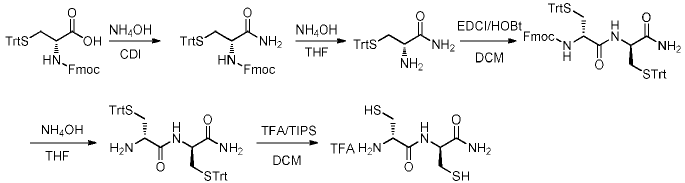

### Step 1: Synthesis of (9H-fluoren-9-yl) methyl (S)-(1-amino-1-oxo-3-(tritylthio)propan-2-yl) carbamate:

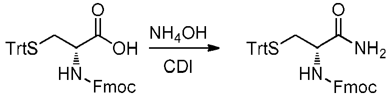

CDI (1.64g, 10.1mmol) was dissolved in a double-neck flask of DCM (50ml) and stirred at room temperature for 2h under N₂ protective conditions. The compound N-(((9H-fluoren-9-yl)methoxy)carbonyl)-S-trityl-D-cysteine (2.93g, 5mmol) was added to a reaction flask and stirred for 15min. After the completion of the reacti on, a saturated saline was added for washi ng (20ml B3). Anhydrous Na₂SO₄ was used to dry the organic phase, and the reaction liquid was concentrated by distillation under reduced pressure. After adding anhydrous methanol (50ml) and stirring for about 1h, the product was precipitated and filtered, and the filter cake was repeatedly washed twice to obtain the white crystal line powder (2.2g, yield: 75.30%). ¹H NM R (400M Hz, CDCl₃) 7.73(m, 2H), 7.54 (d,J =7.6 Hz, 2H), 7.37(m, 8H), 5.69(s, 1H), 5.25(s, 1H), 4.93(d, J =5.6 Hz 1H), 4.43 (m, 2H), 4.17(t, J=6.4 Hz, 1H), 3.80(s, 1H)2.64(m, 2H).

### Step 2: Synthesis of (S)-2-amino-3-(tritylthio) propionamide:

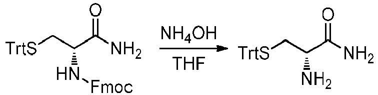

The compound (9H-fluoren-9-yl) methyl (S)-(1-amino-1-oxo-3-(tritylthio)propan-2-yl) carbamate (2.2g, 3.76mmol) was dissolved in a single-neck flask of THF (50ml), NH₄OH (25%, 11.6ml) was added, and stirred at room temperature overnight. After completion of the reaction, the mixture was washed with a saturated saline (30B3ml) and extracted with EA (30ml). The organic phases were combined and dried with anhydrous Na₂SO₄. After concentrating by distillation under reduced pressure, the crude product was separated and purified by silica gel column to obtain a colorless oily product (1.20g, yield: 88.24%). ¹H NMR(400MHz, CDCl₃) 7.44(m, 6H), 7.24(m, 9H), 6.80(s, 1H), 5.46(s, 1H), 2.98(m, 1H), 2.70(m, 1H), 2.55(m, 1H).

### Step3: Synthesis of (9H-fluoren-9-yl) methyl ((S)-1-(((S)-1-amino-1-oxo-3-(tritylthio)propan-2-yl)amino)-1-oxo-3-(tritylthio)propan-2-yl) carbamate:

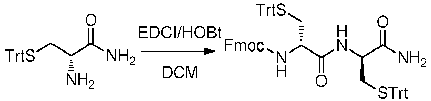

The compound N-(((9H-fluoren-9-yl)methoxy)carbonyl)-S-trityl-D-cysteine (10.33g, 17.64mmol) was dissolved in a single-neck flask of DCM (200ml), EDCI (5.10g, 26.46mmol) and HOBT (3.58g, 26.46mmol) were added, the compound (S)-2-amino-3-(tritylthio)propanamide (7.0g, 19.40mmol) was added, and stirred at room temperature for 20min. After completion of the reaction, the mixture was washed with a saturated saline (100B3ml) and extracted with DCM (20ml). The organic phase was dried with anhydrous Na₂SO₄. After concentrating by distillation under reduced pressure, the crude product was purified by silica gel column chromatography to obtain a colorless oily product (15.0g, yield: 91.46%). ¹H NMR(400MHz, CDCl₃) 7.88(m, 3H), 7.70(m, 3H), 7.40(m, 2H), 7.24(m, 30H), 4.25(m, 3H), 2.35(m, 3H).

### Step4: Synthesis of (S)-2-amino-N-((S)-1-amino-1-oxo-3-(tritylthio)propan-2-yl)-3-(tritylthio) propionamide:

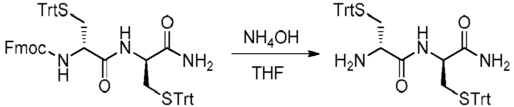

The compound (9H-fluoren-9-yl) methyl ((S)-1-(((S)-1-amino-1-oxo-3-(tritylthio)propan-2-yl)amino)-1-oxo-3-(tritylthio)propan-2-yl) carbamate (3.5g, 3.76mmol) was dissolved in a single-neck flask of THF (30ml), NH₄OH (25%, 11.60ml) was added, and stirred at room temperature overnight. After completion of the reaction, the mixture was washed with a saturated saline (30B3ml) and extracted with EA (30ml). The organic phases were combined and dried with anhydrous Na₂SO₄. After concentrating by distillation under reduced pressure, the crude product was separated and purified by silica gel column to obtain a colorless oily product (1.64g, yield: 61.65%).¹H NMR(400MHz, CDCl₃) 7.90(m, 12H), 7.23(m, 18H), 6.07(s, 1H), 5.15(s, 1H), 3.92(m, 1H), 2.66(m, 2H), 2.94(m, 2H).

### Step 5: Synthesis of (S)-2-amino-N-((S)-1-amino-3-mercapto-1-oxopropan-2-yl)-3-mercaptopropanamide trifluoroacetate:

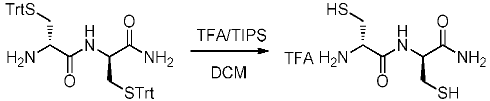

The compound (S)-2-amino-N-((S)-1-amino-1-oxo-3-(tritylthio)propan-2-yl)-3-(tritylthio) propionamide (2.8g, 3.96mmol) was dissolved in a single-neck flask of DCM (30ml), TFA (18g, 158.4mmol) and triethyl silicane (2.76g, 23.8mmol) were added, and stirred at room temperature for about 30min. After the completion of reaction, white solids appeared in the reaction liquid concentrated by distillation under reduced pressure, and anhydrous diethyl ether (50mlB3) was added for stirring and washing, and then filtered to obtain a white-like solid (710rrg, yield: 80%). ¹HNMR(400MHz, MeOD) 4.50(m, 1H), 4.10(t, J =5.6Hz, 1H), 3.28(m, 2H), 2.85(m, 2H).MS: C₆H₁₃N₃O₂S₂, the calculated value was 223.04, and the measured value was 244.1, [M +H]⁺.

### Example 6: Synthesis of (S)-2-amino-N-((S)-1-(((S)-1-amino-3-mercapto-1-oxopropan-2-yl)amino)-3-mercapto-1-oxopropan-2-yl)-3-mercaptopropionamide:

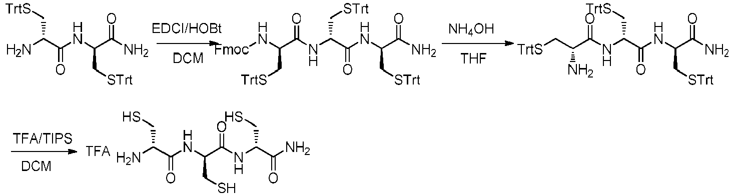

### Step 1: Synthesis of (9H-fluoren-9-yl) methyl ((4S,7S,10S)-4-carbamoyl-6,9-dioxo -1,1,1,13,13,13-hexaphenyl-7-((tritylthio)methyl) -2,12-dithia-5,8-diazatridec-10-yl) carbamate:

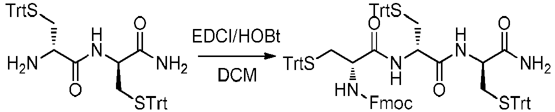

The compound N-(((9H-fluoren-9-yl)methoxy)carbonyl)-S-trityl-D-cysteine (1.24g, 2.12mmol) was dissolved in a single-neck flask of DCM (50ml), EDCI (609.6rrg, 3.18mmol) and HOBT (429.94rrg, 3.18mmol) were added, the compound (S)-2-amino-N-((S)-1-amino-1-oxo-3-(tritylthio)propan-2-yl)-3-(tritylthio) propanamide (1.65g, 2.33mmol) was added, and stirred at room temperature for 20min. DCM (20ml) was added for extraction and saturated saline (20mIB3) for washing. The organic phase was dried with anhydrous Na₂SO₄ and then concentrated. The crude product was purified by silica gel column chromatography to obtain a colorless oily product(2.57g, yield: 92.5%). ¹H NMR(400MHz, CDCl₃) 7.78(t,J=8.6Hz, 2H), 7.48(m, 2H), 7.37(m, 20H), 7.16(m, 29H), 6.52(d,J=8Hz, 1H), 6.36(s, 1H), 6.22(d,J =5.6Hz, 1H), 4.98(s, 1H), 4.82(d,J=5.2Hz,1H), 4.43(m, 1H), 4.22(m, 1H), 4.10(m, 2H), 3.91(m, 2H), 3.52(m, 1H), 2.61(m, 5H).

### Step 2: Synthesis of (S)-2-amino-N-((S)-1-(((S)-1-amino-1-oxo-3-(tritylthio)propan-2-yl)amino)-1-oxo-3-(tritylthio)propan-2-yl)-3-(tritylthio) propionamide:

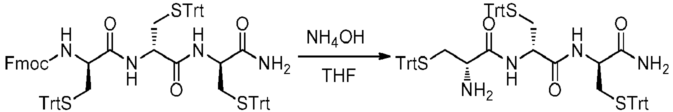

The compound (9H-fluoren-9-yl) methyl ((4S,7S,10S)-4-carbamoyl-6,9-dioxo -1,1,1,13,13,13-hexaphenyl-7-((tritylthio)methyl) -2,12-dithia-5,8-diazatridec-10-yl) carbamate (1.0g, 0.78mmol) was dissolved in a single-neck flask of THF (20ml), NH₄OH (25%, 2.40ml) was added, and stirred at room temperature overnight. After completion of the reaction, the mixture was washed with a saturated saline (10B3ml) and extracted with EA (20ml). The organic phases were combined and dried with anhydrous Na₂SO₄. After concentrating by distillation under reduced pressure, the crude product was separated and purified by silica gel column to obtain a colorless oily product (583.37mg, yield: 71.0%).¹H NMR(400MHz, CDCl₃) 7.22(m, 45H)6.52(d, J=8Hz, 1H), 6.36(s, 1H), 6.22(d, J=5.6Hz, 1H), 4.99(s, 1H), 4.82(d, J=5.2Hz, 1H), 4.22(m, 1H), 3.90(m, 2H), 3.52(m, 1H), 2.60(m, 5H).

### Step 3: Synthesis of (S)-2-amino-N-((S)-1-(((S)-1-amino-3-mercapto-1-oxopropan-2-yl)amino)-3-mercapto-1-oxopropan-2-yl)-3-mercaptopropionami de:

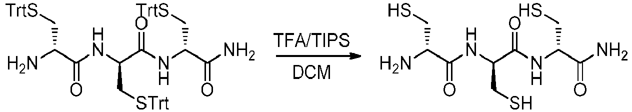

The compound (S)-2-amino-N-((S)-1-(((S)-1-amino-1-oxo-3-(tritylthio)propan-2-yl)amino)-1-oxo-3-(tritylthio)propan-2-yl)-3-(tritylthio) propionamide (1.20g, 1.14mmol) was dissolved in a single-neck flask of DCM (30ml), TFA (5.20g, 45.56mmol) and triethyl silicane (795.36mg, 6.84mmol) were added, and stirred at room temperature for about 30min. After the completion of reaction, white solids appeared in the reaction liquid concentrated by distillation under reduced pressure, and anhydrous diethyl ether (50ml) was added for stirring and washing, and then filtered and repeatedly washed with anhydrous diethyl ether 3 times to obtain the white-like solids(192.78mg,yield: 51.80%). ¹HNMR (400MHz, MeOD) 4.54(m, 1H), 4.42 (m, 1H),4.05(t, J =5.2Hz, 1H), 3.13 (m, 1H), 2.99 (m, 2H), 2.85 (m, 2H), 2.79 (m, 1H). MS: C₉H₁₈N₄O₃S₃, the calculated value was 326.05, and the measured value was 327.1, [M +H]⁺.

### Example 7: Synthesis of (S)-3-mercapto-N-((S)-3-mercapto-1-(methylamino)-1-oxopropan-2-yl)-2-(methylamino) propionamide:

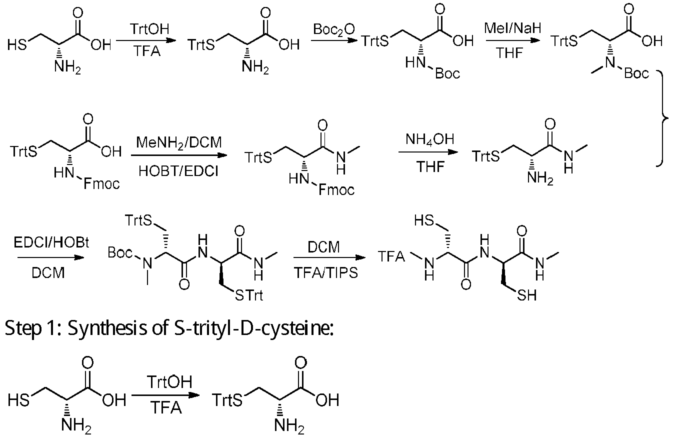

The compound D-cysteine (242.30rrg, 2.0mmo!) was added to a single-neck flask, TFA (4ml) was added, and stirred at room temperature. Tri phenyl methanol (520.68mg, 2.0mmol) was added, stirred at room temperature for 2h, then cooled to 0 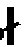 C. Anhydrous diethyl ether (30ml) was added, and a 4 N aqueous NaOH solution was added dropwise with stirring to a pH of about 4-5. A 10% saturated aqueous solution of sodium acetate was added to a pH of about 5-6 and filtered. The filter cake was washed with anhydrous diethyl ether (30mlB2) to obtain a white powdery product (566.4mg, yield: 77.90%). ¹H NMR(400MHz, DMSO-d6) 4.45(m, 1H), 7.21(m, 3H), 2.90(m, 1H), 2.52(m, 1H), 2.26 (t, J = 10.8 Hz, 1H), 1.82(s, 1H).

### Step 2: Synthesis of N-(tert-butoxycarbonyl)-S-trityl-D-cysteine:

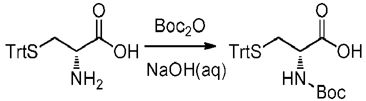

The compound S-trityl-D-cysteine (3.63g, 10mmol) was added to a single-neck flask with 2N NaOH (aq) (70ml), Boc₂O (3.43g, 15.7mmol) was added, and stirred at room temperature overnight. The reaction liquid was acidified with HCl (aq) to a pH value of about 2, extracted with DCM (20mlB3), and washed with a saturated saline (20mlB3). The organic phases were combined, dried with anhydrous Na₂SO₄, concentrated by distillation under reduced pressure and purified by flash column chromatography to obtain a colorless oily product (4.10g, yield: 88.40%). ¹H NMR(400MHz, CDCl₃) 7.39 (m, 6H), 7.25(m, 9H), 4.9(d,J= 7.6 Hz, 1H), 4.10(m, 1H),2.65(d, J= 4.4 Hz, 2H), 1.42(s, 9H).

### Step 3: Synthesis of N-(tert-butoxycarbonyl)-N-methyl-S-trityl-D-cysteine:

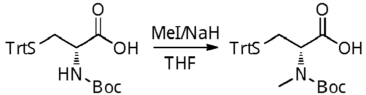

Under the condition of N₂ protection, NaH (60%, 1.035g, 25.88mmol) was dissolved in a three-neck flask of anhydrousTHF (20ml), and the solution (20ml) of the compound N-(tert-butoxycarbonyl)-S-trityl-D-cysteine (4.0g, 8.63mmol) in anhydrous THF was added dropwise at 0 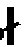 C. After stirring for 5min, iodomethane (9.8g, 69.03mmol) was added dropwise, and stirred at 0 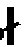 C for 1h and then stirred at room temperature overnight. After completion of the reaction, a phosphate buffer solution (50ml) with pH=7 was added for quenching reaction, and EA (20ml was added for extraction. The mixture was washed with a saturated saline (20ml B3). The organic phases were combined and dried with anhydrous Na₂SO₄. The crude product was separated and purified by silica gel column chromatography to obtain a colorless oily product (3.52g, yield: 85.44%). ¹HNMR (400MHz, CDCl₃) 7.41 (m, 6H), 7.25 (m, 9H), 3.85 (m, 0.5H), 2.63 (m, 0.5H), 2.79 (m, 1H), 2.65 (d, J = 14 Hz, 4H), 1.34 (d, J=10Hz, 9H).

### Step4: Synthesis of (9H-fluoren-9-yl) methyl (S)-(1-(methylamino)-1-oxo-3-(tritylthio)propan-2-yl) carbamate

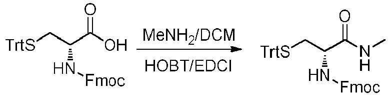

The compound N-(((9H-fluoren-9-yl)methoxy)carbonyl)-S-trityl-D-cysteine (11.75g, 20mmol) was dissolved in a single-neck flask of DCM (200ml), and EDCI (5.75g, 30mmol) and HOBT (4.05g, 30mmol) were added. The THF solution of methyl ami ne (2.0mol/L, 15ml) was added dropwi se, stirred at room temperature for 20min, extracted with DCM (50ml), washed with a saturated saline (100B3ml), and dried with anhydrous Na₂SO₄. After concentrating by distil lation under reduced pressure, anhydrous methanol (200ml) was added and stirred at room temperature for about 1h, and fi Itered to obtain white solids, which were washed repeatedly three times with anhydrous methanol to obtain a white solid (10.84g, yield: 90.64%). ¹H NMR(400MHz, CDCl₃) 7.73(t, J=6.6Hz, 2H), 7.54(d, J=6.8Hz, 2H),7.36(m, 8H), 7.23(m, 11H), 5.73(s, 1H), 4.94(d, J=6.8Hz, 1H), 4.39(d, J=6.4Hz, 2H), 4.16(t, J =6.6 Hz, 1H), 3.78(m, 1H), 2.69(d, J=4.4Hz, 3H), 2.62(m, 2H).

### Step 5: Synthesis of (S)-2-amino-N-methyl-3-(tritylthio) propionamide:

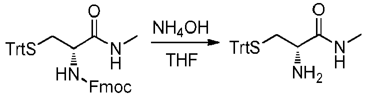

The compound (9H-fluoren-9-yl) methyl (S)-(1-(methylamino) -1-oxo-3-(tritylthio)propan-2-yl) carbamate (10.84g, 18.10mmol) was dissolved in a single-neck flask of THF (100ml), NH₄OH (25%, 28ml) was added, and stirred at room temperature overnight. After completion of the reacti on, the mixture was washed with a saturated saline (30B3ml) and extracted with EA (30ml). The organic phases were combined and dried with anhydrous Na₂SO₄. After concentrating by distillation under reduced pressure, PE(100ml) was added and stirred at room temperature for about 1h, and filtered to obtain white-like crystals, which were washed repeatedly 3 times with PE to obtain a white solid (6.8g, yield: 99.7%). ¹H NMR(400MHz, CDCl₃) 7.42(m, 6H), 7.23(m, 9H), 6.96(s, 1H), 3.00(m, 1H), 2.74(m, 1H), 2.70(d,J=4.8Hz, 3H), 2.50(m, 1H).

### Step 6: Synthesis of tert-butyl methyl ((S)-1-(((S)-1-(methylamino)-1-oxo-3-(tritylthio)propan-2-yl)amino)-1-oxo-3-(tritylthio)propan-2-yl) carbamate:

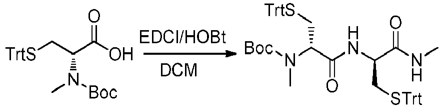

The compound N-(tert-butoxycarbonyl)-N-methyl-S-trityl-D-cysteine (3.52g, 7.37mmol) was dissolved in a single-neck flask of DCM (80ml), EDCI (2.12g, 11.1mmol) and HOBT (1.49g, 11.1mmol) were added, and the compound (S)-2-amino-N-methyl-3-(tritylthio) propanamide (3.05g, 8.1mmol) was added and stirred at room temperature for 20min. After completion of the reaction, the mixture was washed with a saturated saline (30mlB3) and extracted with DCM (30ml). The organic phase was dried with anhydrous Na₂SO₄. After concentrating by distillation under reduced pressure, the crude product was purified by silica gel column chromatography to obtain a colorless oily product (5.58g, yield: 90.58%). ¹HNMR (400M Hz, CDCl₃) 7.32 (m, 30H), 6.30 (s, 1H), 6.16 (s, 1H), 3.94 (m, 2H), 2.70 (m, 1H), 2.63 (s, 3H), 2.57 (s, 3H), 2.50 (m, 2H), 2.39 (m, 1H),1.38(s,9H).

### Step 7: Synthesis of (S)-3-mercapto-N-((S)-3-mercapto-1-(methylamino)-1-oxopropan-2-yl)-2-(methylamino) propionamide:

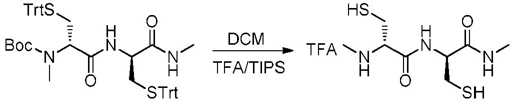

The compound tert-butyl methyl ((S)-1-(((S)-1-(methylamino)-1-oxo-3-(tritylthio)propan-2-yl)amino)-1-oxo-3-(tritylthio)propan-2-yl) carbamate (745.7mg, 0.89mmol) was dissolved in a sirgle-neck flask of DCM (10ml), TFA (5.10g, 44.6mmol) and triethyl silicane (622.33rrg, 5.352mmol) were added, and stirred at room temperature for about 30min. After the completion of reaction, white solids appeared in the reaction liquid concentrated by distillation under reduced pressure, and anhydrous diethyl ether (20ml) was added for stirring and washing, and then filtered and repeatedly washed 3 times to obtain a white powdery solid (215.70rrg, yield: 96.21%). ¹HNMR (400MHz, MeOD) 4.45 (m, 1H), 4.05 (t, J=5.4Hz, 1H), 3.29 (m, 2H), 3.10 (m, 2H), 2.73 (s, 3H), 2.70 (s, 3H). MS: C₈H₁₇N₃O₂S₂, the calculated value was 251.08, and the measured value was 252.1, [M +H]⁺.

### Example 8: Synthesis of (S)-3-mercapto-N-((S)-3-mercapto-1-(((S)-3-mercapto-1-(methylamino)-1-oxopropan-2-yl)amino)-1-oxopropan-2-yl)-2-(methylamino) propanamide:

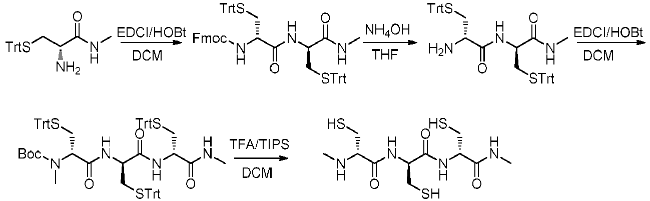

### Step 1: Synthesis of (9H-fluoren-9-yl) methyl ((S)-1-(((S)-1-(methylamino) -1-oxo-3-(tritylthio)propan-2-yl)amino)-1-oxo-3-(tritylthio)propan-2-yl) carbamate:

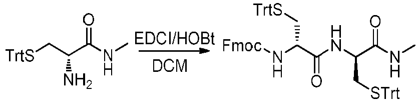

The compound N-(((9H-fluoren-9-yl)methoxy)carbonyl)-S-trityl-D-cysteine (5.63g, 9.6mmol) was dissolved in a single-neck flask of DCM (150ml), EDCI (2.76g, 14.4mmol) and HOBT (1.95g, 14.4mmol) were added, and the compound (S)-2-amino-N-methyl-3-(tritylthio)propanamide (3.80rrg, 10.1mmol) was added, and stirred at room temperature for 20min. After the completion of the reacti on, a saturated sal i ne was added for washing (30mlB3) and extracted with DCM (30ml). Anhydrous Na₂SO₄ was used to dry organic phase, and the reacti on liquid was concentrated by distillation under reduced pressure to form solid. PE (50ml B2) was added and stirred at room temperature for 20mim for fi ltration. The filter cake was stirred by adding anhydrous methanol (50ml B3) at room temperature for 20min and fi Itered to obtain white crystalline powder (8.42g, yield :92.8%) which was directly used for the next step.

### Step 2: Synthesis of (S)-2-amino-N-((S)-1-(methylamino) -1-oxo-3-(tritylthio)propan-2-yl)-3-(tritylthio) propionamide:

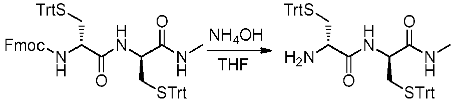

The compound (9H-fluoren-9-yl) methyl ((S)-1-(((S)-1-(methylamino) -1-oxo-3-(tritylthio)propan-2-yl)amino)-1-oxo-3-(tritylthio)propan-2-yl) carbamate (8.42g, 8.92mmol) was dissolved in a single-neck flask of THF (100ml), NH₄OH (25%, 27.5ml) was added, and stirred at room temperature overnight.After completion of the reaction, the mixture was washed with a saturated saline (30B3ml) and extracted with EA (30ml). The organic phases were combined and dried with anhydrous Na₂SO₄. After concentrating by distillation under reduced pressure, PE (50ml B2) was added and stirred at room temperature for about 1h. After filtration, white solids (5.95g, yield: 92.4%) were obtained and di rectly used for the next step.

### Step 3: Synthesis of tert-butyl methyl((4S,7S,10S)-3,6,9-trioxo-13,13,13-triphenyl-4,7-bis((tritylthio)methyl)-12-thia-2,5,8-triazatridec-10-yl) carbamate:

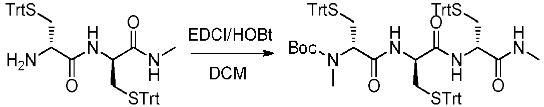

The compound N-(tert-butoxycarbonyl)-N-methyl-S-trityl-D-cysteine (1.97g, 4.12mmol) was dissolved in a single-neck flask of DCM (100ml), EDCI (1.19g, 6.19mmol) and HOBT (836.9mg, 6.19mmol) were added, and the compound (S)-2-amino-N-((S)-1-(methyl amino)-1-oxo-3-(tritylthio)propan-2-yl)-3-(tritylthio)propionamide (3.28g, 4.54mmol) was added and stirred at room temperature for 20min. After completion of the reaction, the mixture was washed with a saturated saline (30mlB3) and extracted with DCM (20ml). The organic phase was dried with anhydrous Na₂SO₄. After concentrating by distillation under reduced pressure, the crude product was purified by silica gel column chromatography to obtain a white-like oily product (2.12g, yield: 43.2%). ¹H NMR(400MHz, CDCl₃) 7.28(m, 45H), 6.64(s, 1H), 5.75(s, 1H), 4.18 (s,2H), 3.80(m, 1H), 3.20(m, 1H), 2.79(m, 1H), 2.65(m, 1H), 2.63(m, 4H), 2.52(m, 1H), 2.36(m, 4H), 2.24(m, 1H), 1.37(s, 9H).

### Step 4: Synthesis of (S)-3-mercapto-N-((S)-3-mercapto-1-(((S)-3-mercapto-1-(methylamino)-1-oxopropan-2-yl)amino)-1-oxopropan-2-yl)-2-(methylamino) propanamide:

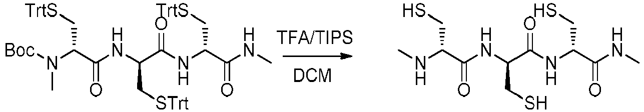

The compound methyl((4S,7S,10S)-3,6,9-trioxo-13,13,13-triphenyl-4,7-bis((tritylthio)methyl)-12-thia-2,5,8-triazatridec-10-yl) carbamate (1.0g, 0.85mmol) was dissolved in a single-neck flask of DCM (40ml), TFA (3.86g, 33.85mmol) and triethylsilane (S93rrg, 5.1mmol) were added, and stirred at room temperature for about 30min. After the completion of reaction, white solids appeared in the reaction liquid concentrated by distillation under reduced pressure, and anhydrous diethyl ether (50ml B3) was added for sti rri ng and washing, and then filtered toobtain a white solid powder (290nrg, yield: 96.67%).¹HNMR(400MHz, MeOD) 4.54(m, 1H), 4.42(m, 1H), 4.05(t,J =5.2Hz, 1H), 3.13(m, 1H),2.99(m, 2H), 2.85(m, 2H), 2.79(m, 1H), 2.73(s, 3H), 2.70(s, 3H).MS: C₁₁ H₂₂N₄O₃S₃, the calculated value was 354.09, and the measured value was 355.1, [M +H]⁺.

### Example 9: Synthesis of (R)-2-amino-3-(((R)-1-amino-3-mercaptopropan-2-yl)amino)propane-1-thiol hydrochloride:

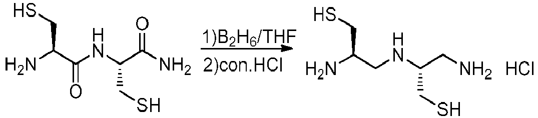

The compound (R)-2-amino-N-((R)-1-amino-3-mercapto-1-oxopropan-2-yl)-3-mercaptopropanamide (0.5g, 2.24mmol) (prepared in Example 1) was added to a reaction flask, anhydrousTH F (20ml) was added, and cooled with an ice-salt bath. A borane tetrahydrofuran solution (1M, 13.44ml, 13.44mmol) was added dropwise under a nitrogen atmosphere. After the dropwise addition was completed, the mixture was stirred at room temperature for 1 hour and heated to reflux for 18 hours. After the reacti on was completed as detected by T L C, the mixture was cooled to 0 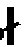 C, and methanol (1 ml) was added for quenchi ng the reacti on. After stirring for 30 mi nutes, concentrated hydrochloric acid (12N, 0.95ml, 11.42mmol) was added and heated to 80 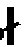 C for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (360mg, yield: 52.6%). ¹H NMR (400MHz, D₂O) 3.18(m, 2H), 2.91-2.52(m, 8H).MS (ES+) m/z 196.35 [M+H]⁺.

### Example 10: Synthesis of (R)-2-(((R)-3-mercapto-2-(methylamino)propyl)amino)-3-(methylamino)propane-1-thiol hydrochloride:

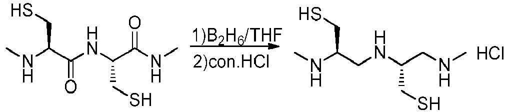

The compound (R)-3-mercapto-N-((R)-3-mercapto-1-(methylamino)-1-oxopropan-2-yl)-2-(methylamino) propanamide (2.0g, 7.96mmol) (prepared in Example 2) was added to a reaction flask, anhydrous T H F (50 ml) was added, and cooled with an ice-salt bath. A borane tetrahydrofuran solution (1M, 47.76ml, 47.76mmol) was added dropwise under a nitrogen atmosphere. After the dropwise addition was completed, the mixture was stirred at room temperature for 1 hour and heated to reflux for 18 hours. After the reaction was completed as detected by TLC, the mixture was cooled to 0 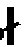 C, and methanol (1ml) was added for quenchi ng the reacti on. After sti rri ng for 30 minutes, concentrated hydrochloric acid (12N, 3.38ml, 40.60mmol) was added and heated to 80 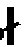 C for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (2.2g, yield: 84.39%).¹H NMR (400M Hz, D₂O) 3.28(s, 6H), 3.17(m, 2H), 2.77-2.75(m, 4H), 2.52-2.50(m, 4H).MS (ES+) m/z 224.40 [M+H]⁺.

### Example 11: Synthesis of (R)-2-amino-3-(((R)-1-(((R)-1-amino-3-mercaptopropan-2-yl)amino)-3-mercaptopropan-2-yl)amino)propane-1-thiol hydrochloride:

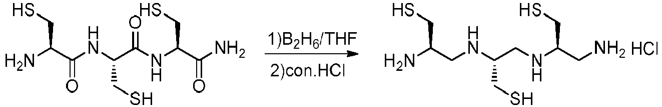

The compound (R)-2-amino-N-((R)-1-((R)-1-amino-3-mercapto-1-oxopropan-2-ylamino)-3-mercapto-1-oxopropan-2-yl)-3-mercaptopropionamide (2.5g, 7.66mmol) (prepared in Example 3) was added to a reaction flask, anhydrous THF (50 ml) was added, and cooled with an ice-salt bath. A borane tetrahydrofuran solution (1M, 68.94ml, 68.94 mmol) was added dropwise under a nitrogen atmosphere. After the dropwise addition was completed, the rri xture was stirred at room temperature for 1 hour and heated to reflux for 18 hours. After the reaction was completed as detected by TLC, the mixture was cooled to 0 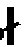 C, and methanol (3ml) was added for quenching the reaction. After stirring for 30 minutes, concentrated hydrochloric acid (12N, 4.7 ml, 57.45mmol) was added and heated to 80 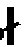 C for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (2.42 g, yield: 73.44%).¹H NMR (400MHz, D₂O) 3.17(m, 3H), 2.92-2.52(m, 12H).MS (ES+) m/z 285.51 [M+H]⁺.

### Example 12: Synthesis of (R)-2-((R)-3-mercapto-2-((R)-3-mercapto-2-(methylamino)propylamine)propylamino)-3-(methylamino)propane-1-thiol hydrochloride:

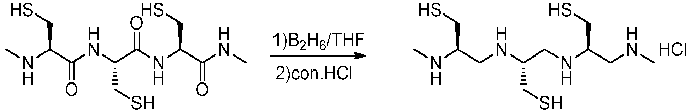

The compound (R)-3-mercapto-N-((R)-3-mercapto-1-((R)-3-mercapto-1-(methylamino)- 1-oxopropan-2-ylamino)-1-oxopropan-2-yl)-2-(methylamino) propanamide (1.5g, 4.59mmol) (prepared in Example4) was added to a reaction flask, anhydrous THF (20 ml) was added, and cooled with an ice-salt bath. A borane tetrahydrofuran solution (1M, 41.31 ml, 41.31 mmol) was added dropwise under a nitrogen atmosphere. After the dropwise addition was completed, the mixture was stirred at room temperature for 1 hour and heated to reflux for 18 hours. After the reaction was completed as detected by T L C, the mixture was cooled to 0éC, and methanol (1ml) was added for quenching the reaction. After stirring for 30 minutes, concentrated hydrochloric acid (12N, 1.9 ml, 22.8 mmol) was added and heated to 80éC for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (2.1g, yield: 69.65%).¹H NMR (400MHz, D₂O) 3.26(s, 6H), 3.17(m, 3H), 2.77-2.75(m, 6H), 2.52-2.50(m, 6H).MS (ES+) m/z 327.55 [M+H]⁺.

### Example 13: Synthesis of (S)-2-amino-3-(((S)-1-amino-3-mercaptopropan-2-yl)amino)propane-1-thiol hydrochloride:

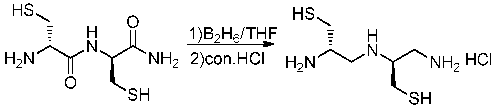

The compound (S)-2-amino-N-((S)-1-amino-3-mercapto-1-oxopropan-2-yl)-3-mercaptopropanamide (1.0g, 4.48mmol) (prepared in Example 5) was added to a reaction flask, anhydrous THF (20ml) was added, and cooled with an ice-salt bath. A borane tetrahydrofuran solution (1M, 26.88ml, 26.88mmol) was added dropwise under a nitrogen atmosphere. After the dropwise addition was completed, the mixture was stirred at room temperature for 1 hour and heated to reflux for 18 hours. After the reacti on was completed as detected by T L C, the mixture was cooled to 0éC, and methanol (1ml) was added for quenching the reaction. After stirring for 30 minutes, concentrated hydrochloric acid (12N, 1.90ml, 22.84mmol) was added and heated to 80éC for 1 hour. After the reacti on was completed, the mixture was cooled to room temperature and concentrated. D i ethyl ether (10ml) was added to the solids obtained and stirred for 1h, filtered, washed with diethyl ether, and dried to obtain the hydrochlorinated target compound as a white solid (780mg, yield: 57.16%).¹H NMR (400MHz, D₂O) 3.18(m, 2H), 2.91-2.52(m, 8H).MS (ES+) m/z 196.35 [M+H]⁺.

### Example 14: Synthesis of (S)-2-(((S)-3-mercapto-2-(methylamino)propyl)amino)-3-(methyl ami no)propane-1-thiol hydrochloride

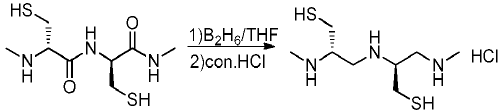

The compound (S)-3-mercapto-N-((S)-3-mercapto-1-(methylamino)-1-oxopropan-2-yl)-2-(methylamino) propanarride (1.0g, 3.98mmol) (prepared in Example 7) was added to a reaction flask, anhydrousTHF (25ml) was added, and cooled with an ice-salt bath. A borane tetrahydrofuran solution (1M, 23.88ml, 23.88mmol) was added dropwise under a nitrogen atmosphere. After the dropwise addition was completed, the mixture was stirred at room temperature for 1 hour and heated to reflux for 18 hours. After the reacti on was completed as detected by TL C, the mixture was cooled to 0éC, and methanol (1 ml) was added for quenchi ng the reaction. After stirring for 30 minutes, concentrated hydrochloric acid (12N, 1.69ml, 20.30mmol) was added and heated to 80éC for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (683mg, yield: 56.26%).¹H NMR (400M Hz, D₂O) 3.28(s, 6H), 3.17(m, 2H), 2.77-2.75(m, 4H), 2.52-2.50(m, 4H).MS (ES+) m/z 224.40 [M+H]⁺.

### Example 15: Synthesis of (S)-2-amino-3-(((S)-1-(((R)-1-amino-3-mercaptopropan-2-yl)amino)-3-mercaptopropan-2-yl)amino)propane-1-thiol hydrochloride

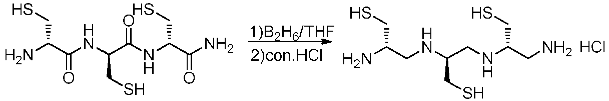

The compound (S)-2-arrino-N-((S)-1-((R)-1-amino-3-mercapto-1-oxopropan-2-ylamino)-3-mercapto-1-oxopropan-2-yl)-3-mercaptopropionamide (1.0g, 3.06mmol) (prepared in Example 6) was added to a reaction flask, anhydrous TH F (10ml) was added, and cooled with an ice-salt bath. A borane tetrahydrofuran solution (1M, 18.36ml, 18.36mmol) was added dropwise under a nitrogen atmosphere. After the dropwise addition was completed, the rri xture was stirred at room temperature for 1 hour and heated to reflux for 18 hours. After the reaction was completed as detected by TLC, the mixture was cooled to 0éC, and methanol (3ml) was added for quenching the reaction. After stirring for 30 minutes, concentrated hydrochloric acid (12N, 1.3ml, 15.61mmol) was added and heated to 80éC for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (760mg, yield: 63.11%).¹H NMR (400MHz, D₂O) 3.17(m, 3H), 2.92-2.52(m, 12H).MS (ES+) m/z 285.51 [M+H]⁺.

### Example 16: Synthesis of (S)-2-((S)-3-mercapto-2-((S)-3-mercapto-2-(methylamino)propylamine)propylamino)-3-(methylamino)propane-1-thiol hydrochloride:

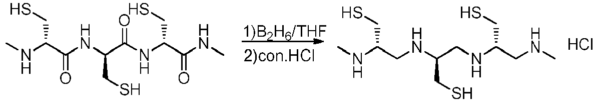

The compound (S)-3-mercapto-N-((S)-3-mercapto-1-((S)-3-mercapto-1-(methylamino)-1-oxopropan-2-ylamino)-1-oxopropan-2-yl)-2-(methylamino) propanamide (1.0g, 2.82mmol) (prepared in Example 8) was added to a reaction flask, anhydrous THF (10m) was added, and cooled with an ice-salt bath. A borane tetrahydrofuran solution (1M, 16.92ml, 16.92mmol) was added dropwise under a nitrogen atmosphere. After the dropwise addition was completed, the mixture was stirred at room temperature for 1 hour and heated to reflux for 18 hours. After the reaction was completed as detected by T L C, the mixture was cooled to 0éC, and methanol (1ml) was added for quenching the reaction. After stirring for 30 minutes, concentrated hydrochloric acid (12N, 1.20ml, 14.38mmol) was added and heated to 80éC for 1 hour. After the reaction was completed, the mixture was cooled to room temperature and concentrated. Diethyl ether (5ml) was added to the solids obtained and stirred for 1h, filtered, washed with diethyl ether, and dried to obtain the hydrochlorinated target compound as a white solid (756nrg, yield: 68.05%).¹H NMR (400MHz, D₂O) 3.26(s, 6H), 3.17(m, 3H), 2.77-2.75(m, 6H), 2.52-2.50(m, 6H).MS (ES+) m/z 327.55 [M+H]⁺.

### Example 17: Synthesis of (S)-2-amino-N-((R)-1-amino-3-mercapto-1-oxopropan-2-yl)-3-mercaptopropanamide trifluoroacetate

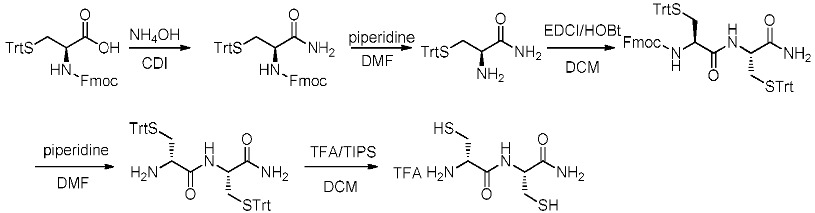

### Step 1: Synthesis of (9H-fluoren-9-yl) methyl (R)-(1-amino-1-oxo-3-(tritylthio)propan-2-yl) carbamate:

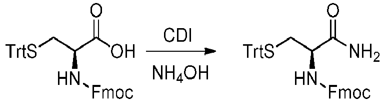

The compound (R)-2-(((9H-fluoren-9-yl)methoxy)carbonyl)amino)-3-(tritylthio) propionic acid (10 g, 17.07 mmol) was dissolved in tetrahydrofuran (50ml). N,N'-carbonyldiimidazole (5.59g, 34.48mmol) was added at 0-5éC. After stirring for 2 hours under nitrogen protection, aqueous ammonia (5ml, 68.28mmol) was added, and reacted at 0-56C for 30 minutes. After the reacti on was completed as detected by T L C, 2 M hydrochloric acid (60ml) was added for quenching. The reaction mixture was extracted with ethyl acetate, the organic phase was washed with a saturated saline, then dried with sodium sulfate, and concentrated to obtain a crude product. After adding anhydrous methanol (20ml) and stirring at room temperature overnight, white solids were precipitated, and filtered to obtain the product in the filter cake. The methanol phasewas concentrated and separated by col umn chromatography (methanol: di chloromethane: 1%-5%) to obtain the target product as a white solid (9.3g, yield: 93.19%). ¹H NMR (400MHz, DMSO-d6) 7.89(d, 2H), 7.74(d, 2H), 7.58(d, 1H), 7.3(m, 18H), 7.11(s, 1H), 4.24(m, 3H), 4.01(m, 1H), 2.39(m, 2H).

### Step 2: Synthesis of (R)-2-amino-3-(tritylthio) propionamide:

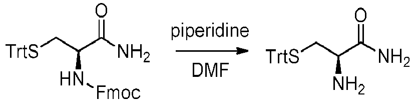

The compound (9H-fluoren-9-yl) methyl (R)-(1-amino-1-oxo-3-(tritylthio)propan-2-yl) carbamate (4g, 6.84mmol) was dissolved in N,N-dimethylformamide (20ml). Piperidine (0.14ml 1.368mmol) was added and reacted at room temperature for 4 hours. After the reaction was completed as detected by TL C, the reacti on mixture was washed with a saturated saline and extracted with dichloromethane. The organic phase was dried with sodium sulfate, concentrated, and separated by column chromatography (methanol: dichloromethane = 1%-5%) to obtain the target product as a yellow oil (2.3g, yield: 92%). ¹H NMR (400M Hz, DMSO-d6) 7.29(m, 17H), 3.08(d, 1H), 2.33(d, 1H), 2.18(s, 1H), 1.85(s, 2H).

### Step3: Synthesis of (9H-fluoren-9-yl) methyl ((R)-1-(((R)-1-amino-1-oxo-3-(tritylthio)propan-2-yl)amino)-1-oxo-3-(tritylthio)propan-2-yl) carbamate:

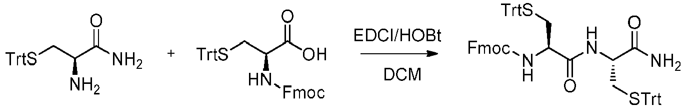

The compound (R)-2-((((9H-fluoren-9-yl)methoxy)carbonyl)amino)-3-(tritylthio) propanoic acid (1.29g, 2.21mmol) was dissolved in dichloromethane (15ml). 1-hydroxybenzotriazole (448rrg, 3.315mmol) and E DCI (635mg, 3.315mmol) were added, and stirred at room temperature for 5min. (9H-fluoren-9-yl) methyl (R)-(1-amino-1-oxo-3-(tritylthio)propan-2-yl) carbamate (960mg, 2.65mmol) was added and reacted at room temperature for 30 minutes. After the reaction was completed as detected by T L C, the mixture was washed with a saturated saline and extracted with dichloromethane. The organic phase was dried with sodium sulfate, concentrated, and separated by column chromatography (methanol : dichloromethane: 1%-5%) to obtain the product as a white solid (2.05g, yield: 99.76%). ¹H NMR (400MHz, DMSO-d6) 7.89(m, 3H), 7.7(m, 3H), 7.4(m, 2H), 7.24(m, 34H), 4.21(m, 4H), 4.10(m, 1H), 2.33(m, 4H).

### Step4: Synthesis of (S)-2-amino-N-((R)-1-amino-1-oxo-3-(tritylthio) propan-2-yl)-3-(tritylthio) propionamide:

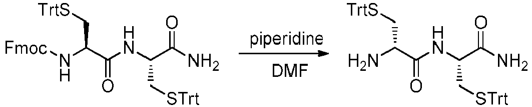

The compound (9H-fluoren-9-yl) methyl ((R)-1-(((R)-1-amino-1-oxo-3-(tritylthio)propan-2-yl) amino)-1-oxo-3-(tritylthio)propan-2-yl) carbamate (2.05g, 2.2mmol) was dissolved in N,N-dimethylformamide (20ml). Piperidine (0.04ml, 0.44mmol) was added and stirred at room temperature for 4 hours. After the reaction was completed as detected by TLC detecti on, the reacti on rri xture was washed with a saturated saline and extracted with dichloromethane. The organic phase was dried with sodium sulfate, concentrated, and separated by column chromatography (methanol : dichloromethane: 1%-5%) to obtain the product as a white solid (460rrg, yield: 29.54%).¹H NMR (400MHz, DMSO-d6) 8.1(s,1H), 7.37-7.14(m, 32H), 4.23(m, 1H), 3.17(m,1H), 2.39(dd, 1H), 2.33(d, 2H), 2.19(m, 1H).

### Step 5: Synthesis of (S)-2-amino-N-((R)-1-amino-3-mercapto-1-oxopropan-2-yl)-3-mercaptopropanamide trifluoroacetate:

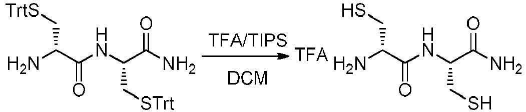

The compound (S)-2-amino-N-((R)-1-amino-1-oxo-3-(tritylthio)propan-2-yl)-3-(tritylthio) propionamide (200rrg, 0.28mmol) was dissolved in dichloromethane (5ml). Triisopropylsilane(0.14ml, 0.7mmol) and trifluoroacetic acid (1 ml) were added at 0éC under a nitrogen atmosphere and stirred in an ice bath for 2 hours. After the reaction was completed as detected by T L C, the mixture was concentrated, diethyl ether was added, and stirred in the ice bath. White solids were precipitated, filtered and dried to obtain the product (79mg, yield: 88.08%).¹H NMR (400MHz, DMSO-d6) 8.72(d, 1H), 8.23(s, 3H), 7.56(s, 1H), 7.32(s, 1H), 4.43(m, 1H), 4.09(m, 1H), 2.99(d, 2H), 2.89(m, 1H), 2.74(m, 1H); HESI:224.05 [M +H]+.

### Example 18: Synthesis of (R)-2-amino-N-((S)-1-amino-3-mercapto-1-oxopropan-2-yl)-3-mercaptopropanamide trifluoroacetate

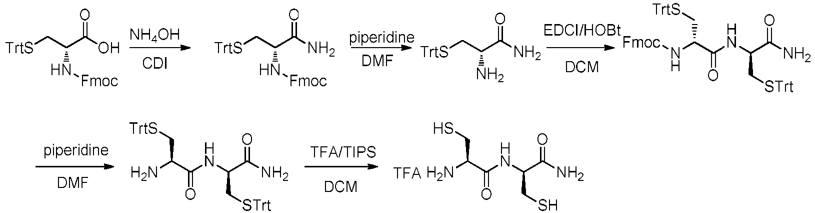

### Step 1: Synthesis of (9H-fluoren-9-yl) methyl (S)-(1-amino-1-oxo-3-(tritylthio)propan-2-yl) carbamate:

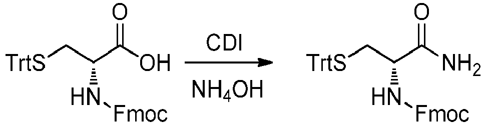

The compound (S)-2-(((9H-fluoren-9-yl)methoxy)carbonyl)amino)-3-(tritylthio) propionic acid (10 g, 17.07 mmol) was dissolved in tetrahydrofuran (50ml). N,N'-carbonyldiimidazole (5.59g, 34.48mmol) was added at 0-5éC. After stirring for 2 hours under nitrogen protection, aqueous ammonia (5ml, 68.28mmol) was added, and reacted at 0-56C for 30 rri nutes. After the reacti on was completed as detected by T L C, 2 M hydrochloric acid (60ml) was added for quenching. The reaction mixture was extracted with ethyl acetate, the organic phase was washed with a saturated saline, then dried with sodium sulfate, and concentrated to obtain a crude product After adding anhydrous methanol (20ml) and stirring at room temperature overnight, white solids were precipitated, and filtered to obtain the product in the filter cake. The methanol phasewas concentrated and separated by column chromatography (methanol: dichloromethane: 1%-5%) to obtain the target product as a white solid (8.93g, yield: 89.48%).¹H NMR (400MHz, DMSO-d6) 7.89(d, 2H), 7.74(d, 2H), 7.58(d, 1H), 7.3(m, 18H), 7.11(s, 1H), 4.24(m, 3H), 4.01(m, 1H), 2.39(m, 2H).

### Step 2: Synthesis of (S)-2-amino-3-(tritylthio) propionamide:

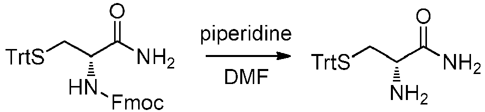

The compound (9H-fluoren-9-yl) methyl (S)-(1-amino-1-oxo-3-(tritylthio)propan-2-yl) carbamate (5.98g, 10.23mmol) was dissolved in N,N-dimethylformamide (20ml). Piperidine (0.2ml, 2.046mmol) was added and reacted at room temperature for 4 hours. After the reaction was completed as detected by T L C, the reacti on mixture was washed with a saturated saline and extracted with dichloromethane. The organic phase was dried with sodium sulfate, concentrated, and separated by column chromatography (methanol : dichloromethane = 1%-5%) to obtain the target product as a yellow oil (2.68g, yield: 72.24%).¹H NMR (400M Hz, DMSO-d6) 7.29(m, 17H), 3.08(d, 1H), 2.33(d, 1H), 2.18(s, 1H), 1.85(s, 2H).

### Step3: Synthesis of (9H-fluoren-9-yl) methyl ((S)-1-(((S)-1-amino-1-oxo-3-(tritylthio)propan-2-yl)amino)-1-oxo-3-(tritylthio)propan-2-yl) carbamate:

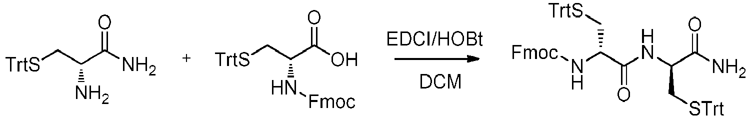

The compound (S)-2-((((9H-fluoren-9-yl)methoxy)carbonyl)amino)-3-(tritylthio) propanoic acid (3.61g, 6.16mmol) was dissolved in dichloromethane (25ml). 1-hydroxybenzotriazole (1.25nrg, 9.24 mmol) and EDCI (1.77rrg, 9.24mmol) were added, and stirred at room temperature for 5min. (9H-fluoren-9-yl) methyl (S)-(1-amino-1-oxo-3-(tritylthio)propan-2-yl) carbamate (2.68rrg, 7.39mmol) was added and reacted at room temperature for 30 mi nutes. After the reacti on was completed as detected by T L C, the mi xture was washed with a saturated saline and extracted with dichloromethane. The organic phase was dried with sodium sulfate, concentrated, and separated by column chromatography (methanol : dichloromethane: 1%-5%) to obtain the product as a white solid (5.56g, yield: 97.03%).¹H NMR (400MHz, DMSO-d6) 7.89(m, 3H), 7.7(m, 3H), 7.4(m, 2H), 7.24(m, 34H), 4.21(m, 4H), 4.10(m, 1H), 2.33(m, 4H).

### Step4: Synthesis of (R)-2-amino-N-((S)-1-amino-1-oxo-3-(tritylthio) propan-2-yl)-3-(tritylthio) propionamide:

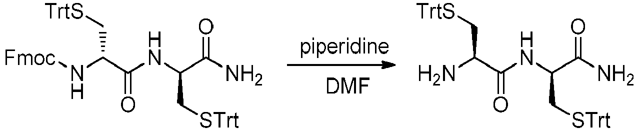

The compound (9H-fluoren-9-yl) methyl ((S)-1-(((S)-1-amino-1-oxo-3-(tritylthio)propan-2-yl) amino)-1-oxo-3-(tritylthio)propan-2-yl) carbamate (5.56g, 5.98mmol) was dissolved in N,N-dimethylformamide (25ml). Piperidine (0.12m, 1.196mmol) was added and stirred at room temperature for 4 hours. After the reaction was completed as detected by T L C detecti on, the reacti on mixture was washed with a saturated sal i ne and extracted with dichloromethane. The organic phase was dried with sodium sulfate, concentrated, and separated by column chromatography (methanol : dichloromethane: 1%-5%) to obtain the product as a white solid (1.27g, yield: 30%).¹H NMR (400MHz, DMSO-d6) 8.1(s,1H), 7.37-7.14(m, 32H), 4.23(m, 1H), 3.17(m,1H), 2.39(dd, 1H), 2.33(d, 2H), 2.19(m, 1H).

### Step 5: Synthesis of (R)-2-amino-N-((S)-1-amino-3-mercapto-1-oxopropan-2-yl)-3-mercaptopropanamide trifluoroacetate:

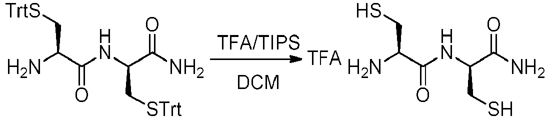

The compound (R)-2-arrino-N-((S)-1-arrino-1-oxo-3-(tritylthio)propan-2-yl)-3-(tritylthio) propionamide (40rrg, 0.057mmol) was dissolved in dichloromethane (5ml). Triisopropylsilane(0.03ml, 0.1425mmol) and trifluoroacetic acid (1 ml) were added at 0éC under a nitrogen atmosphere and stirred in an ice bath for 2 hours. After the reacti on was completed as detected by T LC, the mixture was concentrated, di ethyl ether was added, and stirred in the ice bath. White solids were precipitated, filtered and dried to obtain the product (16rrg, yield: 83.2%).¹H NMR (400MHz, DMSO-d6) 8.72(d, 1H), 8.23(s, 3H), 7.56(s, 1H), 7.32(s, 1H), 4.43(m, 1H), 4.09(m, 1H), 2.99(d, 2H), 2.89(m, 1H), 2.74(m, 1H); HESI:224.05 [M +H]+.

### Example 19: Synthesis of 3-mercapto-N-(3-mercapto-1-(methylamino)-1-oxopropan-2-yl)-2-(methylamino) propionami de tri fl uoroacetate

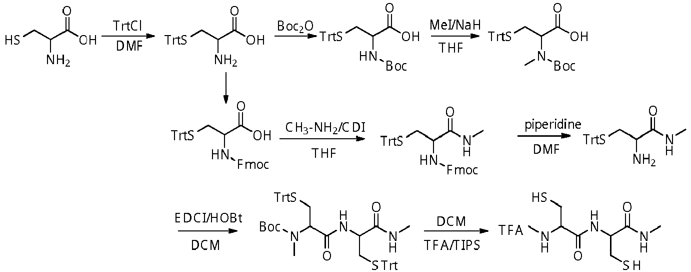

### Step 1: Synthesis of S-trityl-DL-cysteine:

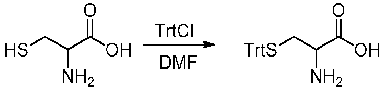

The compound DL-cysteine hydrochloride (10g, 63.45mmol) was dissolved in N,N-dimethylformamide (120ml). Triphenylchloromethane (19.46g, 69.795mmol) was added, heated to 60-65 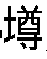 , and reacted for 8h. After the reacti on was completed as detected by T LC, the reaction was cooled to room temperature, and 10% sodium acetate solution (300ml) was added. White solids were then precipitated and filtered. Filter residue was washed with pure water (300ml), then washed with acetone (200ml), and dried to obtain the product as a white solid (17.54g, yield: 76.06%). ¹H NMR (400MHz, DMSO-d6) 7.27(m, 18H), 2.92(dd, 1H), 2.59(dd, 1H), 2.41(dd, 1H).

### Step 2: Synthesis of N-(tert-butoxycarbonyl)-S-trityl-DL-cysteine:

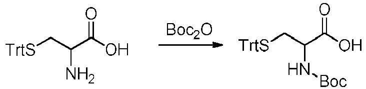

The compound S-trityl-DL-cysteine(5g, 13.76mmol) was dissolved in a mixture of dioxane (40ml), water (20ml) and 1M sodium hydroxide solution (14ml), and stirred in an ice bath. Boc-anhydride (3.5ml, 15.14mmol) was added, then reacted until the mixture was naturally warmed to room temperature, and stirred for 8 hours. After the reaction was completed as detected by T L C, the reaction mixture was concentrated to 20-25ml. Ethyl acetate was added, and the sodi um bi sulfate solution was added dropwi se under the ice bath while sti rri ng. After pH was adj usted to 2-3, ethyl acetate was used for extraction. The organic layer was washed with a saturated saline, then dried with sodi um sulfate, concentrated, and separated by column chromatography (methanol: dichloromethane: 1%-5%) to obtain the product as a white solid (4.72g, yield :73.98%).¹H NMR (400MHz, DMSO-d6) 7.26(m, 16H), 3.77(d, 1H), 2.51(m, 1H), 2.36(dd, 1H), 1.4(d, 9H).

### Step 3: Synthesis of N-(tert-butoxycarbonyl)-N-methyl-S-trityl-DL-cysteine:

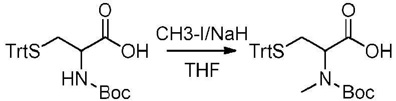

The compound N-(tert-butoxycarbonyl)-S-trityl-DL-cysteine (1g, 2.16mmol) was dissolved in anhydrous tetrahydrofuran (6ml). Sodium hydride (259.2mg, 6.48mmol) was dissolved in anhydrous tetrahydrofuran (10ml). The solution of amino acid in tetrahydrofuran was added dropwise to the solution of sodium hydride in tetrahydrofuran in an ice bath. T hen, methyl iodide (1.08ml, 17.28mmol) was slowly added dropwi se and stirred overnight. After the reaction was completed as detected by TLC, phosphate buffer at pH=7 was added for quenching. pH was adjusted to 6-7 with a saturated ammonium chloride solution, and was extracted with ethyl acetate. The organic layer was washed with a saturated saline, then dried with sodium sulfate, concentrated, and separated by column chromatography (methanol : dichloromethane: 1%-5%) to obtain the product as a white solid (462rrg, yield: 44.85%).¹H NMR (400MHz, DMSO-d6) 7.3(m, 15H), 3.75(s, 1H), 2.7(s, 1H), 2.66(d, 4H), 1.4(d, 9H).

### Step 4: Synthesis of (RS)-2-(((9H-fluoren-9-yl)methoxy)carbonyl)amino)-3-(tritylthio) propionic acid:

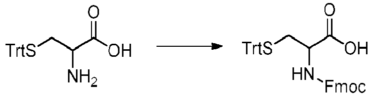

The compound S-trityl-DL-cysteine (5g, 13.76mmol) was dissolved in tetrahydrofuran (30ml) and water (30ml), sodium hydrogen carbonate (2.31g, 27.52mmol) was added with stirring, and 9-fluorenyl methyl-N-succinimidyl carbonate (4.39g, 13mmol) was stirred at room temperature for 3.5 hours. After the reactants were consumed, the reaction mixture was extracted with dichloromethane. The organic layer was washed with a saturated saline, then dried with sodium sulfate, concentrated and separated by column chromatography (methanol: dichloromethane: 1%-5%) to obtain a white solid (6.61g, yield: 82.02%). ¹H NMR (300 MHz, DMSO) 7.89 (d, J = 7.5 Hz, 2H), 7.73 (d, J = 7.5 Hz, 2H), 7.52 ⁻ 7.14 (m, 19H), 4.38 ⁻ 4.09 (m, 3H), 3.84 (dd, J = 8.6, 5.1 Hz, 1H), 2.67 ⁻ 2.56 (m, 1H), 2.41 (dd,J = 12.3, 4.6 Hz, 1H).

### Step 5: Synthesis of (9H-fluoren-9-yl) methyl (RS)-(1-(methylamino)-1-oxo-3-(tritylthio)propan-2-yl) carbamate:

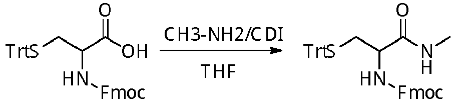

The compound (RS)-2-(((9H-fluoren-9-yl)methoxy)carbonyl)amino)-3-(tritylthio) propionic acid (6.6g, 11.27mmol) was dissolved in tetrahydrofuran (25ml). N,N'-carbonyldiimidazole (3.7g, 22.77mmol) was added at 0-5éC. After stirring for 2 hours under nitrogen atmosphere, methylamine (2ml, 45.08mmol) was added, and reacted at 0-56C for 2 hours. After the reactants were consumed, 2M hydrochloric acid (30ml) was added for quenching, and the reaction mixture was extracted with dichloromethane. The organic layer was washed with a saturated saline, then dried with sodium sulfate and concentrated to obtain a crude product. Methanol (20ml) was added and stirred overnight at room temperature. White solids were precipitated, and filtered to obtain the product in filter residue. The methanol phase was concentrated, and separated by column chromatography (methanol : dichloromethane: 1%-5%) to obtain a white solid (6.5g, yield: 96.32%).¹H NMR (400M Hz, DMSO) 7.89(d, 2H), 7.81(d, 1H), 7.74(d, 2H), 7.66(d, 1H), 7.41(t, 2H), 7.29(m, 17H), 4.31(d, 1H), 4.22(t, 2H), 4.00(d, 1H), 2.53(d, 3H), 2.39(d, 2H).

### Step 6: Synthesis of (RS)-2-amino-N-methyl-3-(tritylthio) propionamide

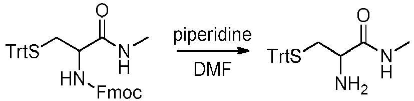

The compound (9H-fluoren-9-yl) methyl (RS)-(1-(methylamino)-1-oxo-3-(tritylthio)propan-2-yl) carbamate (1g, 1.67mmol) was dissolved in N,N-dimethylformarride (20ml). Piperidine (0.03ml, 0.334 mmol) was added and reacted at room temperature for 4 hours. After the reaction was completed as detected by T L C, the reaction mixture was washed with a saturated saline and extracted with dichloromethane. The organic phase was dried with sodium sulfate, concentrated, and separated by column chromatography (methanol : dichloromethane: 1%-5%) to obtain the product as a yellowish white solid (540nrg, yield: 85.88%).¹H NMR (400MHz, CDCl₃) 7.77(d, 1H), 7.29(m, 15H), 3.08(m, 1H), 2.55(d. 3H), 2.37(dd, 1H), 2.19(dd, 1H), 1.80(s, 2H).

### Step 7: Synthesis of tert-butyl methyl ((RS)-1-(((RS)-1-(methylamino)-1-oxo-3-(tritylthio)propan-2-yl)amino)-1-oxo-3-(tritylthio)propan-2-yl) carbamate:

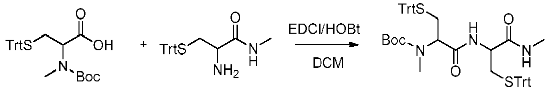

The compound N-(tert-butoxycarbonyl)-N-methyl-S-trityl-DL-cysteine (500g, 1.328mmol) was dissolved in dichloromethane(5ml). 1-hydroxybenzotriazole (269.32rrg, 1.992mmol) and EDCI (381.87rrg, 1.992mmol) were added, and stirred at room temperature for 5min. (RS)-2-amino-N-methyl-3-(tritylthio) propanamide (634rrg, 1.328mmol) was added and stirred at room temperature for 30 minutes. After the reaction was completed as detected by TLC, the rri xture was washed with a saturated sal i ne and extracted with dichloromethane. The organic phase was dried with sodium sulfate, then concentrated and purified with T L C (dichloromethane: methanol: 15: 1) to obtain the product as a white solid (940mg, yield: 84.66%).¹H NMR (400MHz, CDCl₃) 7.39(m, 12H), 7.22(m, 20H), 4.1(d, 1H), 3.95(s, 1H), 2.61(dd, 10H), 1.39(s, 9H).

### Step 8: Synthesis of (RS)-3-mercapto-N-((RS)-3-mercapto-1-(methylamino)-1-oxopropan-2-yl)-2-(methylamino) propionarride trifluoroacetate:

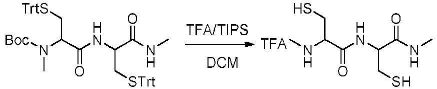

The compound tert-butyl methyl ((RS)-1-(((RS)-1-(methylamino)-1-oxo-3-(tritylthio)propan-2-yl)anrino)-1-oxo-3-(tritylthio)propan-2-yl) carbamate (200rrg, 0.239mmol) was dissolved in dichloromethane: trifluoroacetic acid: triisopropylsilane (50 : 47 : 3 by volume) (5ml), stirred at room temperature for 5min. After the reaction was completed as detected by T L C, the mixture was concentrated, diethyl ether was added, and stirred in an ice bath. White solids were precipitated, filtered and dried to obtain the product (70rrg, yield: 84.07%).¹H NMR (400MHz, DMSO) 8.89(d,J =8.1 Hz, 2H), 8.12 (d, J = 4.3 Hz, 1H), 4.48 ⁻ 4.34(m, 1H),4.04(s, 1H), 3.10 (dt, J = 12.5, 6.2 Hz, 1H), 3.04 ⁻ 2.93 (m, 1H), 2.91⁻2.81 (m, 1H), 2.78⁻2.65 (m, 1H), 2.62 (d, J = 4.6 Hz, 3H), 2.56 (s, 3H), 2.37 (t, J = 13.7 Hz, 1H), 1.29 (d, J = 7.0 Hz, 1H).

### Example 20: Synthesis of 2-amino-N-(1-((1-amino-3-mercapto-1-oxopropan-2-yl)amino)-3-mercapto-1-oxopropan-2-yl)-3-mercaptopropylamide trifluoroacetate

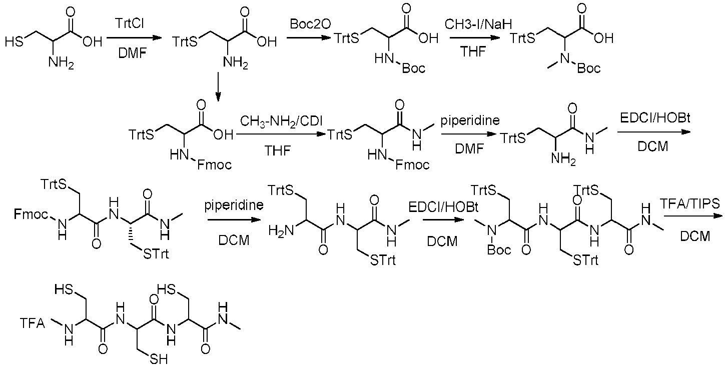

### Step 1: Synthesis of S-trityl-DL-cysteine:

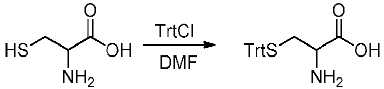

The compound DL-cysteine hydrochloride (10g, 63.45mmol) was dissolved in N,N-dimethylformamide (120ml). Triphenylchloromethane (19.46g, 69.795mmol) was added, heated to 60-65 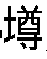 , and reacted for 8h. After the reacti on was completed as detected by T LC, the reaction was cooled to room temperature, and 10% sodium acetate solution (300ml) was added. White solids were then precipitated and filtered. Filter residue was washed with pure water (300ml), then washed with acetone (200ml), and dried to obtain the product as a white solid (17.54g, yield: 76.06%). ¹H NMR (400MHz, DMSO-d6) 7.28(m, 18H), 2.92(dd, 1H), 2.59(dd, 1H), 2.41(dd, 1H).

### Step 2: Synthesis of N-(tert-butoxycarbonyl)-S-trityl-DL-cysteine:

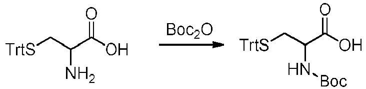

The compound S-trityl-DL-cysteine(5g, 13.76mmol) was dissolved in a mixture of dioxane (40ml), water (20ml) and 1M sodium hydroxide solution (14ml), and stirred in an ice bath. Boc-anhydride (3.5ml, 15.14mmol) was added, then reacted until the mixture was naturally warmed to room temperature, and stirred for 8 hours. After the reaction was completed as detected by T LC, the reaction mixture was concentrated to 20-25ml. Ethyl acetate was added, and the sodi um bisulfate sol uti on was added dropwi se under the ice bath while sti rri ng. After pH was adj usted to 2-3, ethyl acetate was used for extraction. The organic layer was washed with a saturated saline, then dried with sodium sulfate, concentrated, and separated by column chromatography (methanol : dichloromethane: 1%-5%) to obtain the product as a white solid (4.72g, yield :73.98%).¹H NMR (400MHz, DMSO-d6) 7.26(m, 16H), 3.78(d, 1H), 2.51(m, 1H), 2.36(dd, 1H), 1.4(d, 9H).

### Step 3: Synthesis of N-(tert-butoxycarbonyl)-N-methyl-S-trityl-DL-cysteine:

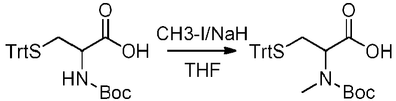

The compound N-(tert-butoxycarbonyl)-S-trityl-DL-cysteine (1g, 2.16mmol) was dissolved in anhydrous tetrahydrofuran (6ml). Sodium hydride (259.2mg, 6.48mmol) was dissolved in anhydrous tetrahydrofuran (10ml). The solution of amino acid in tetrahydrofuran was added dropwise to the solution of sodium hydride in tetrahydrofuran in an ice bath. T hen, methyl iodide (1.08ml, 17.28mmol) was slowly added dropwi se and stirred overnight. After the reaction was completed as detected by TL C, phosphate buffer at pH=7 was added for quenching. pH was adjusted to 6-7 with a saturated ammonium chloride solution, and was extracted with ethyl acetate. The organic layer was washed with a saturated saline, then dried with sodium sulfate, concentrated, and separated by column chromatography (methanol : dichloromethane: 1%-5%) to obtain the product as a white solid (462rrg, yield: 44.85%).¹H NMR (400MHz, DMSO-d6) 7.3(m, 15H), 3.75(s, 1H), 2.8(s, 1H), 2.66(d, 4H), 1.4(d, 9H).

### Step 4: Synthesis of (RS)-2-(((9H-fluoren-9-yl)methoxy)carbonyl)amino)-3-(tritylthio) propionic acid:

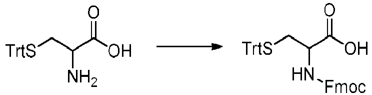

The compound S-trityl-DL-cysteine (5g, 13.76mmol) was dissolved in tetrahydrofuran (30ml) and water (30ml), sodium hydrogen carbonate (2.31g, 27.52mmol) was added with stirring, and 9-fluorenyl methyl-N-succinimidyl carbonate (4.39g, 13mmol) was stirred at room temperature for 3.5 hours. After the reactants were consumed, the reaction mixture was extracted with dichloromethane. The organic layer was washed with a saturated saline, then dried with sodium sulfate, concentrated and separated by column chromatography (methanol: dichloromethane: 1%-5%) to obtain a white solid (6.61g, yield: 82.02%). ¹H NMR (300 MHz, DMSO) 7.89 (d, J = 7.5 Hz, 2H), 7.73 (d, J = 7.5 Hz, 2H), 7.52 ⁻ 7.14 (m, 19H), 4.38 ⁻ 4.09 (m, 3H), 3.84 (dd, J = 8.6, 5.1 Hz, 1H), 2.67 ⁻ 2.56 (m, 1H), 2.41 (dd,J = 12.3, 4.6 Hz, 1H).

### Step 5: Synthesis of (9H-fluoren-9-yl) methyl (RS)-(1-(methylamino)-1-oxo-3-(tritylthio)propan-2-yl) carbamate:

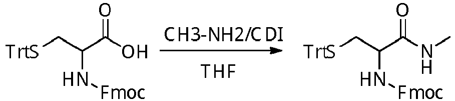

The compound (RS)-2-(((9H-fluoren-9-yl)methoxy)carbonyl)amino)-3-(tritylthio) propionic acid (6.6g, 11.27mmol) was dissolved in tetrahydrofuran (25ml). N,N'-carbonyldiimidazole (3.7g, 22.77mmol) was added at 0-5éC. After stirring for 2 hours under nitrogen atmosphere, methylamine (2ml, 45.08mmol) was added, and reacted at 0-56C for 2 hours. After the reactants were consumed, 2M hydrochloric acid (30ml) was added for quenching, and the reaction mixture was extracted with dichloromethane. The organic layer was washed with a saturated saline, then dried with sodium sulfate and concentrated to obtain a crude product. Methanol (20ml) was added and stirred overnight at room temperature. White solids were precipitated, and filtered to obtain the product in filter residue. The methanol phase was concentrated, and separated by column chromatography (methanol : dichloromethane: 1%-5%) to obtain a white solid (6.5g, yield: 96.32%).¹H NMR (400M Hz, DMSO) 7.89(d, 2H), 7.81(d, 1H), 7.74(d, 2H), 7.66(d, 1H), 7.41(t, 2H), 7.29(m, 17H), 4.31(d, 1H), 4.22(t, 2H), 4.00(d, 1H), 2.53(d, 3H), 2.39(d, 2H).

### Step 6: Synthesis of (RS)-2-amino-N-methyl-3-(tritylthio) propionamide

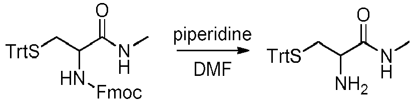

The compound (9H-fluoren-9-yl) methyl (RS)-(1-(methylamino)-1-oxo-3-(tritylthio)propan-2-yl) carbamate (1g, 1.67mmol) was dissolved in N,N-dimethylformarride (20ml). Piperidine (0.03ml, 0.334 mmol) was added and reacted at room temperature for 4 hours. After the reaction was completed as detected by T L C, the reaction mixture was washed with a saturated saline and extracted with dichloromethane. The organic phase was dried with sodium sulfate, concentrated, and separated by column chromatography (methanol: dichloromethane: 1%-5%) to obtain the product as a yellowish white solid (540nrg, yield: 85.88%).¹H NMR (400MHz, CDCl₃) 7.77(d, 1H), 7.29(m, 15H), 3.08(m, 1H), 2.55(d. 3H), 2.37(dd, 1H), 2.19(dd, 1H), 1.80(s, 2H).

### Step 7: Synthesis of (9H-fluoren-9-yl) methyl ((RS)-1-(((RS)-1-(methylamino) -1-oxo-3-(tritylthio)propan-2-yl)amino)-1-oxo-3-(tritylthio)propan-2-yl) carbamate:

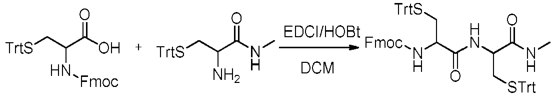

The compound (RS)-2-((((9H-fluoren-9-yl)methoxy)carbonyl)amino)-3-(tritylthio) propanoic acid (1g, 1.71mmol) was dissolved in dichloromethane (15ml). 1-hydroxybenzotriazole (347rrg, 2.565 mmol) and EDCI (492mg, 2.565mmol) were added, and stirred at room temperature for 5min. (R)-2-amino-N-methyl-3-(tritylthio) propanamide (644rrg, 1.71 mmol) was added and reacted at room temperature for 30 rri nutes. After the reaction was completed as detected by T L C, the mixture was washed with a saturated saline and extracted with dichloromethane. The organic phase was dried with sodium sulfate, then concentrated, and separated by column chromatography (methanol : dichloromethane: 1%-5%) to obtain the product as a white solid (1.06g, yield: 65.63%).¹H NMR (400MHz, DMSO-d6) 7.89(d, 2H), 7.71(m, 4H), 7.40(m, 2H), 7.38-7.25(m, 30H), 4.25(m, 4H), 4.01(m, 1H), 2.50-2.33(m, 7H).

### Step 8: Synthesis of (RS)-2-amino-N-((RS)-1-(methylamino)-1-oxo-3-(tritylthio)propan-2-yl)-3-(tritylthio) propionamide:

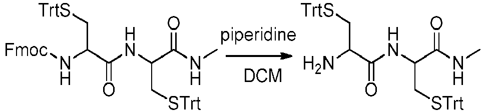

The compound (9H-fluoren-9-yl) methyl ((RS)-1-(((RS)-1-(methylamino)-1-oxo-3-(tritylthio)propan-2-yl) amino)-1-oxo-3-(tritylthio)propan-2-yl) carbamate (1.06g, 1.12mmol) was dissolved in N,N-dimethylformamide (10ml). Piperidine (0.02ml, 0.224mmol) was added and reacted at room temperature for 4 hours. After the reacti on was completed as detected by T L C detecti on, the reacti on rri xture was washed with a saturated saline and extracted with dichloromethane. The organic phase was dried with sodium sulfate, then concentrated, and separated by column chromatography (methanol: dichloromethane: 1%-5%) to obtain the product as a white solid (790rrg, yield: 97.7%).¹H NMR (400MHz, DMSO-d6) 8.12(s, 1H), 7.83(d, 1H), 7.27(m, 30H), 4.25(s, 1H), 3.29(m, 2H), 3.20(s, 1H), 2.65-2.23(m, 5H).

### Step 9: Synthesis of tert-butyl methyl((4RS,7RS,10RS)-3,6,9-trioxo-13,13,13-triphenyl-4,7-bis((tritylthio)methyl)-12-thia-2,5,8-triazatridec-10-yl) carbamate:

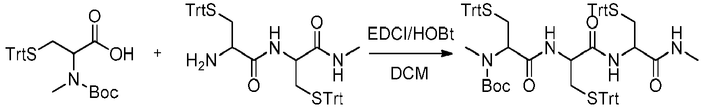

The compound N-(tert-butoxycarbonyl)-N-methyl-S-trityl-DL-cysteine (505rrg, 1.06mmol) was dissolved in dichloromethane(10ml). 1-hydroxybenzotriazole(215rrg, 1.59mmol) and EDCI (304.8rrg, 1.59mmol) were added, and stirred at room temperature for 5min. (RS)-2-amino-N-((RS)-1-(methylamino)-1-oxo-3-(tritylthio)propan-2-yl)-3-(tritylthio) propanamide (765.3mg, 1.06mmol) was added and reacted at room temperature for 30 minutes. After the reaction was completed as detected by T L C, the mixture was washed with a saturated saline and extracted with dichloromethane. The organic phase was dried with sodium sulfate, concentrated, and separated by column chromatography (methanol : dichloromethane: 1%-5%) to obtain the product as a white solid (1.1g, yield: 88%).¹H NMR (400MHz, DMSO-d6) 8.11(d, 1H), 7.73(d, 2H), 7.32-7.21(rn 45H), 4.25(m, 3H), 2.62(m, 1H), 2.49(m, 6H) 2.47-2.23(m, 5H), 1.35-1.21(d, 9H).

### Step 10: Synthesis of (RS)-3-mercapto-N-((RS)-3-mercapto-1-(((RS)-3-mercapto-1-(methylamino)-1-oxopropan-2-yl)amino)-1-oxopropan-2-yl)-2-(methylamino) propanamide trifluoroacetate:

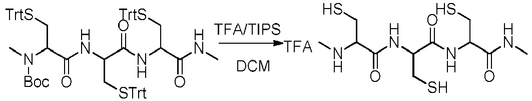

The compound tert-butyl methyl((4RS,7RS,10RS)-3,6,9-trioxo-13,13,13-triphenyl-4,7-bis((tritylthio)methyl)-12-thia-2,5,8-triazatridec-10-yl) carbamate (400rrg, 0.339mmol) was dissolved in dichloromethane: trifluoroacetic acid : triisopropylsilane (50 : 47 : 3 by volume) (10ml), stirred at room temperature for 5min. After the reaction was completed as detected by T L C, the mixture was concentrated, diethyl ether was added, and stirred in an ice bath. White solids were precipitated, filtered and dried to obtain the product (131mg, yield: 85.4%).¹H NMR (400 MHz, DMSO) 8.92 (dd, J = 14.8, 9.8 Hz, 3H), 8.58 ⁻ 8.33 (m, 1H), 7.97 (dd,J = 22.8, 4.4 Hz, 1H), 4.64⁻4.50 (m, 1H), 4.34 (td,J = 13.4, 7.9 Hz, 1H), 4.05 (d, J = 5.4 Hz, 1H), 3.17 ⁻ 2.66 (m, 7H), 2.60 (d, J = 3.9 Hz, 3H), 2.56 (d, J = 5.7 Hz, 3H), 2.36 ⁻ 2.27 (m, 1H).

### Examples of biological activity:

Example A: The protective effects of compounds 1, 2, 3 and 4 on survival rate, white blood cells and organs of mice 30 days after i rradi ati on.

Material: The gamma ray irradiation device is a ¹³⁷Cs irradiator, with a dose rate of 0.7Gy/min. C57B L/6 mice, male, weighing 21-22 g, purchased from Beijing HFK Bioscience Co. Ltd., certificate number SCXK (Beijing) 2014-0004, grouped as: no irradiation group, irradiation and blank solvent group, irradiation and administration group, with 5 mice in each group. The structures of compounds 1, 2, 3 and 4 (prepared from Examples 1, 7, 2 and 3, respectively) are shown in Table 1.

**Table 1 Nomenclature and structural formula of compounds 1-4**

| Compound No. | Compound name | Structural formula |
|---|---|---|
| Compound 1 (Example 1) | (R)-2-amino-N-((R)-1-amino-3-mercapto-1-oxopropan-2-yl)-3-mercaptopropanamide trifl uoroacetate | 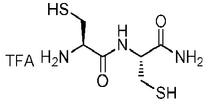 |
| Compound 2 (Example 7) | (S)-3-mercapto-N-((S)-3-mercapto-1-(methylamino)-1-oxopropan-2-yl)-2-(methylamino) propionamide trifluoroacetate | 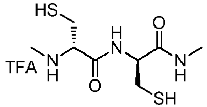 |
| Compound 3 (Example 2) | (R)-3-mercapto-N-((R)-3-mercapto-1-(methylamino)-1-oxopropan-2-yl)-2-(methylamino) propionamide trifluoroacetate | 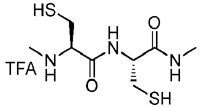 |
| Compound 4 (E xampl e 3) | (R)-2-amino-N-((R)-1-(((R)-1-amino-3-mercapto-1-oxopropan-2-yl)amino)-3-mercapto-1 -oxopropan-2-yl)-3-mercaptopropanamide trifluoroacetate | 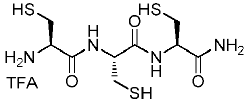 |

Method: Irradiation and drug treatment: ¹³⁷Cs 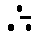 ray irradiation was carried out on the whole body at an irradiation dose rate of 0.7Gy/min, and the absorbed dose of mice was 6.8Gy, 7.2Gy and 7.5Gy, respectively; aminofostine, compound 1, compound 2, compound 3, and compound 4 were dissolved in normal saline, shaken evenly when administering them (200mg/kg BW), and injected intraperitoneally 30min before irradiation. The 30-day survival of the mice in each group was observed, the survival rates were calculated, the body weights were followed, and the organs and white blood cells of mi ce in each group were compared after 30 days.

Results: At the same administration dose of the four compounds (200mg/kg, one dose at 24 hours before irradiation and one dose at 30 minutes before irradiation), one-time whole body irradiation with 6.8Gy, 7.2Gy and 7.5Gy ¹³⁷Cs gamma rays was performed, respectively, compared with the radiation and blank solvent group. The 30-day survival rate of mice was 100% when compound 1 was used in the cases of irradiations of 6.8Gy and 7.2Gy, respectively. The survival rate and body weight of mice in each group with different irradiation doses are shown in Figures 1, 3 and 5. The effects of normal control group and compound 1, compound 2, compound 3 and compound 4 on organs and leukocytes in mice 30 days after irradiation with 7.2Gy are shown in Figure 4. The effects of normal control group and compound 1 on organs and leukocytes in mice 30 days after irradiation with 6.8Gy and 7.5Gy, respectively, are shown in Figures 2 and 6.

Example B: This example studies the effects of different compounds (i.e., Example 17, Example 2, Example 3, Example 18, Example 8, Example 1, Example4) on the survival rate of mice without radiation dose, respectively.

Laboratory animals: C57 male mice, 8-10 weeks old, 20-22g, were divided into 4 groups, 5 or 10 or 15 or 20 or 25 mice in each group. Irradiation conditions: 137Cs- ∴rays, the dose rate was 0.7Gy/min, and the absorbed doses of mice were 6.8Gy, 7.2Gy and 7.5Gy, respectively, one-time whole body irradiation (if not specified, the irradiation rays were all 137Cs- ∴rays)

### Administration and grouping:

In normal control group, intraperitoneal injection of normal saline was performed.

In i rradi ati on control group, intraperitoneal injection of normal sal i ne was performed 30min before the irradiation.

In amifostine + 6.8Gy (or 7.2Gy or 7.5Gy) group, intraperitoneal injection of amifostine (dissolved in normal saline, at a dosage of 200mpk) was performed 30min before the irradiation.

In Example 17 + 6.8Gy (or 7.2Gy or 7.5Gy) group, Example 17 (dissolved in normal saline, at a dosage of 200mpk) was intraperitoneally injected 30min before the irradiation.

Observation index: The death and weight of the mice were recorded, and the survival rate was calculated on the 30^{th} day. The surviving mice were sacrificed, dissected, and each organ index (the weight of each organ as a percentage of the body weight of the mouse) was calculated.

### Experimental results:

| Group | 6.8Gy (Survival number /Test number Survival rate) | | 7.2Gy (Survival number /Test number Survival rate) | | 7.5Gy (Survival number/Test number Survival rate) | |
|---|---|---|---|---|---|---|
| Irradiation control group | 3/5 | 60% | 1/10 | 10% | 0/5 | 0% |
| A mifosti ne, 200mpk, IP | 5/5 | 100% | 9/10 | 90% | 5/5 | 100% |
| Example 17, 200mpk, IP | 5/5 | 100% | 9/10 | 90% | 0/5 | 0% |

The experimental operation was the same as the above, for the effects of different dosages on the survival rate of mice, except that the intraperitoneal injection of Example 17 was performed 90min before the irradiation.

| Group | 7.5Gy (Survival number /Test number Survival rate) | |
|---|---|---|
| 7.5Gy irradiation group | 0/5 | 0% |
| Amifostine, 200mpk, IP | 4/5 | 80% |
| Example 17, 400mpk, IP | 0/5 | 0% (intraperitoneal injection 90min before irradiation) |
| Example 17, 800mpk, IP | 0/5 | 0% i ntraperi toneal injection 90min before irradiation |

The i rradi ati on was invalid 90 mi notes after admi ni strati on.

Note: In the toxicity test of Example 17, muscle tremors and other toxic reactions were observed after i ntraperi toneal injection of 400mpk and 800mpk, followed by i rradi ati on 90min after admi ni strati on.

Example 2, Example 3, E xampl e 18, E xampl e 8, E xampl e 1 and Example 4 were tested at different absorbed doses using the above similar condi ti ons or operati ons. The experimental resul is are as follows:

| Group | 7.2Gy (Survival number/Test number Survival rate) | |
|---|---|---|
| 7.2Gy irradiation group | 1/15 | 6.67% |
| Amifostine, 200mpk, IP | 12/15 | 80% |
| Example 2, 200mpk, IP | 10/15 | 66.67% |

| Group | 7.2Gy (Survival number /Test number Survival rate) | |
|---|---|---|
| 7.2Gy irradiation group | 1/15 | 6.67% |
| Amifostine, 200mpk, IP | 12/15 | 80% |
| Example 3, 200mpk, IP | 10/15 | 66.67% |

| Group | 7.2Gy (Survival number /Test number Survival rate) | |
|---|---|---|
| 7.2Gy irradiation group | 0/5 | 0% |
| Amifostine, 200mpk, IP | 4/5 | 80% |
| Example 18, 200mpk, IP | 4/5 | 80% |

| Group | 7.2Gy (Survival number /Test number Survival rate) | |
|---|---|---|
| 7.2Gy irradiation group | 0/5 | 0% |
| Amifostine, 200mpk, IP | 4/5 | 80% |
| Example 8, 200mpk, IP | 1/5 | 20% |

| Group | 7.2Gy (Survival number /Test number Survival rate) | |
|---|---|---|
| 7.2Gy irradiation group | 0/10 | 0% |
| Amifostine, 200mpk, IP | 7/10 | 70% |
| Example 1, 200mpk, IP | 6/10 | 60% |

| Group | 7.2Gy (Survival number /Test number Survival rate) | |
|---|---|---|
| Irradiation control group | 4/25 | 16% |
| Amifostine, 200mpk, IP | 20/25 | 80% |
| Example4, 200mpk, IP | 20/25 | 80% |

| Group | 7.5Gy (Survival number /Test number Survival rate) | |
|---|---|---|
| 7.5Gy irradiation group | 0/10 | 0% |
| Amifostine, 200mpk, IP | 1/10 | 10% |
| E xampl e 4, 800mpk, IP | 7/10 | 70% |

| Group | 7.5Gy (Survival number /Test number Survival rate) | |
|---|---|---|
| 7.5Gy irradiation group | 2/10 | 20% |
| Amifostine, 365mpk, IP | 10/10 | 100% |
| Example 4, 517mpk, IP | 9/10 | 90% |

| Group | 10Gy (Survival number /Test number Survival rate) | |
|---|---|---|
| 10Gy irradiation group | 0/20 | 0% |
| Amifostine, 517mpk, IP | 15/20 | 75% |
| E xampl e 4, 365mpk, IP | 15/20 | 75% |

| Group | 12.5Gy (Survival number /Test number Survival rate) | |
|---|---|---|
| 12.5Gy irradiation group | 0/10 | 0% |
| Amifostine, 517mpk, IP | 5/10 | 50% |
| E xampl e 4, 365mpk, IP | 4/10 | 40% |

The above results indicated that the mice using compounds of Example 17, Example 2, Example 3, Example 18, Example 8, Example 1, and Example4 had survival rates comparable to those using amifostine when the absorbed dose was 6.8Gy and 7.2Gy, among which the mice using the compound of Example4 had a survival rate equivalent to those using amifostine when the absorbed dose was even higher, such as as high as 12.5Gy.

### Example C: Acute toxicity test:

Laboratory animals: C57 male mice, 8-10 weeks old, 20-22g, 5 or 10 mice in each group.

Administration and grouping: Each group was intraperitoneally injected with one corresponding dose of amifostine.

Each group was intraperitoneally injected with one corresponding dose of Example 17 (IP).

The 30-day survival rate of mice in each group was recorded. The results are shown in the table:

| Dosage (mpk) | 400mpk (Surviva I number /Test number) | 600mpk (Surviva I number /Test number) | 800rrpk (Surviva I number /Test number) | 900mpk (Surviva I number /Test number) | 1000mp k (Surviva I number /Test number) | 1100mp k (Surviva I number /Test number) | 1200mp k (Surviva I number /Test number) | 1600mp k (Surviva I number /Test number) | 3200mp k (Surviva I number /Test number) |
|---|---|---|---|---|---|---|---|---|---|
| A mi fosti n e (IP) | 5/5 | 5/5 | 3/5 | NA | NA | NA | NA | 0/5 | NA |
| Example 17 (IP) | 5/5 | NA | 5/5 | NA | NA | NA | NA | 415 | NA |
| Example 4 (IP) | 10/10 | 5/5 | 10/10 | 10/10 | 6/6 10/10 | 8/10 | 6/10 | 3/10 | NA |

Acute toxicity tests indicated that the compound of the present invention, especially Example4, was as safe as or even better than ami fosti ne at high doses, in which the compound of E xarrpl e 4 even had a 100% survival rate at a dose of 1000mpk.

### Example D: Protective effect of the compounds in the present invention on the hematopoietic system:

Laboratory animals: C57 male mice, 8-10 weeks old, 20-22g, 5 mice in each group.

Administration and grouping: In normal control group, intraperitoneal injection of normal saline was performed, without irradiation.

In administration control group, intraperitoneal injection of Example 4 (800mpk) was performed, without irradiation.

In irradiation control group, intraperitoneal injection of normal saline was performed 30min before the i rradi ati on at 4Gy.

In irradiation administration group, intraperitoneal injection of Example 4 (800mpk) was performed 30rri n before the i rradi ati on at 4Gy.

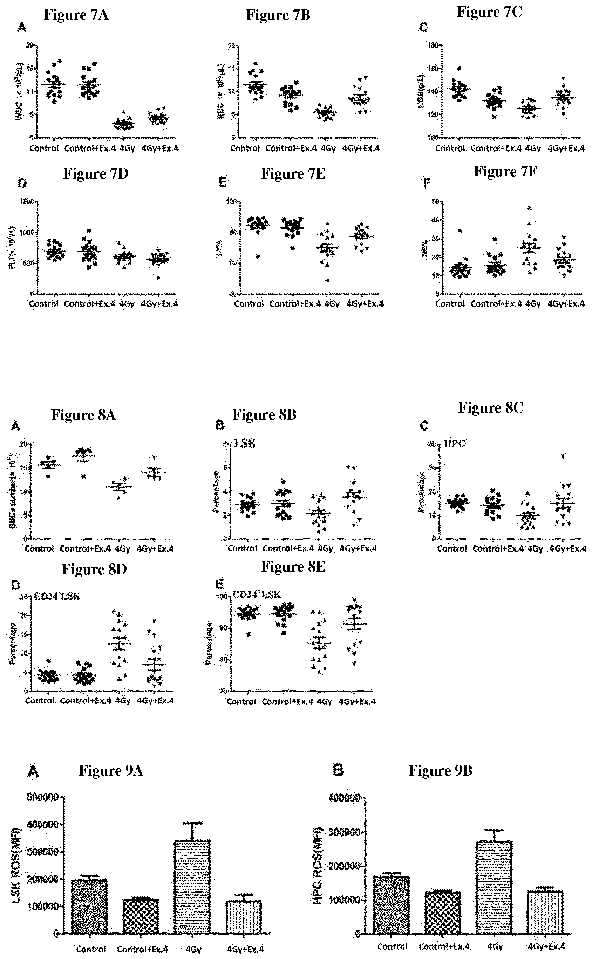

The mice were sacrificed on the 15^{th} day after the irradiation, followed by blood collection. Femoral bone marrow cells, spleen and thymus were weighed, and cells were extracted for detecting the peripheral white blood cell (WBC) count, peripheral red blood cell (RBC) count, peripheral hemoglobin (HGB) concentration, peripheral platelet (PLT) count, peripheral blood lymphocyte ratio (LY %), and peripheral blood neutrophilratio (N E %). The results are shown in Figure 7. The results showed that the number of WBC and RBC in the peripheral blood of mice was reduced by irradiation, and the number of these cells was increased by administration of Example4 with significant difference; irradiation reduced LY % and increased NE% in peripheral blood of mice, and administration of Example4 alleviated this abnormal differentiation with significant difference.

In addition, the number of leukocytes in bone marrow, ratio of hematopoietic stem cell (L SK) in bone marrow cells, ratio of hematopoietic progenitor cells (HPC) in bone marrow cells, ratio of C D34-LSK in bone marrow cells, and ratio of CD 34+LSK i n bone marrow cell s were detected. The results are shown in Figure 8. The results showed that the number of W B C, LSK% and HPC% in the bone marrow of mi ce was decreased by irradiation, and the number of these cells was increased by administration of Example 4 with significant difference; irradiation increased CD34-LSK% and decreased CD34+LSK% in the bone marrow of mice, and administration of Example4 alleviated this abnormal differentiation with significant difference.

The effects of irradiation on LSK intracellular reactive oxygen species (ROS) level and H PC intracellular ROS level were also detected. The results are shown in Figure 9. The results showed that irradiation increased the ROS levels in LSK and HPC cells in the bone marrow of mice, and administration of E xampl e 4 reduced the ROS levels with significant difference.

### Example E: Protective effect of the compounds of the present invention on the intestinal tract

### Example E 1: Survival rate of radiation intestinal damage

Laboratory animals: C57 male mice, 8-10 weeks old, 20-22g, 5 mice in each group.

Administration and grouping: In irradiation control group, intraperitoneal injection of normal saline was performed 30min before the i rradi ati on, with local abdominal irradiation at 18Gy.

In amifostine group, intraperitoneal injection was performed 30min before the local abdominal irradiation at 18Gy, wherein ami fosti ne was dissolved in normal saline, 200mpk.

In Example4 group, intraperitoneal injection was performed 30min before the local abdominal irradiation at 18Gy, wherein Example4 was dissolved in normal saline, 800mpk.

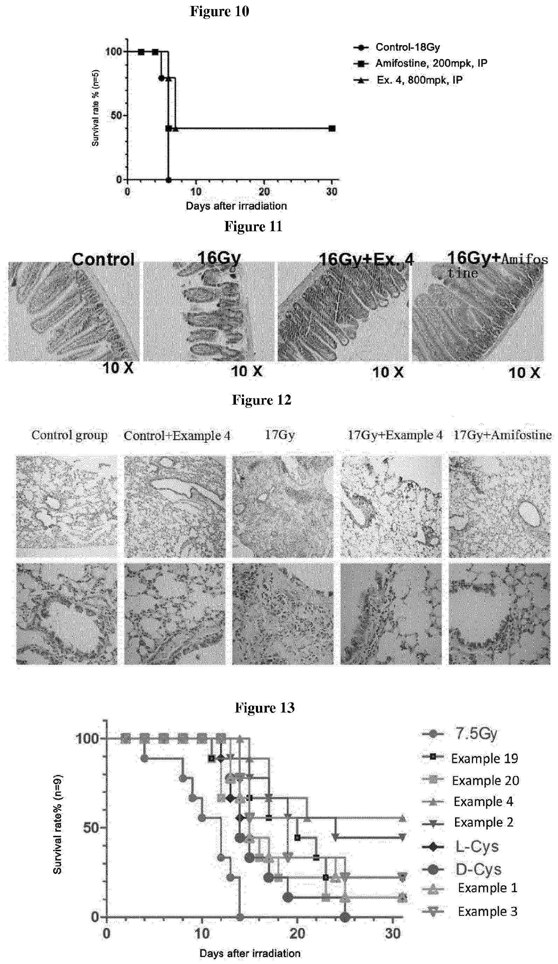

The 30-day survival rate of mice in each group was recorded. The results are shown in Figure 10.

### Example E 2: HE staining on the 5^{th} day after local intestinal irradiation

### Laboratory animals: C57 male mice, 8-10 weeks old, 20-22g, 3 mice in each group

Administration and grouping: In normal control group, intraperitoneal injection of normal saline was performed, with false irradiation.

In irradiation control group, intraperitoneal injection of normal saline was performed 30min before the irradiation, with local abdominal irradiation at 16Gy

In amifostine group, intraperitoneal injection was performed 30min before the local abdominal irradiation at 16Gy, wherein amifostine was dissolved in normal saline, 365mpk.

In Example4 group, intraperitoneal injection was performed 30min before the local abdominal irradiation at 16Gy, wherein Example4 was dissolved in normal saline, 517mpk.
The mice were sacrificed on the 5^{th} day after the i rradi ati on, and the small i ntesti ne was taken and sectioned for HE staining. The results are shown in Figure 11, and Figure 11 shows that the normal unirradiated intestinal villi had clear and dense structures; the structure of villi in the small intestine changed after i rradi ati on; the i ntesti nal villi in the Example 4 irradiation group and the ami fosti ne irradiation group showed slight changes compared with the irradiation group.

### Example F: Effect of Example4 on radiation pneumonia:

Laboratory animals: C57 male mice, 8-10 weeks old, 20-22g, 3 mice in each group

Administration and grouping: Mice were intraperitoneally injected with normal saline, Example4 (400mpk), and amifostine (200mpk) respectively 30rrin before irradiation, and the right lungs were irradiated at 17Gy by X rays. The right lungs were taken on the 60^{th} day for HE staining (n=3):
Two months after irradiation of the unilateral lung, the most important lung manifestation was radiation pneumonia. The lung tissues in mice of the control group showed obvious vacuolar structure, and no alveolar wall thickening was observed. The alveolar structure of mice in the 17Gy group was changed, which was manifested as a large area of inflammatory infiltration of the lung (the blue dots were inflammatory cells), and the alveoli were filled with inflammatory cells. Both amifostine and Example4 could partially relieve inflammatory cell infiltration and reduce the incidence of radiation pneumonia. The results are shown in Figure 12.

### Example G: This example investigated the effect of stereoisomerism on survival rate

In this Example, the effects of compounds, such as Example 1

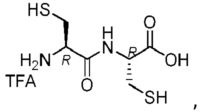

### Example 2

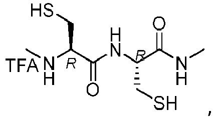

E xampl e 3 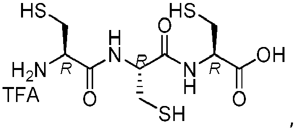 Example 4 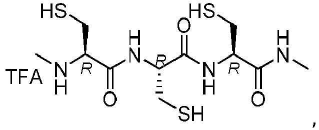 Example 19 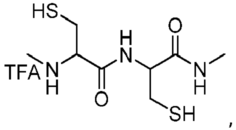 Example 20 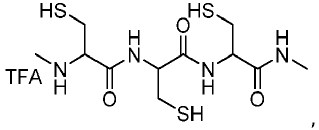 as well as L -cystei ne 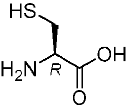 and D-cysteine 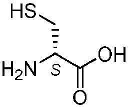 on the survival rate of mice under irradiation were further studied.

Laboratory animals: C57 male mice, 19-21g, purchased from Beij ing HFK Bioscience Co. Ltd., license number SCX K (Beijing), 9 mice in each group.

Administration and grouping: In 7.5Gy irradiation control group, intraperitoneal injection of normal saline was performed 30min before the irradiation.

The other admi ni strati on groups were treated by i ntraperi toneal i nj ecti on of normal saline solutions of the corresponding drugs, 200mg/kg, 30min before the irradiation.

The experimental results are shown in the table below and Figure 13:

| | 7.5Gy (Survival number / Test number) | | 7.5Gy (Survival number / Test number) | | 7.5Gy (Survival number / Test number) |
|---|---|---|---|---|---|
| Irradiation control group | 0/9 (9 deaths) | | | | |
| L-Cys | 1/9 (8 deaths) | | | | |
| D-Cys | 0/9 (9 deaths) | | | | |
| Example 4 | 5/9 (4 deaths) | Example 20 | 1/9 (8 deaths) | Example 3 | 2/9 (7 deaths) |
| Example 2 | 4/9 (5 deaths) | Example 19 | 2/9 (7 deaths) | Example 1 | 1/9 (8 deaths) |

The survival rate in the case of Example 4 was approximately 56%, significantly higher than that of cysteine trimer of Example 3 (approximately 22%) and the racemate of Example 20; the survival rate in the case of Example 2 was approximately 44%, significantly higher than that of cysteine dimer of Example 1 (approximately 11%) and the racemate of Example 19. It indicates that N-methylation of cysteine polymer can significantly improve the survival rate of irradiated mice and further enhance the irradiation protection ability of the compounds.

## Claims

1. A compound of formula (I): 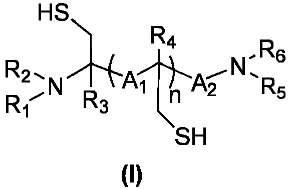 wherein
A₁ is selected from: -C(O)NR⁸-, and -R⁷-NR⁸-;
A₂ is selected from: carbonyl, and unsubstituted C₁₋₆ alkylene;
R¹ is selected from: hydrogen, and unsubstituted C₁-C₅ alkyl;
R² is unsubstituted C₁-C₅ alkyl;
R⁵ is selected from: hydrogen, unsubstituted C₁-C₅ alkyl;
R⁶ is unsubstituted C₁-C₅ alkyl;
n is an integer from 1 to 10;
R³ and R⁴ are independently selected from: hydrogen, and unsubstituted C₁₋₆ alkyl;
R⁷ is unsubstituted C₁-C₆ alkylene;
R⁸ is selected from: hydrogen, and unsubstituted C₁-C₆ alkyl;
or a stereoisomer thereof or a pharmaceutically acceptable salt, or a solvate thereof.

2. The compound of claim 1, wherein A₂ is selected from: carbonyl, and unsubstituted C₁₋₃ alkylene;
R¹ is selected from: hydrogen, and C₁₋₃ alkyl;
R² is C₁₋₃ alkyl;
R⁵ is selected from: hydrogen, and C₁₋₃ alkyl;
R⁶ is C₁₋₃ alkyl;
n is an integer from 1 to 10;
R³ and R⁴ are independently selected from: hydrogen, and unsubstituted C₁₋₃ alkyl;
R⁷ is unsubstituted C₁-C₃ alkylene; and
R⁸ is selected from: hydrogen, and unsubstituted C₁-C₃ alkyl;
wherein the chiral carbon directly attached to R³ and R⁴ is in R configuration or S configuration;
or a stereoisomer thereof or a pharmaceutically acceptable salt, or a solvate thereof.

3. The compound of claim 1 or 2, wherein A₂ is selected from: carbonyl, and methylene;
R¹ is selected from: hydrogen, methyl, and ethyl;
R² is selected from: methyl, and ethyl;
R⁵ is selected from: hydrogen, methyl, and ethyl;
R⁶ is selected from: methyl, and ethyl;
n is an integer from 1 to 10;
R³ and R⁴ are independently selected from: hydrogen, and methyl;
R⁷ is methylene; and
R⁸ is selected from: hydrogen, methyl, and ethyl.

4. The compound of claim 1 or 2, wherein the compound has the following general formula: 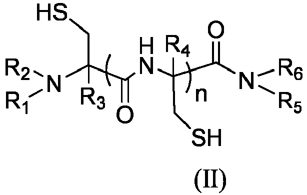 wherein the varaibles R₁, R₂, R₃, R₄, R₅, R₆ and n are defined as in claim 1.

5. The compound of claim 1 or 2, wherein n is an integer of 1 to 5.

6. The compound of claim 1 or 2, wherein n is an integer of 1.

7. The compound of claim 1 or 2, wherein n is an integer of 2.

8. The compound of claim 1 or 2, wherein the compound has the following general formulas: 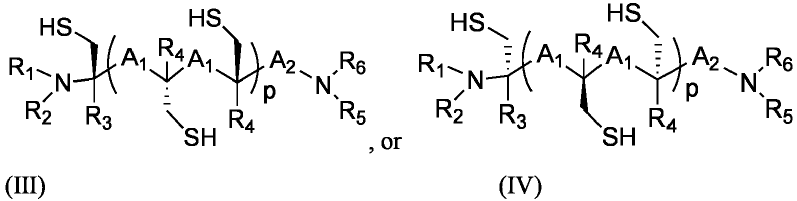 wherein p is an integer from 1 to 5.

9. The compound of claim 8, wherein p is an integer of 1.

10. The compound of claim 1, wherein the compound is selected from:

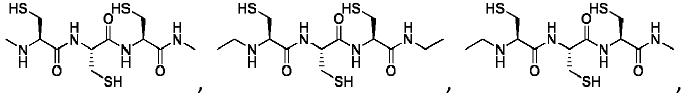

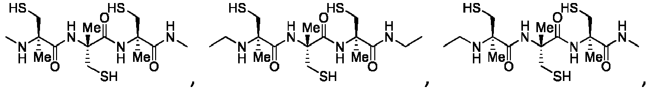

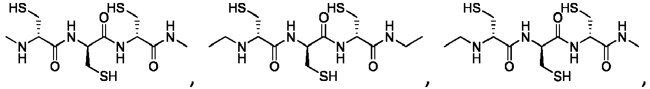

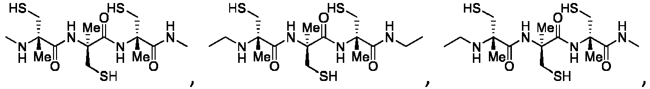

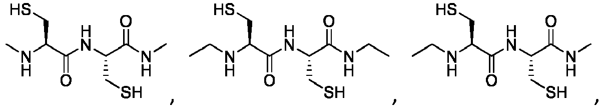

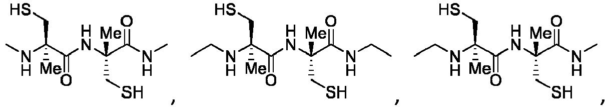

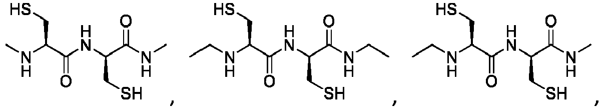

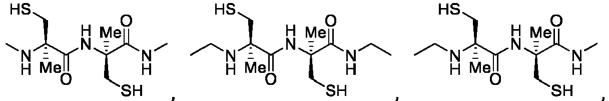

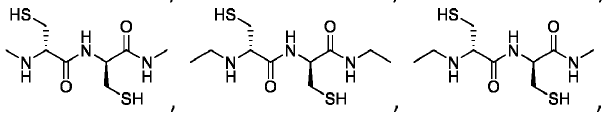

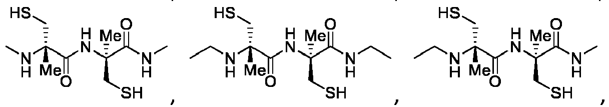

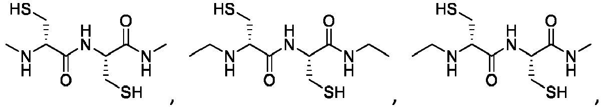

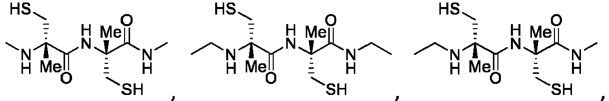

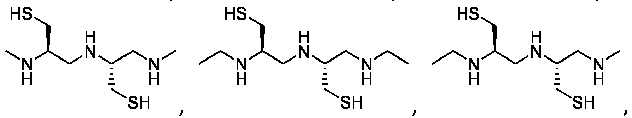

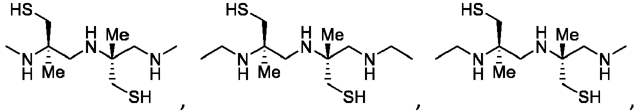

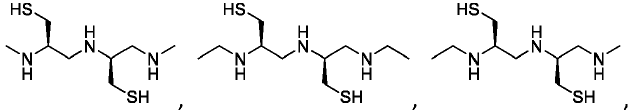

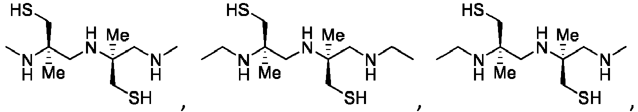

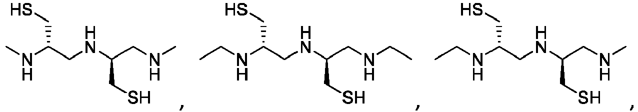

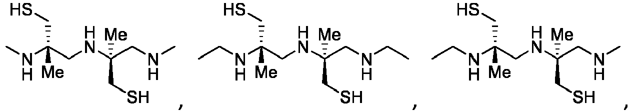

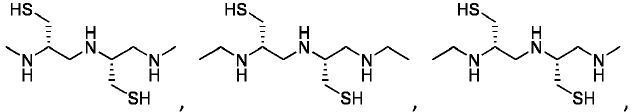

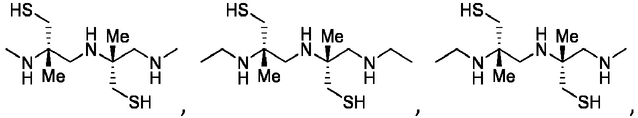

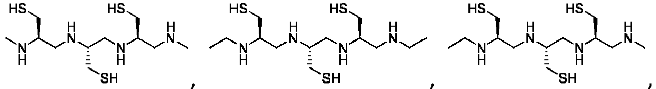

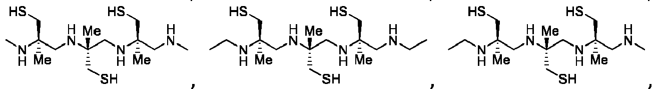

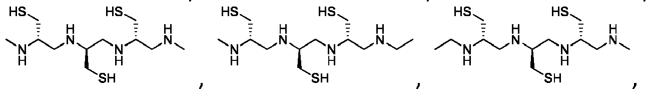

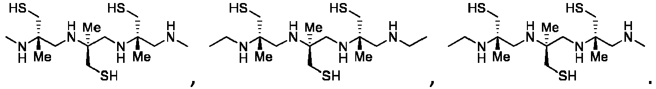

11. The compound of claim 1, wherein the compound is

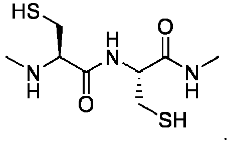

12. The compound of claim 1, wherein the compound is

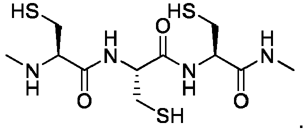

13. A pharmaceutical composition comprising the compound of any one of claims 1-12 and one or more pharmaceutically acceptable vehicles, carriers, adjuvants, auxiliaries or diluents.

14. The compound of any one of claims 1-12 for use in the treatment and/or prevention of radiation damage or chemotherapy damage.

15. The compound for use according to claim 14, wherein the radiation comprises ionizing radiation, non-ionizing radiation or a combination of various types of radiation; and
the ionizing radiation comprises alpha rays, beta rays, gamma rays, X rays, and neutron radiation;
the radiation damage comprises direct damage and indirect damage caused by radiation; and
the chemotherapy damage results from anti-tumor drugs acting on DNA, RNA and tubulin; and/or
the compound is used alone or in combination with a radioprotective agent.

16. The compound of any one of claims 1-12 for use in the treatment and/or prevention of sunburn damage.

17. The compound of any one of claims 1-12 for use in the treatment of tumors.

18. The compound of any one of claims 1-12 for use in combination with radiation therapy or chemotherapy to treat tumors.

## Patentansprüche

1. Verbindung von Formel (I): 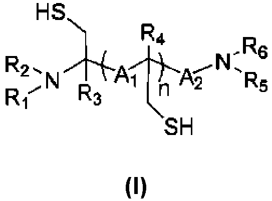 wobei
A₁ ausgewählt ist aus: -C(O)NR⁸- und -R⁷-NR⁸-;
A₂ ausgewählt ist aus: Carbonyl und unsubstituiertem C₁₋₆-Alkylen;
R¹ ausgewählt ist aus: Wasserstoff und unsubstituiertem C₁-C₅-Alkyl;
R² unsubstituiertes C₁-C₅-Alkyl ist;
R⁵ ausgewählt ist aus: Wasserstoff, unsubstituiertem C₁-C₅-Alkyl-,
R⁶ unsubstituiertes C₁-C₅-Alkyl ist;
n eine ganze Zahl von 1 bis 10 ist;
R³ und R⁴ unabhängig voneinander ausgewählt sind aus: Wasserstoff und unsubstituiertem C₁₋₆-Alkyl;
R⁷ unsubstituiertes C₁-C₆-Alkylen ist;
R⁸ ausgewählt ist aus: Wasserstoff und unsubstituiertem C₁-C₆-Alkyl;
oder einem Stereoisomer davon oder einem pharmazeutisch verträglichen Salz oder einem Solvat davon.

2. Verbindung nach Anspruch 1, wobei A₂ ausgewählt ist aus: Carbonyl und unsubstituiertem C₁₋₃-Alkylen;
R¹ ausgewählt ist aus: Wasserstoff und C₁₋₃-Alkyl;
R² C₁₋₃-Alkyl ist;
R⁵ ausgewählt ist aus: Wasserstoff und C₁₋₃-Alkyl;
R⁶ C₁₋₃-Alkyl ist;
n eine ganze Zahl von 1 bis 10 ist;
R³ und R⁴ unabhängig voneinander ausgewählt sind aus: Wasserstoff und unsubstituiertem C₁₋₃-Alkyl;
R⁷ unsubstituiertes C₁-C₃-Alkylen ist; und
R⁸ ausgewählt ist aus: Wasserstoff und unsubstituiertem C₁-C₃-Alkyl;
wobei der chirale Kohlenstoff, der an R³ und R⁴ direkt gebunden ist, in R-Konfiguration oder S-Konfiguration ist;
oder einem Stereoisomer davon oder einem pharmazeutisch verträglichen Salz oder einem Solvat davon.

3. Verbindung nach Anspruch 1 oder 2, wobei A₂ ausgewählt ist aus: Carbonyl und Methylen;
R¹ ausgewählt ist aus Wasserstoff, Methyl und Ethyl;
R² ausgewählt ist aus: Methyl und Ethyl;
R⁵ ausgewählt ist aus Wasserstoff, Methyl und Ethyl;
R⁶ ausgewählt ist aus: Methyl und Ethyl;
n eine ganze Zahl von 1 bis 10 ist;
R³ und R⁴ unabhängig voneinander ausgewählt sind aus: Wasserstoff und Methyl;
R⁷ Methylen ist; und
R⁸ ausgewählt ist aus: Wasserstoff, Methyl und Ethyl.

4. Verbindung nach Anspruch 1 oder 2, wobei die Verbindung die folgende allgemeine Formel aufweist: 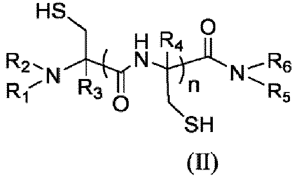 wobei die Variablen R₁, R₂, R₃, R₄, R₅, R₆ und n wie in Anspruch 1 definiert sind.

5. Verbindung nach Anspruch 1 oder 2, wobei n eine ganze Zahl von 1 bis 5 ist.

6. Verbindung nach Anspruch 1 oder 2, wobei n eine ganze Zahl von 1 ist.

7. Verbindung nach Anspruch 1 oder 2, wobei n eine ganze Zahl von 2 ist.

8. Verbindung nach Anspruch 1 oder 2, wobei die Verbindung die folgenden allgemeinen Formeln aufweist: 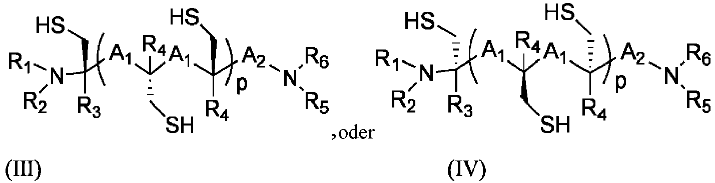 wobei p eine ganze Zahl von 1 bis 5 ist.

9. Verbindung nach Anspruch 8, wobei p eine ganze Zahl von 1 ist.

10. Verbindung nach Anspruch 1, wobei die Verbindung ausgewählt ist aus:

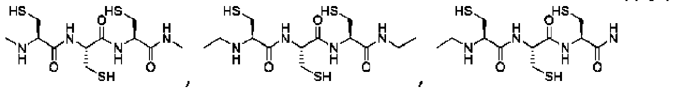

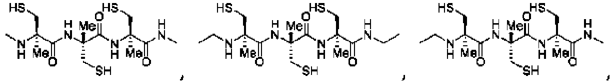

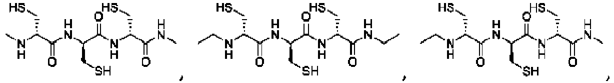

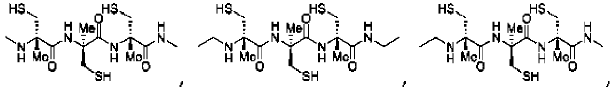

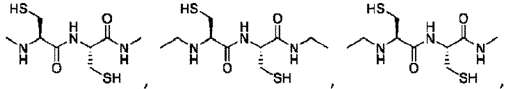

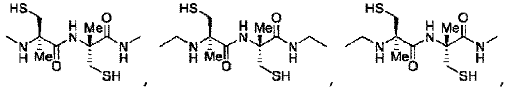

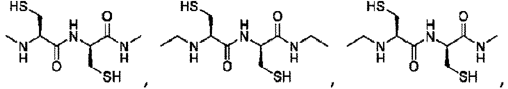

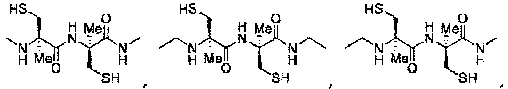

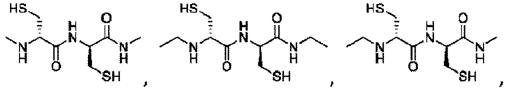

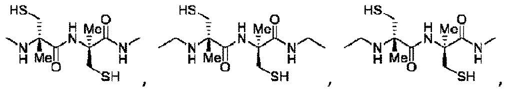

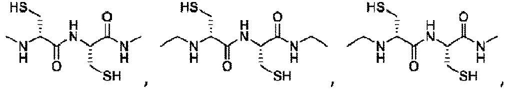

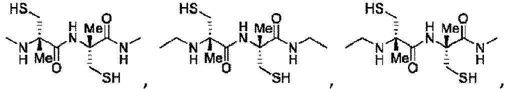

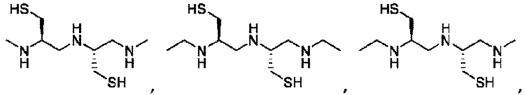

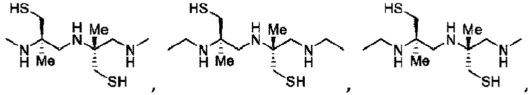

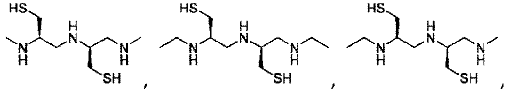

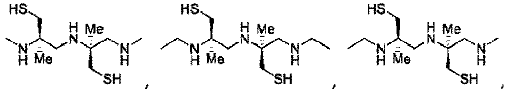

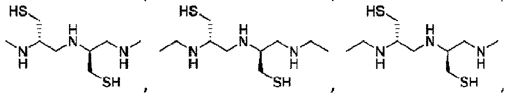

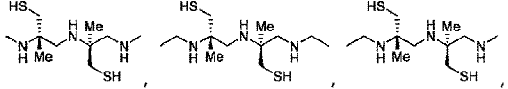

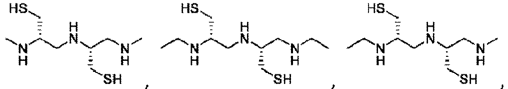

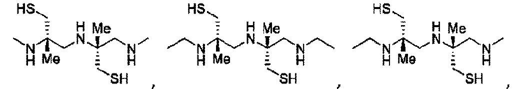

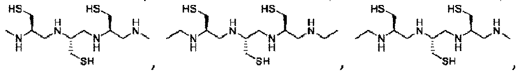

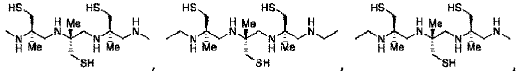

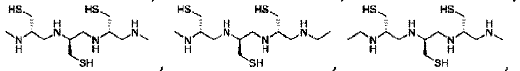

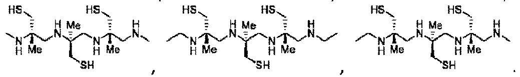

11. Verbindung nach Anspruch 1, wobei die Verbindung ist

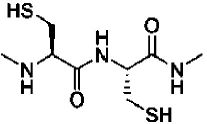

12. Verbindung nach Anspruch 1, wobei die Verbindung ist

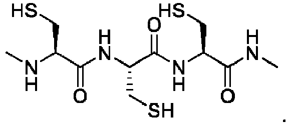

13. Pharmazeutische Zusammensetzung, umfassend die Verbindung nach einem der Ansprüche 1 bis 12 und einen oder mehrere pharmazeutisch verträgliche Vehikel, Träger, Adjuvanzien, Hilfsstoffe oder Verdünnungsmittel.

14. Verbindung nach einem der Ansprüche 1 bis 12 zur Verwendung bei der Behandlung und/oder Vorbeugung von Strahlenschaden oder Chemotherapieschaden.

15. Verbindung zur Verwendung nach Anspruch 14, wobei die Strahlung ionisierende Strahlung, nicht ionisierende Strahlung oder eine Kombination verschiedener Arten von Strahlung umfasst; und
die ionisierende Strahlung Alphastrahlen, Betastrahlen, Gammastrahlen, Röntgenstrahlen und Neutronenstrahlung umfasst;
der Strahlenschaden direkten Schaden und indirekten Schaden, die durch Strahlung verursacht werden, umfasst; und
der Chemotherapieschaden aus Antitumorarzneimitteln, die auf DNA, RNA und Tubulin wirken, resultiert; und/oder
die Verbindung allein oder in Kombination mit einem Strahlenschutzmittel verwendet wird.

16. Verbindung nach einem der Ansprüche 1 bis 12 zur Verwendung bei der Behandlung und/oder Vorbeugung von Sonnenbrandschaden.

17. Verbindung nach einem der Ansprüche 1 bis 12 zur Verwendung bei der Behandlung von Tumoren.

18. Verbindung nach einem der Ansprüche 1 bis 12 zur Verwendung in Kombination mit Strahlentherapie oder Chemotherapie, um Tumore zu behandeln.

## Revendications

1. Composé de formule (I) : 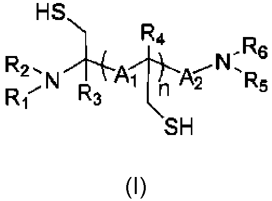 dans laquelle
A₁ est choisi parmi : -C(O)NR⁸-, et -R⁷-NR⁸- ;
A₂ est choisi parmi : carbonyle et alkylène en C_{1 à 6} non substitué ;
R¹ est choisi parmi : hydrogène et alkyle en C₁ à C₅ non substitué ;
R² est alkyle en C₁ à C₅ non substitué ;
R⁵ est choisi parmi : hydrogène, alkyle en C₁ à C₅ non substitué ;
R⁶ est alkyle en C₁ à C₅ non substitué ;
n est un nombre entier allant de 1 à 10 ;
R³ et R⁴ sont indépendamment choisis parmi : hydrogène, et alkyle en C_{1 à 6} non substitué ;
R⁷ est alkylène en C₁ à C₆ non substitué ;
R⁸ est choisi parmi : hydrogène et alkyle en C₁ à C₆ non substitué ;
ou un stéréoisomère de celui-ci ou un sel pharmaceutiquement acceptable, ou un solvate de celui-ci.

2. Composé selon la revendication 1, dans lequel A₂ est choisi parmi : carbonyle et alkylène en C_{1 à 3} non substitué ;
R¹ est choisi parmi : hydrogène et alkyle en C_{1 à 3} ;
R² est alkyle en C_{1 à 3} ;
R⁵ est choisi parmi : hydrogène et alkyle en C_{1 à 3} ;
R⁶ est alkyle en C_{1 à 3} ;
n est un nombre entier allant de 1 à 10 ;
R³ et R⁴ sont indépendamment choisis parmi : hydrogène, et alkyle en C_{1 à 3} non substitué ;
R⁷ est alkylène en C₁ à C₃ non substitué ; et
R⁸ est choisi parmi : hydrogène, et alkyle en C₁ à C₃ non substitué ;
dans laquelle le carbone chiral directement fixé à R³ et R⁴ est en configuration R ou en configuration S ;
ou un stéréoisomère de celui-ci ou un sel pharmaceutiquement acceptable, ou un solvate de celui-ci.

3. Composé selon la revendication 1 ou 2, dans lequel A₂ est choisi parmi : carbonyle, et méthylène ;
R¹ est choisi parmi : hydrogène, méthyle et éthyle ;
R² est choisi parmi : méthyle et éthyle ;
R⁵ est choisi parmi : hydrogène, méthyle et éthyle ;
R⁶ est choisi parmi : méthyle et éthyle ;
n est un nombre entier allant de 1 à 10 ;
R³ et R⁴ sont indépendamment choisis parmi : hydrogène et méthyle ;
R⁷ est méthylène ; et
R⁸ est choisi parmi : hydrogène, méthyle et éthyle.

4. Composé selon la revendication 1 ou 2, dans lequel le composé a la formule générale suivante : 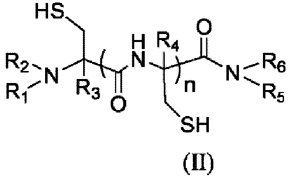 dans laquelle les variables R₁, R₂, R₃, R₄, R₅, R₆ et n sont définis comme dans la revendication 1.

5. Composé selon la revendication 1 ou 2, dans lequel n est un nombre entier de 1 à 5.

6. Composé selon la revendication 1 ou 2, dans lequel n est un nombre entier de 1.

7. Composé selon la revendication 1 ou 2, dans lequel n est un nombre entier de 2.

8. Composé selon la revendication 1 ou 2, dans lequel le composé a les formules générales suivantes : 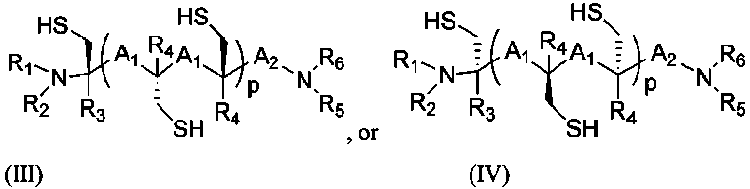 dans lesquelles p est un nombre entier allant de 1 à 5.

9. Composé selon la revendication 8, dans lequel p est un nombre entier de 1.

10. Composé selon la revendication 1, dans lequel le composé est choisi parmi :

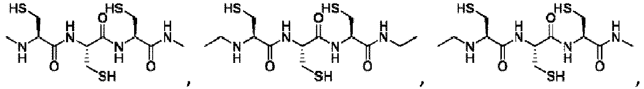

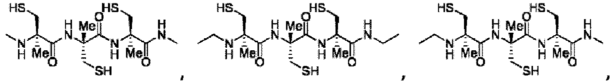

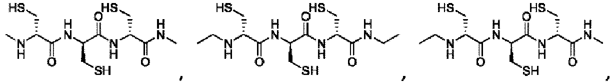

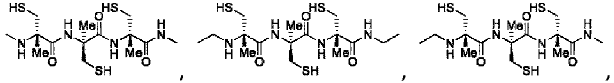

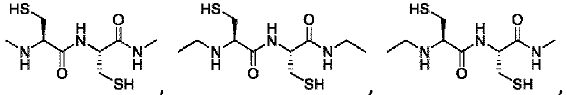

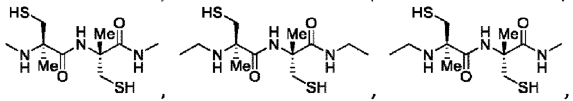

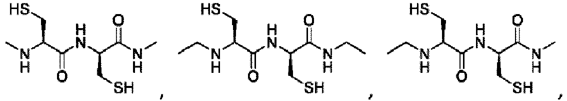

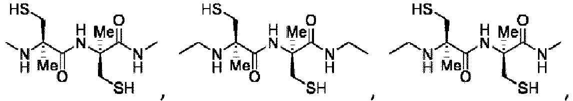

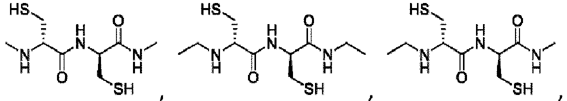

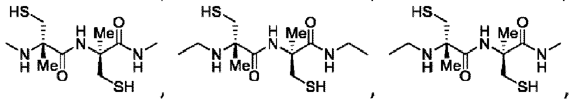

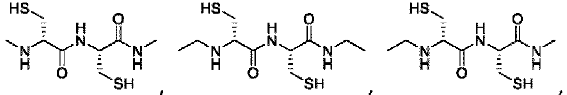

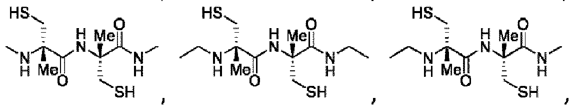

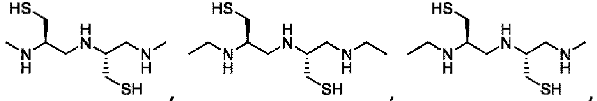

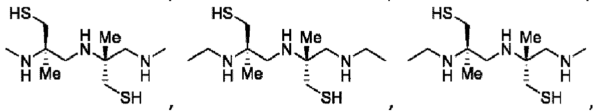

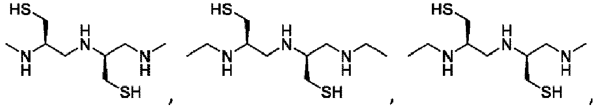

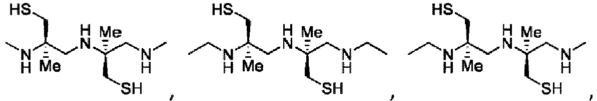

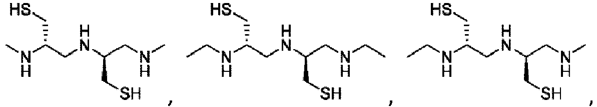

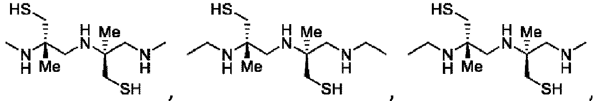

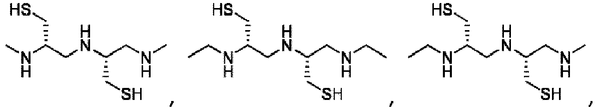

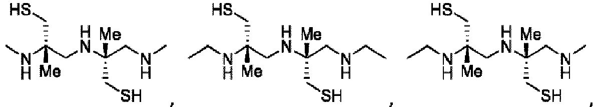

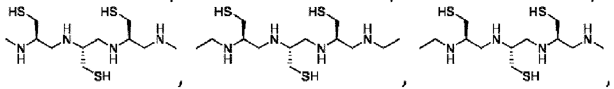

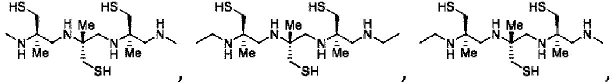

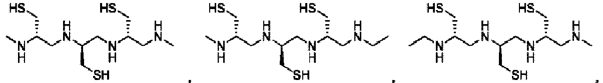

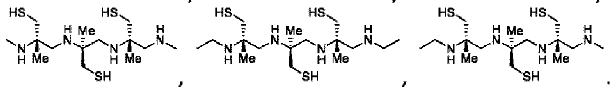

11. Composé selon la revendication 1, dans lequel le composé est

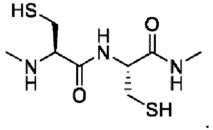

12. Composé selon la revendication 1, dans lequel le composé est

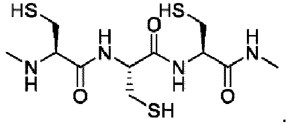

13. Composition pharmaceutique comprenant le composé selon l'une quelconque des revendications 1 à 12 et un ou plusieurs véhicules, supports, adjuvants, auxiliaires ou diluants pharmaceutiquement acceptables.

14. Composé selon l'une quelconque des revendications 1 à 12 destiné à être utilisé dans le traitement et/ou la prévention de dommages causés par rayonnement ou de dommages causés par chimiothérapie.

15. Composé destiné à être utilisé selon la revendication 14, dans lequel le rayonnement comprend un rayonnement ionisant, un rayonnement non ionisant ou une combinaison de divers types de rayonnement ; et
le rayonnement ionisant comprend des rayons alpha, des rayons bêta, des rayons gamma, des rayons X, et un rayonnement neutronique ;
les dommages sur le rayonnement comprennent des dommages directs et des dommages indirects provoqués par un rayonnement ; et
les dommages causés par la chimiothérapie résultent de médicaments antitumoraux agissant sur l'ADN, l'ARN et la tubuline ; et/ou
le composé est utilisé seul ou en combinaison avec un agent radioprotecteur.

16. Composé selon l'une quelconque des revendications 1 à 12 destiné à être utilisé dans le traitement et/ou la prévention des dommages causés par des coups de soleil.

17. Composé selon l'une quelconque des revendications 1 à 12 destiné à être utilisé dans le traitement de tumeurs.

18. Composé selon l'une quelconque des revendications 1 à 12 destiné à être utilisé en combinaison avec une radiothérapie ou une chimiothérapie pour traiter des tumeurs.
